(19) Europäisches Patentamt
European Patent Office
Office européen des brevets

(11) EP 0 452 151 B1

(12) # EUROPEAN PATENT SPECIFICATION

(45) Date of publication and mention of the grant of the patent: **16.07.1997 Bulletin 1997/29**

(21) Application number: **91303269.4**

(22) Date of filing: **12.04.1991**

(51) Int. Cl.$^6$: **C07D 495/04**, A61K 31/38 // (C07D495/04, 335:00, 333:00), (C07D495/04, 335:00, 307:00)

(54) **Substituted aromatic sulfonamides as antiglaucoma agents**

Substituierte aromatische Sulfonamide als Mittel gegen Glaukom

Sulfonamides aromatiques substitués comme agents antiglaucomiques

(84) Designated Contracting States:
**AT BE CH DE DK ES FR GB GR IT LI LU NL SE**

(30) Priority: **12.04.1990 US 507805**

(43) Date of publication of application: **16.10.1991 Bulletin 1991/42**

(73) Proprietor: **Merck & Co., Inc.**
**Rahway New Jersey 07065-0900 (US)**

(72) Inventors:
- **Baldwin, John J.**
  **Gwynedd Valley, PA 19437 (US)**
- **Ponticello, Gerald S.**
  **Lansdale, PA 19446 (US)**
- **Habecker, Charles N.**
  **Lansdale, PA 19446 (US)**
- **Selnick, Harold G.**
  **Ambler, PA 19002 (US)**

(74) Representative: **Thompson, John Dr. et al**
**Merck & Co., Inc.**
**European Patent Department**
**Terlings Park**
**Eastwick Road**
**Harlow, Essex CM20 2QR (GB)**

(56) References cited:
**EP-A- 0 193 132**
**EP-A- 0 411 704**
**US-A- 4 677 115**
**US-A- 4 797 413**

Remarks:
The file contains technical information submitted after the application was filed and not included in this specification

EP 0 452 151 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.14.11/3.4

## Description

This invention relates to novel aromatic sulfonamides useful in the preparation of medicaments for the treatment of elevated intraocular pressure. More particularly this invention relates to compounds having the structural formula:

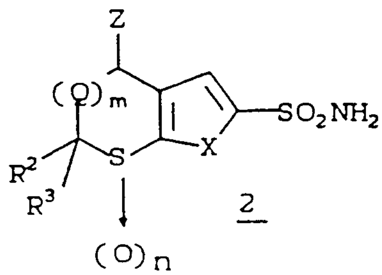

2

as well as the pharmaceutically and ophthalmologically acceptable salts thereof. This invention also relates to pharmaceutical compositions and the use thereof for systemic and ophthalmic use employing a novel compound of this invention as active ingredient for the treatment of elevated intraocular pressure, especially when accompanied by pathological damage such as in the disease known as glaucoma.

BACKGROUND OF THE INVENTION

Glaucoma is an ocular disorder associated with elevated intraocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Only recently have clinicians noted that many β-adrenergic blocking agents are effective in reducing intraocular pressure. While many of these agents are effective in reducing intraocular pressure, they also have other characteristics, e.g. membrane stabilizing activity, that are not acceptable for chronic ocular use. (S)-1-tert-Butylamino--[(4-morpholino-1,2,5-thiadiazol-3-yl)oxy]-2-propanol, a β-adrenergic blocking agent, was found to reduce intraocular pressure and to be devoid of many unwanted side effects associated with pilocarpine and, in addition, to possess advantages over many other β-adrenergic blocking agents, e.g. to be devoid of local anesthetic properties, to have a long duration of activity, and to display minimal tolerance.

Although pilocarpine, physostigmine and the β-blocking agents mentioned above reduce intraocular pressure, none of these drugs manifests its action by inhibiting the enzyme carbonic anhydrase and, thereby, impeding the contribution to aqueous humor formation made by the carbonic anhydrase pathway.

Agents referred to as carbonic anhydrase inhibitors block or impede this inflow pathway by inhibiting the enzyme, carbonic anhydrase. While such carbonic anhydrase inhibitors are now used to treat intraocular pressure by oral, intravenous or other systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective. Despite the desirability of directing the carbonic anhydrase inhibitor only to the desired ophthalmic target tissue, no topically effective carbonic anhydrase inhibitors are available for clinical use.

However, topically effective carbonic anhydrase inhibitors are reported in U.S. Patents 4,386,098; 4,416,890; and 4,426,388. The compounds reported therein are 5 (and 6)-hydroxy-2-benzothiazolesulfonamides and acyl esters thereof.

More recently, U.S. Patents 4,677,115 and 4,797,413 describe topically effective carbonic anhydrase inhibitors which are thienothiopyran-2 sulfonamides differing from the compounds of the present application in the nature of the substituent on the thiopyran moiety adjacent to the sulfur atom.

European patent specification No. 0 411 704-A describes a class of carbonic anhydrase inhibitors of the thieno[2,3-b]thiopyran-2-sulfonamide type with a substituted-alkyl-amino group in the 4-position. EP-0 411 704-A is a reference to the present application under Article 54(3) EPC.

US Patent No. 4,863,922 describes topically effective carbonic anhydrase inhibitors of the same class as those disclosed in US Patent No. 4,797,413 discussed above.

DETAILED DESCRIPTION OF THE INVENTION

The novel compounds of this invention are those with structural formula:

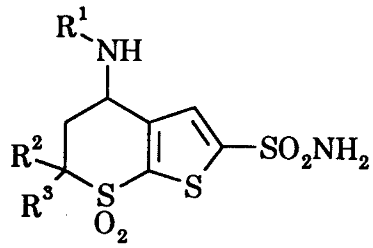

its isomers, or a pharmaceutically acceptable salt thereof wherein:

$R^1$ is $C_{1-6}$alkyl;
$R^2$ is hydrogen or $C_{1-6}$alkyl;
$R^3$ is

(a) $C_{1-5}$alkoxy-$C_{1-5}$alkyl,
(b) hydroxy-$C_{1-5}$alkoxy-$C_{1-5}$alkyl,
(c) $C_{1-5}$alkoxy-$C_{1-5}$alkoxy-$C_{1-5}$alkyl,
(d) hydroxy-$C_{1-5}$alkylamino-$C_{1-5}$alkyl,
(e) hydroxy-$C_{1-5}$alkylthio-$C_{1-5}$alkyl, or
(f) $C_{2-5}$alkenyloxy-$C_{1-5}$alkyl.

The presence of substituents on the dihydrothiopyran ring results in compounds with asymmetric carbons. This invention contemplates all of the enantiomers and diastereomers and mixtures thereof.

In the following specification and claims where the absolute configuration of a compound is expressed as cis(S,R)-, the (S) refers to the configuration at the 4-position, and the (R) refers to the configuration at the carbon adjacent to the -$S(O)_2$- of the thiopyran ring, which is the 6-position.

The ophthalmologically acceptable salts coming within the purview of this invention include the ophthalmologically acceptable acid addition salts. Acids useful for preparing these acid addition salts include, inter alia, inorganic acids, such as the hydrohalic acids (e.g., hydrochloric and hydrobromic acid), sulfuric acid, nitric acid, and phosphoric acids, and organic acids such as maleic, fumaric, tartaric, citric, acetic, benzoic, 2-acetoxybenzoic, salicylic, succinic, p-aminobenzoic, isethionic, lactic, p-acetmidobenzoic, methanesulfonic, or ethanedisulfonic acid.

The novel processes for preparing the novel compounds of this invention generally comprise as last step, formation of the $NHR^1$ substituent.

Reduction of the N-acyl group with borane-dimethylsulfide complex in an ethereal solvent such as THF, diethylether, or 1,2-dimethoxyethane provides an alkylamino as exemplified below by reduction of acetamido to ethylamino. The amide starting materials can be prepared by acylation of the 4-amino compounds.

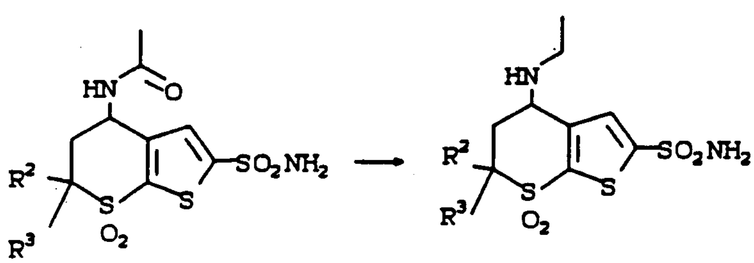

Alkylamino groups are also available from the corresponding 4-hydroxy compounds by treatment of the 4-hydroxy with toluenesulfonyl chloride in pyridine at about -20°C to 5°C for about 3 to 10 hours followed by the addition of an alkylamine at a temperature below about 15°C followed by warming to about 30°-60° C. for about 5 to 16 hours as shown below:

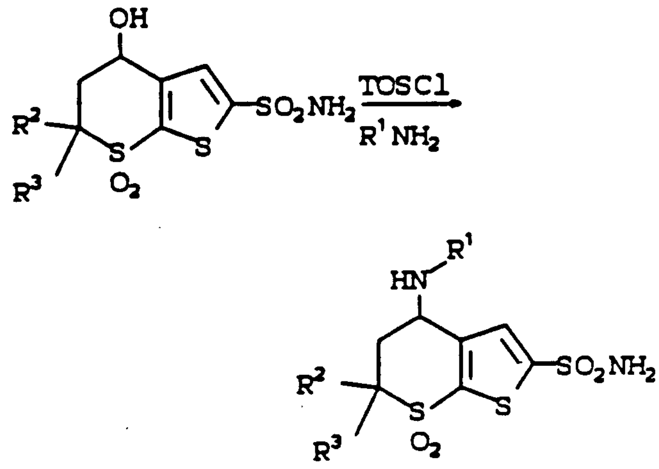


4-Alkylamines are also prepared from the 4-oxo compounds by the following scheme:

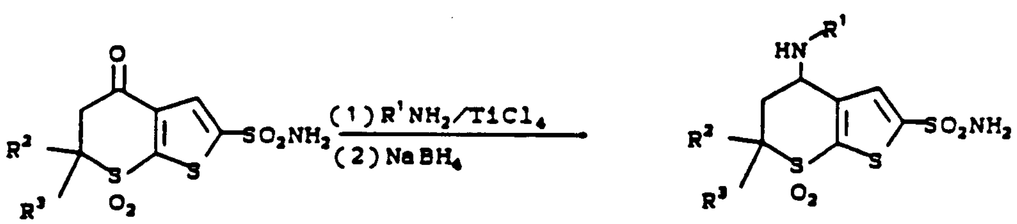


In this process a solution of the keto compound in a solvent such as diethylether, THF, 1,2-dimethoxyethane, benzene, toluene or mixtures thereof at about -20°C to 0°C is treated quickly with about a one molar excess of an amine of formula $R^1NH_2$ followed by titanium tetrachloride dropwise. After about 1 to 5 hours the mixture is filtered and evaporated. The residue is treated with a complex metal hydride such as sodium borohydride, in excess in a $C_{1-3}$alkanol, preferably methanol, at about room temperature for up to 24 hours. Excess hydride is destroyed with aqueous acid and the product is isolated by standard techniques.

This invention is particularly concerned with formulations adapted for topical ocular administration in the form of solutions, ointments, solid water-soluble inserts or gels for the treatment of glaucoma and other stages of elevated intraocular pressure and contain about 0.1% to 15% by weight of medicament, especially about 0.5% to 2% by weight of medicament, the remainder being comprised of carriers and other excipients well known in the art. The compounds of this invention are also useful for treating psoriasis, and therefore, this invention is also concerned with the topical use of the above formulations for the treatment of psoriasis.

The medicament in the novel topical ocular formulations for use in treating glaucoma and other stages of elevated intraocular pressure comprises one of the novel compounds of this invention either alone or in combination with a β-adrenergic blocking agent such as timolol maleate, a para-sympathomimetic agent such as pilocarpine, or an angiotensin converting enzyme (ACE) inhibitor such as enalapril. The medicament combination may also contain other agents that reduce intraocular pressure, e.g., a potassium channel agonist such as cromokalin, an agonist at the cannabinoid receptor such as tetrahydrocannabinoid or WIN 55212-2, 2-ethyl-2,3-dihydro-5-methylpyrrolo[1,2,3-de]-1,4-benzoxazine-6-ethanamine, a dopamine agonist, ANF or a prostoglandin. In such combinations the two active agents are present in approximately equal amounts.

The medicament in the novel topical formulations for use in treating psoriasis comprises one of the novel compounds of this invention in free-base form. The compounds of this invention can also be orally administered to patients in need of treatment for psoriasis in typical pharmaceutical formulations such as tablets, capsules and elixirs. Although the dose will vary from patient to patient depending upon the nature and severity of disease, the patient's weight, and other factors which those skilled in the art will recognize, the dosage range will generally be about 1 to 500 mg, per patient per day, which can be administered in doses from once to three times a day. Preferably, the dosage range will be about 2 to 100 mg, per patient per day.

One novel aspect of this invention comprises the use of a compound of this invention for the manufacture of a medicament for the treatment of elevated intraocular pressure. Of primary concern is the treatment by topical ocular administration of about 0.1 to 25 mg and especially 0.1 to 10 mg of such compound per day, either by single dose or on a 2 to 4 dose per day regimen.

Another aspect of this invention comprises the use of a compound of this invention for the manufacture of a medicament for the treatment of psoriasis by oral or topical administration.

As used herein, the a designation refers to the trans configuration and the β designation refers to the cis configuration, except for in Example 42 where the α designation refers to the first compound to elute from the chromatography column.

EXAMPLE 2

5,6-Dihydro-6-(2-ethoxyethyl)-4-ethylamino-4H-thieno-[2,3-b]thiopyran-2-sulfonamide-7,7-dioxide

Step A: Preparation of 3-(2-thienylthio) glutaric acid.

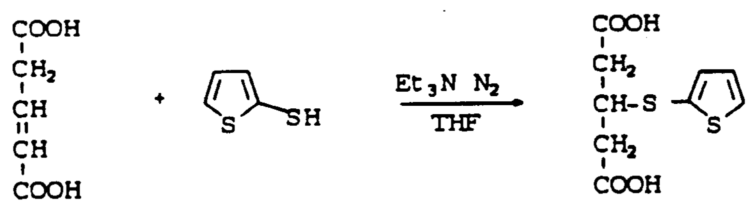


10

To a stirred solution of glutaconic acid (50.0 g, 0.43 mol) in dry tetrahydrofuran (500 ml) under nitrogen was added triethylamine (130 ml, 0.90 mol) followed by 2-thiophenethiol (50.0 g, 0.43 mol). The mixture was stirred at room temperature over the weekend. The mixture was concentrated in vacuo and the residual oil was poured into cold 6N HCl (600 ml). This was extracted with ethyl acetate (200 and 4 x 100 ml). The combined extracts were washed free of strong acid using saturated sodium chloride, dried, filtered and concentrated in vacuo. The product, 10, was a beige solid (103.4 g), m.p. 119-123°C. Yield was 98%.

Step B: Preparation of 5,6-dihydro-4H-4-oxothieno-[2,3-b]thiopyran-6-acetic acid hydrochloride

10

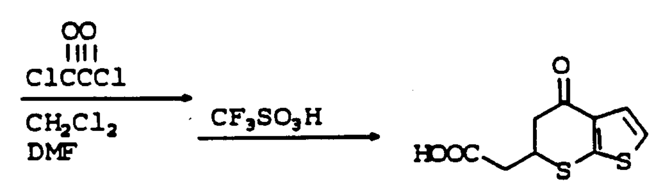


11

To a stirred suspension of 3-(2-thienylthio) glutaric acid (103.4 g, 0.42 mol) in dry methylene chloride (400 ml) was added dimethylformamide (3 ml) followed by the dropwise addition of oxalyl chloride (87 ml, 1.0 mol) added over 1 1/4 hours. The resulting solution was stirred for 2 1/2 hours at room temperature and then was concentrated in vacuo. The residual oil was taken up in dry methylene chloride (350 ml) and the solution was cooled to -78°C. Trifluoromethanesulfonic acid (74.3 ml, 0.84 mol) was added dropwise over 1/2 hour and stirring was continued for ¼ hour at -78°C. Then the temperature was allowed to rise to 15°C. over 1 1/4 hours and the mixture was poured into ice and water (1500 ml). This mixture was stirred overnight under nitrogen in an open beaker. The semi-solid which had formed was separated

by decanting the aqueous solution. The solid remaining was stirred in ether (800 ml) and a tan solid formed. The solid was dissolved in ethyl acetate and the combined ether-ethyl acetate solutions were washed free of strong acid with saturated sodium chloride, dried, filtered and concentrated in vacuo. The product 11. was a tan solid (93.8 g), mp 112-117°C. Yield was 98%.

Step C: Preparation of Ethyl 5,6-dihydro-4-oxo-4H-thieno[2,3-b]thiopyran-6-acetate

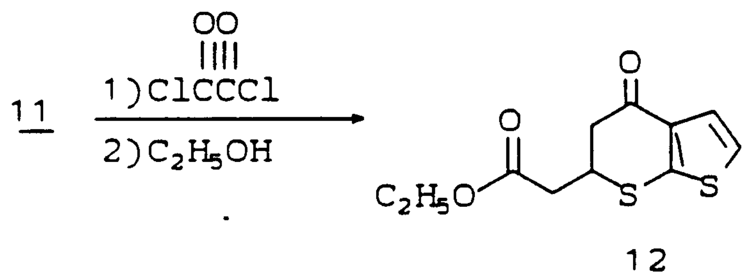


12

5,6-Dihydro-4-oxo-4H-thieno[2,3-b]thiopyran-6-acetic acid (78.0 g, 0.34 mol) was stirred in dry methylene chloride (450 ml) containing dimethylformamide (1/2 ml) and oxalyl chloride (45 ml, 0.51 mol) was added dropwise over 20 minutes. The mixture was stirred at room temperature for 3 hours and then was concentrated in vacuo. The residual oil was taken up in ice cold ethanol (200 ml). The solution was stirred at room temperature for 2 hours and was concentrated in vacuo. The residual oil was taken up in ethyl acetate and was washed with satruated sodium bicarbonate, saturated sodium chloride and was dried, filtered and concentrated in vacuo to give 72.2 g, of the liquid ester. Crude yield was 83%.

Step D: Preparation of Ethyl 5,6-dihydro-4-hydroxy-4H-thieno[2,3-b]thiopyran-6-acetate

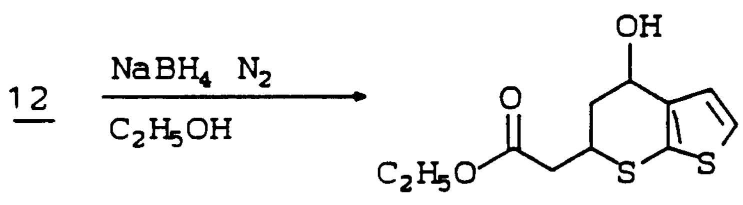


13

To a stirred solution of ethyl 5,6-dihydro-4-oxo-4H-thieno[2,3-b]thiopyran-6-acetate (72.2 g, 0.28 mol) in ethnaol (300 ml) under nitrogen and cooled to 0°C was added sodium borohydride (2.65 g, 0,07 mol). The mixture was stirred for 1 1/2 hours at 0°C and then additional sodium borohydride (1.32 g, 0.035 mol) was added. Stirring was continued for 2 hours at room temperature. Acetone (25 ml) was added and the mixture was concentrated in vacuo. The residual oil was dissolved in ethyl acetate (350 ml), washed with saturated sodium chloride, dried, filtered and concentrated in vacuo. The residual oil was purified by chromatography on silica gel (300 g) using 70:30 hexane:ethyl acetate. The alcohol was recovered as a yellow solid (48 g). Yield was 66%.

Step E: Preparation of Ethyl 5,6-dihydro-4-methoxyethoxymethoxy-4H-thieno[2,3-b]thiopyran-6-acetate

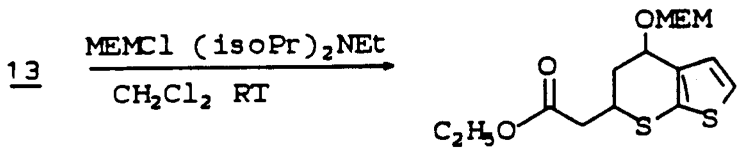


14

Ethyl 5,6-dihydro-4-hydroxy-4H-thieno[2,3-b]-thiopyran-6-acetate (38.6 g, 0.15 mol) was dissolved in dry methylene chloride (200 ml) and diisopropylethylamine (21.3 g, 0.165 mol) was added with cooling followed by MEM chloride (20.6 g, 0.165 mol). The mixture was stirred at room temperature overnight. Then water (300 ml) was added. The aqueous layer was extracted with methylene chloride. The methylene chloride solutions were combined, washed with saturated sodium bicarbonate and water, dried, filtered and concentrated in vacuo. The MEM ether was obtained as a liquid (48.6 g). Yield was 93%.

Step F: Preparation of 5,6-dihydro-6-(2-hydroxyethyl)-4-methoxyethoxyethoxy-4H-thieno-[2,3-b]thiopyran

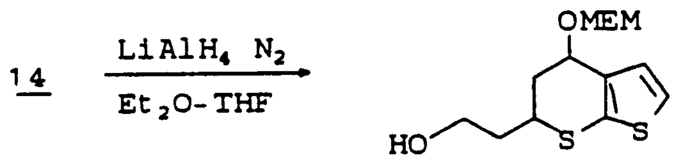


15

To a stirred suspension of lithium aluminum hydride (7.9 g, 0.21 mol) under nitrogen in anhydrous ether (300 ml) cooled to 0°C was added dropwise over 3/4 hours ethyl 5,6-dihydro-4-methoxyethoxymethoxy-4H-thieno[2,3-b]thiopyran-6-acetate (48.6 g, 0.14 mol) in dry tetrahydrofuran (150 ml). The mixture was stirred at 0°C for 1 hour and at room temperature for 2 hours. Then it was cooled to 0°C and was decomposed by adding carefully dropwise water (6 ml), 10% sodium hydroxide (8 ml) and water (16 ml). The mixture was filtered and the solids were washed with ether and tetrahydrofuran. The filtrate and washings were combined, dried, filtered and concentrated in vacuo. The product was a yellow liquid (40.3 g). Yield was 95%.

Step G: Preparation of 5,6-dihydro-6-(2-ethoxyethyl)-4-methoxyethoxymethoxy)-4H-thieno[2,3-b]thiopyran

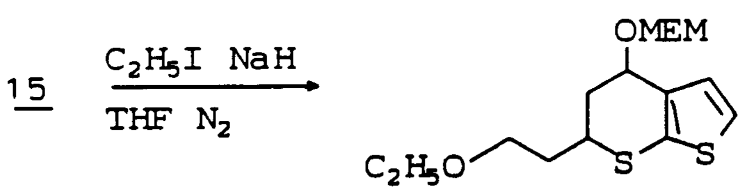


16

Sodium hydride (7.8 g, 0.163 mol of 50% dispersion in mineral oil) was washed free of mineral oil using petroleum

ether under nitrogen and was suspended in dry tetrahydrofuran (50 ml). The suspension was coolded to 0°C and was stirred as 5,6-dihydro-6-(2-hdyroxyethyl)-4-methoxyethoxymethoxy-4H-thieno[2,3-b]thiopyran (40.0 g, 0.13 mol) dissolved in dry tetrahydrofuran (150 ml) was added over 1/2 hour. The mixture was stirred at 0°C for an additional 1/2 hour and then at 35°C for 3/4 hours. Ethyl iodide (20.8 ml, 0.26 mol) was added and the mixture was stirred overnight at 65°C. Additional ethyl iodide (20 ml and 10 ml) was added over a 12 hour reflux period to complete the reaction. The mixture was concentrated in vacuo. The oil-solid residue was taken up in ether (200 ml) and was washed with water, dried, filtered and concentrated in vacuo to obtain an amber oil (40.0g). Crude yield 93%.

Step H: Preparation of 5,6-dihydro-6-(2-ethoxyethyl)-4-methoxyethoxymethoxy-4H-thieno[2,3-b]thiopyran-2-sulfonamide

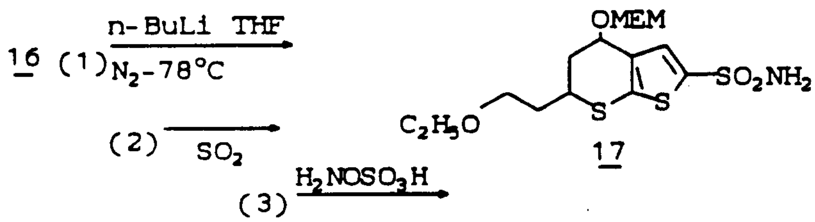


5,6-Dihydro-6-(2-ethoxyethyl)-4-methoxyethoxymethoxy-4H-thieno[2,3-b]thiopyran (13.9 g, 0.042 mol) was dissolved in dry tetrahydrofuran (100 ml) and the solution was cooled to -78°C under nitrogen. Then n-butyl lithium (20 ml, 0.05 mol of 2.5 M in hexane) was added dropwise over 1/2 hour. The solution was stirred at -78°C for an additional hour. Then anhydrous $SO_2$ was passed over the surface at -78°C to -40°C until the mixture became acidic. Stirring at -40 to -78°C was continued for 2 1/4 hours and then to room temperature over 1/2 hour. The solution was then concentrated in vacuo. The residual lithio salt was dissolved in water (75 ml) containing sodium acetate (9.8 g, 0.12 mol) and the solution was cooled to 0°C. Hydroxylamine-O-sulfonic acid (11.3 g, 0.10 mol) was added and the mixture was stirred at room temperature overnight. The reaction mixture was diluted with saturated sodium bicarbonate (100 ml) and ethyl acetate (200 ml). The aqueous layer was separated and extracted with ethyl acetate. The ethyl acetate solutions were combined, washed with water, dried, filtered and concentrated in vacuo to a pale tan solid (15.8 g). Yield 91%. Recrystallization from ethanol gave material with mp 99-101°C.

Step I: Preparation of 5,6-dihydro-6-(2-ethoxyethyl)-4-methoxyethoxymethoxy-4H-thieno[2,3-b]thiopyran-2-sulfonamide-7,7-dioxide

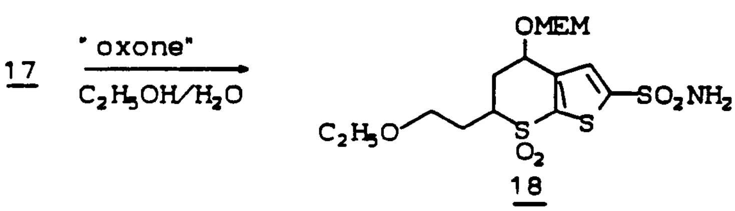


5,6-Dihydro-6-(2-ethoxyethyl)-4-methoxyethoxymethoxy-4H-thieno[2,3-b]thiopyran-2-sulfonamide (28.3 g, 0.069 mol) was dissolved in ethanol (300 ml) with warming and water (150 ml) was added. "Oxone"® (6.79 g, 0.11 mol) was added and the mixture was stirred at room temperature for 4 3/4 hours. Then the mixture was cooled in ice and sodium bicarbonate was added portionwise until the mixture became basic. The mixture was filtered and the solids were washed with ethanol and ethyl acetate. The combined filtrate and washings were concentrated in vacuo. Ethyl acetate (400 ml) was added to the residue with a minimum amount of water to dissolve the remaining salts. The ethyl acetate was separated, dried, filtered and concentrated in vacuo to a pale yellow oil (28.5 g). Yield was 93%.

Step J: Preparation of 5,6-dihydro-6-(2-ethoxyethyl)-4-hydroxy-4H-thieno[2,3-b]thiopyran-2-sulfonamide-7,7-dioxide

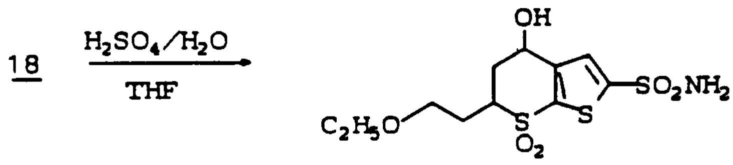


19

5,6-Dihydro-6-(2-ethoxyethyl)-4-methoxyethoxymethoxy-4H-thieno[2,3-b]thiopyran-2-sulfonamide-7,7-dioxide (28.4 g, 0.064 mol) was dissolved in tetrahydrofuran (50 ml) and a 50/50 (volume/volume) solution of sulfuric acid/water (100 ml) was added. The solution was stirred at room temperature for 2 1/2 hours. Then the solution was carefully poured into sodium bicarbonate (200 g), ice, and water (100 ml). Then ethyl acetate (300 ml) was added and the mixture was filtered. The solids were washed with ethyl acetate. The aqueous layer was separated and was extracted with ethyl acetate. The ethyl acetate solutions were combined, washed with saturated sodium chloride, dried, filtered and concentrated in vacuo to an amber oil (23.0 g). This material was not pure but was used without further purification.

Step K: Preparation of 4-acetamido-5,6-dihydro-6-(2-ethoxyethyl)-4H-thieno[2,3-b]thiopyran-2-sulfonamide-7,7-dioxide

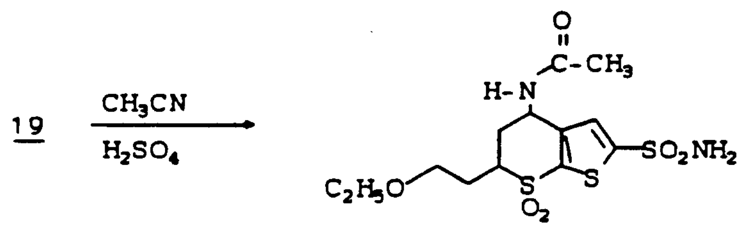


20

To a stirred solution of 5,6-dihydro-6(2-ethoxyethyl)-4-hydroxy-4H-thieno[2,3-b]thiopyran-7,7-dioxide (12.3 g, 0.035 mol) in dry acetonitrile (100 ml) under nitrogen and cooled to -10°C was added dropwise over 1/4 hour concentrated sulfuric acid (9.3 ml, 0.175 mol). The solution was stirred for 3 hrs at -10°C and then from -10°C to room temperature overnight. The mixture was cooled to -10°C and another 9.3 ml of sulfuric acid was added and stirring at room temperature was continued overnight to complete the reaction. Then the solution was poured into ice and water (200 ml) and was extracted with ethyl acetate (150 and 2 x 50 ml). The combined ethyl acetate extracts were washed with saturated sodium chloride, saturated sodium bicarbonate and again with saturated sodium chloride, dried, filtered and concentrated <u>in vacuo</u> to a tan foam (6.8 g). Yield was 49%.

Step L: Preparation of 5,6-dihydro-6-(2-ethoxyethyl)-4-ethylamino-4H-thieno[2,3-b]thiopyran-2-sulfonamide-7,7-dioxide

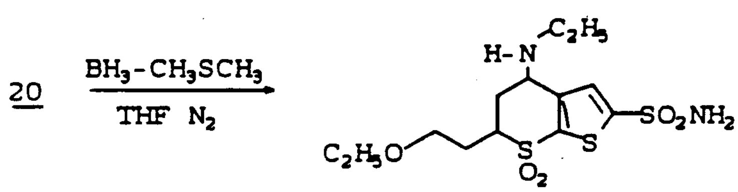


21

To a stirred solution of 4-acetamido-5,6-dihydro-6-(2-ethoxyethyl)-4H-thieno[2,3-b]thiopyran-2-sulfonamide-7,7-dioxide (6.8 g, 0.017 mol) in tetrahydrofuran (20 ml) heated under nitrogen to reflux was added borane-methyl sulfide (17 ml, 0.170 mol) dropwise over 1/2 hr. The reaction was heated at reflux for 2 hr. while allowing the methyl sulfide to distill off. Then the mixture was concentrated in vacuo to a yellow gum. Then 6N HCl (30 ml) was added followed by water (30 ml) and the mixture was heated on the steam bath for 1/4 hour. The solution was basified with excess sodium bicarbonate and was extracted with ethyl acetate (300 ml). The aqueous solution was again extracted with ethyl acetate. The combined extracts were washed with saturated sodium chloride, dried, filtered and concentrated in vacuo to obtain a white foam (5.3 g) mixture of cis and trans isomers. Chromatography on silica gel gave 2.28 g of the a-isomer and 0.17 g of the β-isomer.

a-isomer (trans)

The hydrochloride of the a-isomer was prepared by dissolving 0.19 g of the a-isomer in ethanol (3 ml) and adding 6N ethanolic HCl (0.1 ml). The white HCl salt (0.21 g), mp. 135-138C. was collected by filtration.

β-isomer (cis)

The hydrochloride of the β-isomer was prepared by dissolving 2.44 g of the β-isomer in ethanol (15 ml) and adding 6N ethanolic HCl (1.2 ml). The white solid (2.0 g), mp dec. > 128°C. was collected by filtration.

EXAMPLE 3

Enantiomers of 5,6-dihydro-6-(2-ethoxyethyl)-4-ethylamino-4H-thieno-[2,3-b]thiopyran-2-sulfonamide-7,7-dioxide

Step A: Preparation of the trans, (-) rotatory enantiomer.

21 (trans-isomer)

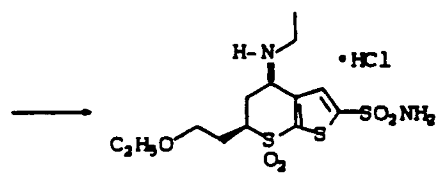

21 (trans-isomer) (-) rotatory enantiomer

The trans-isomer racemate (3.37 g, 0.009 mol) was dissolved in acetonitrile (50 ml) at reflux and di-p-toluoyl-D-tartatric acid monohydrate (0.88 g, 0.002 mol) was added. The solution was left overnight. Then it was cooled and filtered to obtain 1.4 g. This material was recrystallized from acetonitrile and 1.16 g of the pure salt was recovered, mp 179-181°C dec. [a]$^{25}_{D}$ = (+) 34.8 ($CH_3OH$).

The free base was prepared by shaking the salt with ethyl acetate (25 ml) and saturated sodium bicarbonate (10 ml). The ethyl acetate solution was washed with saturated sodium chloride, dried, filtered and concentrated in vacuo to obtain 0.78 g of colorless oil which solidfied to a white solid, mp 123-125°C. [a]$^{25}_{D}$ = (-) 23.4 ($CH_3OH$).

The hydrochloride salt was prepared by dissolving 0.74 g of the free base in warm ethanol (10 ml) and adding 0.33 ml of 6N ethanolic HCl. Then the solution was diluted with an equal volume of ether. After cooling the mixture was filtered to yield 0.74 g of white solid, mp 185-187.5°C dec.
[a]$^{25}_{D}$ = (-) 5.5 ($CH_3OH$)

Step B: Preparation of the trans, (+)rotatory enantiomer.

21 (trans-isomer)

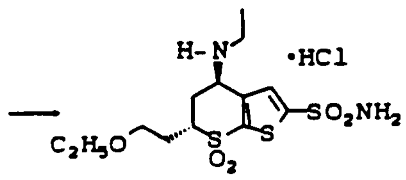

(trans-isomer)
21 (+) rotatory enantiomer

The trans-isomer enriched in the (+) rotatory enantiomer (1.0 g, 0.0026 mol) was dissolved in hot acetonitrile (10 ml) and di-p-toluoyl-L-tartaric acid monohydrate (0.48 g, 0.0012 mol) was added. The solution was left at room temperature overnight. Then the mixture was cooled and filtered to obtain 0.8 g, of salt, mp 175-177°C which upon recrystallization from acetonitrile gave 0.57 g, mp 178-180°C.
$[a]_D^{25}$ = (-) 34.8 ($CH_3OH$).

The free base was prepared by shaking this material with ethyl acetate (25 ml) and saturated sodium bicarbonate. The ethyl acetate extract was washed with saturated sodium chloride, dried, filtered and concentrated in vacuo to a viscous oil which solidified to a white solid (0.42 g), mp 123-125°C. $[a]_D^{25}$ = (+) 23.4 ($CH_3OH$).

The hydrochloride salt was prepared by dissolving 0.35 g of the free base in ethanol (7 ml) and adding 6N ethanolic HCl (0.16 ml). Dilution with an equal volume of ether and refrigeration yielded a white solid (0.39 g), mp. 185-188°C dec. $[a]_D^{25}$ =(+) 5.2 ($CH_3OH$).

Step C: Preparation of the cis (+) rotatory enantiomer.

21(cis-isomer)

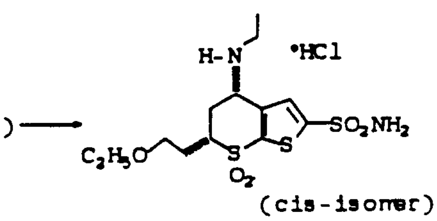

(cis-isomer)
21 (+)rotatory enantiomer

The cis-isomer racemate (1.3g, 0.0034 mol) was dissolved in acetonitrile (10 ml) and di-p-toluoyl-D-tartaric acid (0.34g, 0.00084 mol) was added. Solid precipitated immediately. The salt was redissolved by refluxing with 40 ml of acetonitrile and 10 ml of ethanol and was left to crystallize slowly overnight. Filtration gave 0.6g. of the salt. Recrystallization from acetonitrile gave 0.46g of the pure salt, mp 209-209-5°C, with decomposition.

The free base was prepared by shaking 0.87g (0.667 mmol) of the salt with 10 ml of saturated sodium bicarbonate and extracting with 25 and 2 x 15 ml of ethyl acetate. The combined ethyl acetate extracts were washed with saturated NaCl solution, dried, filtered and concentrated in vacuo at room temperature. There was recovered 0.52g of the free base as a colorless oil.

The hydrochloride salt was prepared using ethanolic hydrogen chloride in ethanol (7 ml) and there was obtained 0.44g of white solid, mp 198-202°C.
$[a]_D^{25}$ = (+) 57.64 ($CH_3OH$)

Step D: Preparation of the cis (-) rotatory enantiomer.

21 (cis-isomer)

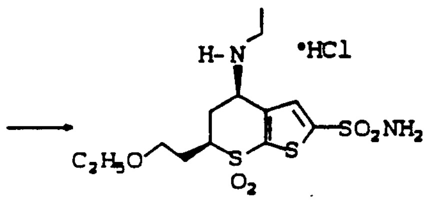

(cis-isomer)

21 (-)rotatory enantiomer

The cis-isomer enriched in the (-) rotatory enantiomer (0.82g and containing 0.0017 mol of the (-) rotatory enantiomer) was dissolved in acetonitrile (10 ml) and di-p-toluoyl-L-tartaric acid (0.34g, 0.00085 mol) was added. An immediate precipitation occurred. The mixture was heated with 40 ml of acetonitrile and 15 ml of methanol and the solution was left to crystallize slowly overnight. The mixture was filtered and 0.47g of white solid was recovered, mp 214-215.5°C, with decomposition. A second crop of 0.38g was obtained which was recrystallized from 10 ml of acetonitrile -$H_2O$ (1:1) to give 0.24g, mp 212-213°C.

The free base was prepared by dissolving 0.71g of the diastereomeric salt in 15 ml of saturated sodium bicarbonate and 15 ml of ethyl acetate. The ethyl acetate was separated and the aqueous phase was reextracted with 2 x 15 ml of ethyl acetate. The ethyl acetate extracts were combined and washed with saturated NaCl, dried, filtered and concentrated in vacuo at room temperature. The free base was obtained as a white solid, 0.45g.

The hydrochloride salt was prepared by dissolving 0.45g of the free base in 7 ml of hot ethanol and adding a slight excess of ethanolic hydrogen chloride/ether (10 ml) was added and the white solid was collected: 0.39g, mp 198-202°C. $[a]_D^{25}$ = (-) 58.3 ($CH_3OH$)

EXAMPLE 4

Trans and cis 5,6-dihydro-6-ethoxymethyl-4-ethyl-amino-4H-thieno-[2,3-b]thiopyran-2-sulfonamide-7,7-dioxide

Step A: Preparation of 2-(2-thienylthio)succinic acid

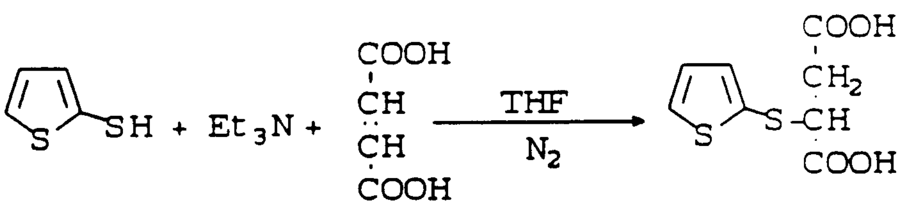


22

To a stirred solution of maleic acid (6.38g, 0.055 mol) in tetrahydrofuran (50 ml) under a nitrogen atmosphere was added 2-thiophenethiol (5.0 ml, 0.055 mol) and triethylamine (14.2g, 0.14 mol). The mixture was stirred at gentle reflux for 16 hours overnight. The solvent was removed in vacuo and the residual oil was poured into 3N HCl (200 ml). The product was extracted into ethyl acetate (125 ml) in three portions, washed with saturated NaCl solution and dried over $Na_2SO_4$. The solution was filtered and concentrated in vacuo to give the product as a light beige solid, 11.9g, mp 136-138.5°C of 95% purity by HPLC. Yield was 93%.

Step B: Preparation of 5,6-dihydro-4-oxo-4H-thieno [2,3-b]thiophene-6-carboxylic acid

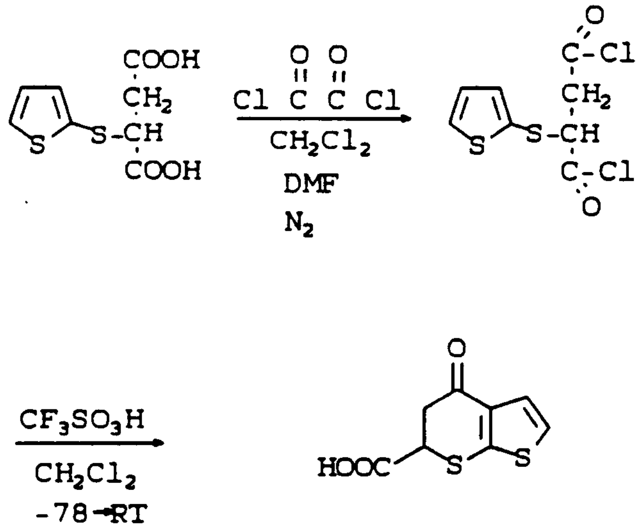


23

To a stirred suspension of 2-(2-thienylthio) succinic acid (75.5g, 0.325 mol) in methylene chloride (500 ml) under nitrogen atmosphere was added dimethylformamide (3 ml) followed by the dropwise addition of oxalyl chloride (70.7 ml, 0.81 mol) over a 1/2 hour period. The mixture was stirred at ambient temperature for 2 1/2 hours and the resulting solution was concentrated in vacuo to a brown oil. Then 1/2 of this oil was dissolved in methylene chloride (200 ml), cooled to about -78°C and stirred as trifluoromethanesulfonic acid (50 g, 0.33 mol) was added dropwise over 5 minutes. After 1/4 hour at -78°C, the cooling bath was removed and the temperature was allowed to rise to room temperature. After 4 3/4 hours, the mixture was poured into ice and water. Methylene chloride (400 ml) was added and filtered to obtain product as a pale gray solid (4.1 g). The methylene chloride layer was separated, washed with $H_2O$, dried over $Na_2SO_4$, filtered and concentrated in vacuo to a black gum. The gum was dissolved in ethyl acetate (150 ml). This solution was extracted with 10 x 50 ml of 0.25 N KOH. The individual extracts were acidified and solids were filtered and dried. Total product obtained was 19 g or 55% yield. Pure product melted at 182.5 - 184°C.

Step C: Preparation of N,N-dimethyl-4-oxo-4H-thieno[2,3-b]thiopyran-6-carboxamide

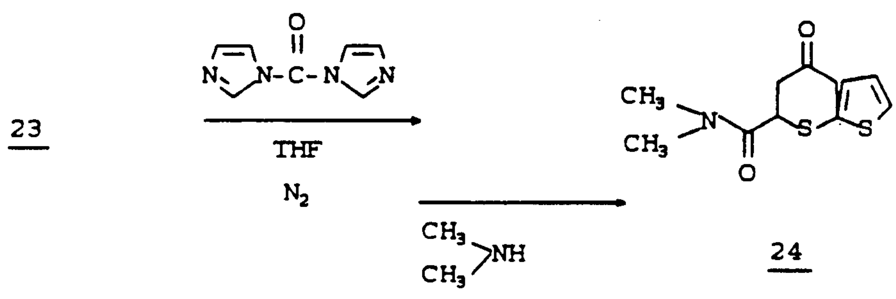


23

24

The reaction was run under a nitrogen atmosphere. To a stirred solution of 4-oxo-4H-thieno[2,3-b]thiopyran-6-carboxylic acid (10.7 g, 0.05 mol) in tetrahydrofuran (50 ml) was added carbonyldiimidazole (8.9 g., 0.055 mol). The mixture was stirred at ambient temperature for 3/4 hours. Anhydrous dimethylamine was bubbled into the thick suspension at 0°C until an excess was present. The resulting solution was stirred at 0°C for 3/4 hours and the solvent was removed in vacuo. The residual oil was diluted with $H_2O$ (50 ml) and the solid which separated was filtered and dried to give 7.14

g of product, mp 126.5-128° of 97% purity by HPLC. The aqueous filtrate was concentrated in vacuo and the residual gum was chromatographed on silica gel (200 g) using 10% methanol in chloroform. An additional 3.15 g of impure product was recovered. Yield was about 80%.

Step D: Preparation of 5,6-dihydro-N,N-dimethyl-4-hydroxy-4H-thieno[2,3-b]thiopyran-6-carboxamide

24

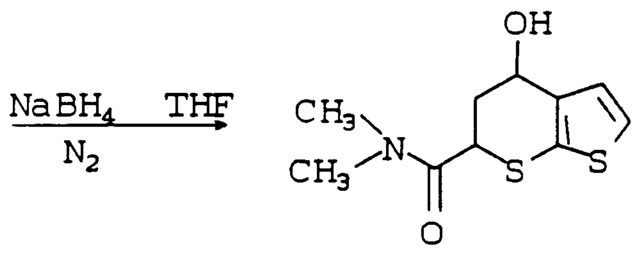


25

5,6-Dihydro-N,N-dimethyl-4-oxo-4H-thieno-[2,3-b]thiopyran-6-carboxamide (26.2 g, 0.109 mol) was stirred in tetrahydrofuran (250 ml) at room temperature under nitrogen and sodium borohydride (8.25 g, 0.218 mol) was added. The mixture was stirred at ambient temperature for 4 hours. Then it was cooled in ice and ethyl acetate (100 ml) was added followed by the dropwise addition of 3N HCl (100 ml). The aqueous layer was separated and extracted with ethyl acetate (50 ml). The combined organic solutions were washed with saturated sodium chloride and saturated sodium bicarbonate, dried over $NaSO_4$, filtered and concentrated in vacuo at room temperature. The product, 25, was essentially pure by TLC. Yield was quantitative.

Step E: Preparation of 5,6-dihydro-N,N-dimethyl-4-methoxyethoxyethoxy-4H-thieno[2,3-b]thiopyran-6-carboxamide

25

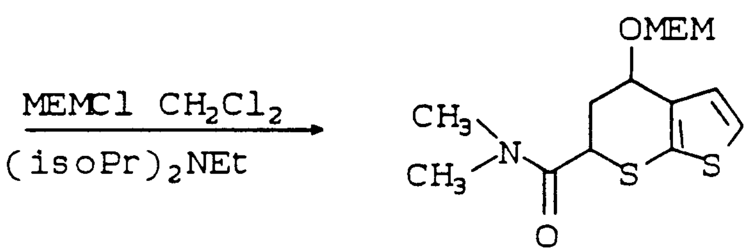


26

To a stirred solution of 5,6-dihydro-N,N-dimethyl-4-hydroxy-4H-thieno[2,3-b]thiopyran-5-carboxamide, 25, (63.4 g, 0.26 mol) in methylene chloride 500 ml) was added dissopropylethylamine (40.3 g, 0.312 mol) followed by MEM chloride (38.9 g, 0.312 mol). The mixture was stirred at ambient temperature overnight. The dark solution was poured into ice and water (500 ml). The aqueous layer was separated and was extracted with methylene chloride (100 ml). The methylene chloride solutions were combined, washed with cold 1.5N HCl, saturated $NaHCO_3$, and water, dried, filtered and concentrated in vacuo at room temperature. A dark amber oil was obtained which solidified to a brown solid upon standing. Crude yield of 26 was 79.7 g (92%).

Step F: Preparation of 5,6-dihydro-6-dimethylaminomethyl-4-methoxyethoxymethoxy-4H-thieno-[2,3-b]thiopyran

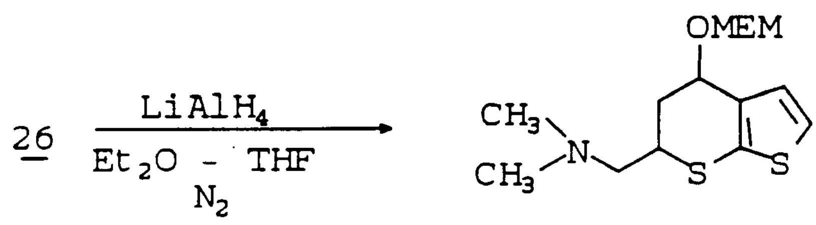


27

To a stirred suspension of lithium aluminum hydride (11.4 g, 0.20 mol) in anhydrous ether (200 ml) under nitrogen and cooled in ice was added a solution of 5,6-dihydro-N,N-dimethyl-4-methoxyethoxymethoxy-4H-thieno[2,3-b]thiopyran-6-carboxamide (80 g, 0.28 mol), 26, in dry tetrahydrofuran (150 ml) dropwise over 1 1/2 hours. Stirring was continued at ice bath temperature overnight. The suspension was cooled in ice and quenched under nitrogen by adding dropwise water (12 ml), 20% sodium hydroxide (9 ml) and water (42 ml). Then additional ether (100 ml) was added and the mixture was filtered. The solids were washed with ether and with ethyl acetate. The filtrate and washings were washed with saturated sodium chloride (3 x 50 ml), dried over $Na_2SO_4$, filtered and concentrated in vacuo at room temperature. A viscous oil was obtained (66.9 g). Crude yield of 27 was 88%.

Step G: Preparation of 5,6-dihydro-6-dimethylaminomethyl-4-methoxyethoxymethoxy-4H-thieno[2,3-b]thiopyran-2-sulfonamide

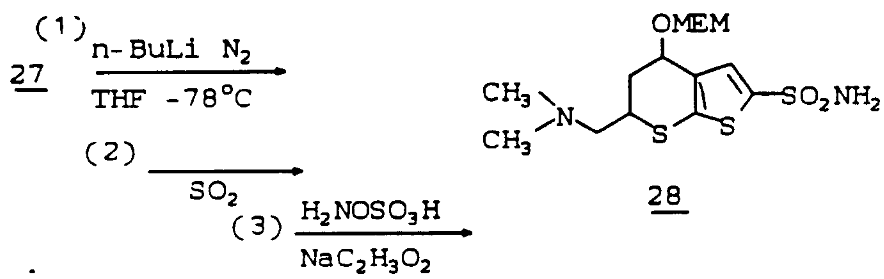


28

5,6-Dihydro-6-dimethylaminomethyl-4-methoxyethoxymethoxy-4H-thieno[2,3-b]thiopyran, 27 (66.9 g, 0.21 mol) was dissolved in dry tetrahydrofuran (400 ml) under nitrogen and the solution was cooled to -78°C. Then n-butyl lithium (100.8 ml), 0.252 mol of 2.5 M in hexane) was added dropwise with stirring over 1 1/2 hours. Stirring was continued for another 1 1/2 hours at -78°C and then anhydrous $SO_2$ was bubbled over the surface of the solution until the mixture became acidic (about 1 hour). The mixture was stirred at -78°C for 2 hours and then warmed to room temperature over 1/2 hour. The resulting solution was concentrated in vacuo, to remove excess $SO_2$ and tetrahydrofuran. The residual lithio salt was taken up in water (300 ml) containing sodium acetate (58.4 g, 0.59 mol). Hydoxylamine-O-sulfonic acid (59.4 g, 0.525 mol) was added followed by additional sodium acetate (16 g, 0.2 mol) to adjust to pH 5.0. The mixture was stirred at room temperature overnight and then was cooled in ice, basified with sodium bicarbonate and filtered. The filtrate was extracted with 20% methanol-chloroform (500 and 2 x 200 ml) and the solids were washed free of product with 20% methanol-chloroform. These solutions were combined, washed with saturated sodium chloride, dried, filtered and concentrated in vacuo. The product was obtained as an oil (70.9 g). Yield of 28 was 85%. Recrystallization from ethanol gave material with mp 132-134°C.

Step H : Preparation of 5,6-dihydro-6-methylene-4-methoxyethoxymethoxy-4H-thieno[2,3-b]thiopyran-2-sulfonamide-7,7-dioxide

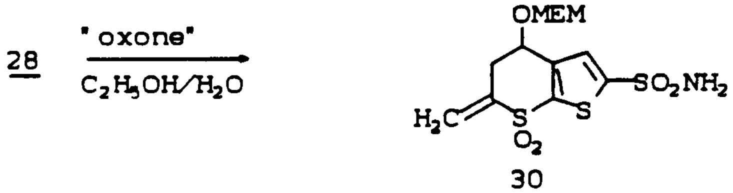


30

5,6-Dihydro-6-dimethylaminomethyl-4-methoxyethoxymethoxy-4H-thieno[2,3-b]thiopyran-2-sulfonamide (39.7 g, 0.10 mol) was dissolved in ethanol (300 ml) with warming. The solution was diluted with water (150 ml), cooled to 20°C and Oxone® (92.2 g, 0.15 mol) was added. The mixture was stirred at ambient temperature for 3 hours. Additional Oxone® (25 g, 0.04 mol) was added and stirring was continued for 1 1/2 hours. Then the mixture was neutralized by pouring into ethyl acetate (300 ml) and sodium bicarbonate (82 g, 1.0 mol). The mixture was filtered and the solids were washed with ethyl acetate. The filtrate and washing were concentrated in vacuo at room temperature. The oily residue was taken up in ethyl acetate and the solution was washed with saturated sodium bicarbonate and saturated sodium chloride, dried, filtered and concentrated in vacuo at room temperature. The product was a viscous amber oil (31.4 g). Yield of 30 was 82%

Step I: Preparation of 5,6-dihydro-6-ethoxymethyl-4-methoxyethoxymethoxy-4H-thieno[2,3-b]thiopyran-2-sulfonamide-7,7-dioxide

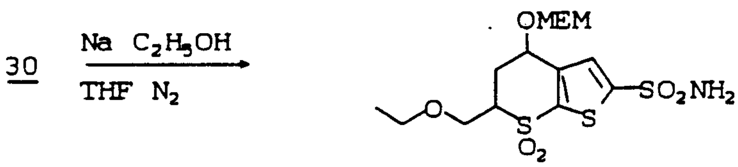


31

Sodium spheres (2.0 g 0.088 mol) were added to dry ethanol (100 ml) under nitrogen. The mixture was stirred until the sodium had reacted completely (1/2 hr). This sodium ethoxide solution was added over several minutes to a cold solution of 5,6-dihydro-6-methylene-4-methoxyethoxymethoxy-4H-thieno[2,3-b]thiopyran-2-sulfonamide 30, (15.3 g, 0.04 mol) in tetrahydrofuran (50 ml). The resulting solution was stirred at ambient temperature for 1 hour. Then the mixture was acidified with 6N HCl (15 ml, 0.09 mol) and saturated sodium bicarbonate (25 ml) was carefully added. The basic solution was concentrated in vacuo and the oily residue was taken up in chloroform (150 ml) and water (50 ml). The aqueous layer was separated and extracted with chloroform (2 x 40 ml). The combined chloroform solutions were washed with water, dried, filtered and concentrated in vaco. An amber gum was obtained (15.5 g). Yield of 31 was 89%.

Step J: Preparation of 5,6-dihydro-6-ethoxymethyl-4-hydroxy-4H-thieno[2,3-b]thiopyran-2-sulfonamide-7,7-dioxide

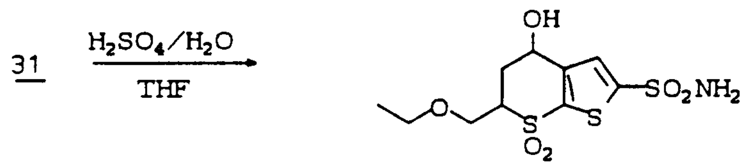


32

5,6-Dihydro-6-ethoxymethyl-4-methoxyethoxymethoxy-4H-thieno[2,3-b]thiopyran-2-sulfonamide-7,7-dioxide, 31, (15.5 g, 0.036 mol) was dissolved in tetrahydrofuran (100 ml) and the solution was stirred at 0°C as sulfuric acid-water (200 ml of 1/1 volume/volume) was added dropwise over 3/4 hour. The mixture was stirred at ambient temperture for 2 3/4 hours. Then it was poured into ethyl acetate (300 ml), ice and sodium bicarbonate (336 g, 4.0 mol) with stirring. The neutralized mixture was filtered and the solids were washed with ethyl acetate (500 ml). The aqueous layer was separated and was extracted with ethyl acetate (2 x 75 ml). The combined ethyl acetate solutions were washed with saturated sodium chloride, dried, filtered and concentrated in vacuo. The product was a pale tan solid foam (10.9 g). Yield of 32 was 89%.

Step K: Preparation of 5,6-dihydro-6-ethoxymethyl-4-ethylamino-4H-thieno[2,3-b]thiopyran-2-sulfonamide-7,7-dioxide (cis and trans isomers)

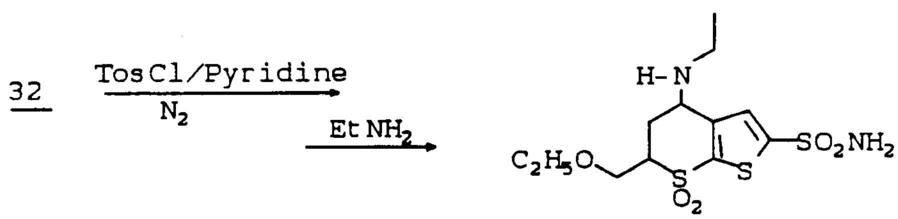


33

5,6-Dihydro-6-ethoxymethyl-4-hydroxy-4H-thieno[2,3-b]thiopyran-2-sulfonamide-7,7-dioxide (4.25 g, 0.0123 mol) was dissolved in dry pyridine (25 ml) and the solution was cooled to -10°C . p-Toluenesulfonyl chloride (5.14 g, 0.027 mol) was added and the mixture was stirred at -10°C for 5 hours. Anhydrous ethylamine (26 ml, 0.4 mol) was added and the mixture was stirred for 2 hours at room temperature. Finally, 70% ethylamine (32 ml, 0.40 mol) was added and the mixture was heated at 50°C overnight. The dark mixture was concentrated in vacuo and the residual oil was taken up in ethyl acetate (100 ml) and saturated sodium bicarbonate (100 ml). The aqueous layer was separated and extracted with ethyl acetate. The combined ethyl acetate solutions were washed with saturated sodium chloride, dried, filtered and concentrated in vacuo. The oil obtained was a 2:1 mixture of cis and trans isomers which were separated by silica gel chromatography using 7.5 % methanol-chloroform. Total recovery was 2.43 g. Yield of 33 was 54%.

α-isomer

The less polar trans isomer was a waxy solid. The HCl salt melted at 145-148°C, dec.

β-isomer

The more polar cis isomer was a white solid, mp. 154-157°C. The HCl salt melted at 229-231°C, dec.

EXAMPLE 5

Enantiomers of 5,6-dihydro-6-ethoxymethyl-4-ethylamino-4H-thieno[2,3-b]thiopyran-2-sulfonamide-7,7-dioxide

Step A: Preparation of the trans, (-) rotatory enantiomer

33 (trans-isomer)

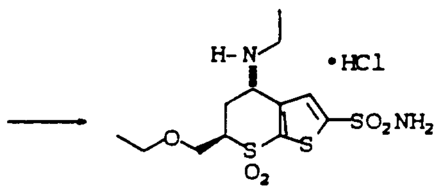

33 (trans-isomer) (-) rotatory enantiomer

Racemic trans ($\alpha$-isomer)-5,6-dihydro-6-ethoxymethyl-4-ethylamino-4H-thieno[2,3-b]thiopyran-2-sulfonamide-7,7-dioxide (1.15 g, 3.12 mmol) was dissolved in hot acetonitrile (10 ml) and di-p-toluoyl-L-tartaric acid monohydrate (0.325 g, 0.78 mmol) was added. The solution was left at room temperature ovenight. Filtration gave a white solid salt (0.83 g). Three recrystallyzations from acetonitrile gave 0.32 g of pure salt, $[\alpha]^{25}_{D}$ = (-) 57.5 ($CH_3OH$).

The free base was prepared by shaking 0.76 g of the salt with ethyl acetate (25 ml) and saturated sodium bicarbonate (10 ml). The aqueous layer was separated and extracted with ethyl acetate. The combined ethyl acetate solutions were washed with saturated sodium chloride, dried, filtered and concentrated in vacuo to a colorless oil (0.5 g), $[\alpha]^{25}_{D}$ = (-) 25.2 ($CH_3OH$).

The oil was converted to the hydrochloride salt by dissolving in ethanol (5 ml) and adding 6N ethanolic HCl (0.22 ml). The mixture was cooled and filtered to obtain the hydrochloride salt as a white solid (0.43 g), mp. 202.5-204°C, $[\alpha]^{25}_{D}$ = (-) 1.7 ($CH_3OH$).

Step B: Preparation of the trans, (+) rotatory enantiomer.

33 (trans-isomer)

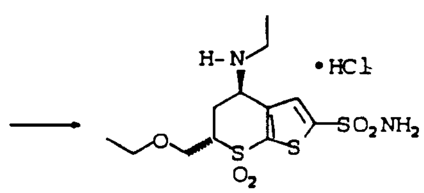

33 (trans-isomer) (+) rotatory enantiomer

The trans-isomer of 5,6-dihydro-6-ethoxymethyl-4-ethylamino-4H-thieno[2,3-b]thiopyran-2-sulfonamide-7,7-dioxide enriched in the (+)-rotatory enantiomer (0.87 g, 24 mmol) was dissolved in hot acetonitrile (10 ml) and di-p-toluoyl-D-tatric acid (0.48 g, 1.2 mmol) was added. The solution was left at room temperature overnight. Filtration gave a white solid salt (0.65 g). Recrystallization from acetonitrile gave 0.56 g of pure salt; $[\alpha]^{25}_{D}$ = (+) 58.5 ($CH_3OH$).

The free base was prepared by shaking the salt in ethyl acetate (25 ml) and saturated sodium bicarbonate (10 ml). The aqueous layer was separated and extracted with ethyl acetate. The combined ethyl acetate solutions were washed with saturated sodium chloride, dried, filtered and concentrated in vacuo to a colorless oil (0.42 g).

The oil was converted to the hydrochloride salt by dissolving in ethanol (7 ml) and adding 6N ethanolic HCl (0.20

ml). The mixture was cooled, diluted with ether (40 ml) and filtered to obtain the hydrochloride salt as a white solid (0.36 g, mp 202-204°C; $[\alpha]_D^{25}$ = (+) 1.2 ($CH_3OH$).

EXAMPLE 6

5,6-Dihydro-6-methoxymethyl-4-(n-propylamino)-4H-thieno[2,3-b]thiopyran-2-sulfonamide-7,7-dioxide (trans-isomer, and cis-isomer)

Step A: Preparation of 5,6-dihydro-4-methoxyethoxymethoxy-6-methoxymethyl-4H-thieno[2,3-b]thiopyran-2-sulfonamide-7,7-dioxide.

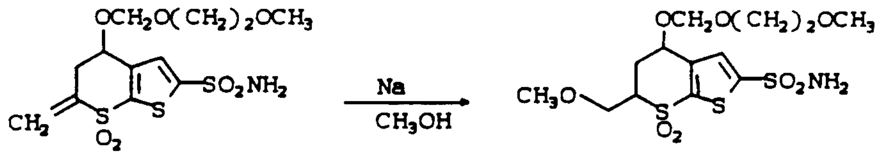


34 35

Sodium (1.7 g, 0.075 m) was added portionwise to absolute methanol (150 ml) under nitrogen with stirring. After solution was effected, the solution was added to 5,6-dihydro-4-methoxyethoxymethoxy-6-methylene-4H-thieno[2,3-b]thiopyran-2-sulfonamide-7,7-dioxide (12.43 g, 0.032 m) dissolved in methanol (55 ml) with stirring under nitrogen. After 20 hours, the reaction mixture was cooled in ice, acidified with 6N hydrochloric acid (19 ml) and then basified with saturated sodium bicarbonate solution. The mixture was concentrated in vacuo to remove methanol and the aqueous oil suspension was extracted with ethyl acetate (2 x 150 ml). After washing with saturated sodium bicarbonate solution and with saturated sodium chloride solution and drying over sodium sulfate, the solvent was evaporated in vacuo to yield a viscous oily product weighing 12.40 g (93%) which was used in the next step without further purification.

Step B: Preparation of 5,6-dihydro-4-hydroxy-6-methoxymethyl-4H-thieno[2,3-b]thiopyran-2-sulfonamide-7,7-dioxide.

35

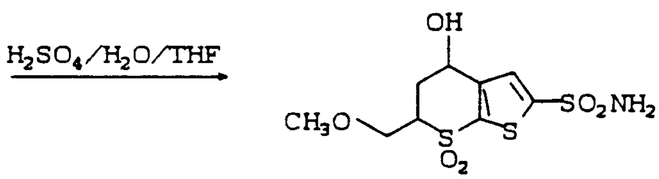


36

A solution of 5,6-dihydro-4-methoxyethoxymethoxy-6-methoxymethyl-4H-thieno[2,3-b]thiopyran-2-sulfonamide-7,7-dioxide (12.4 g, 0.030 m) in tetrahydrofuran (90 ml) was cooled to -5°C and stirred while a solution of concentrated sulfuric acid (90 ml) in water (90 ml) was added dropwise over 45 minutes while maintaining the temperature below 5°C. After stirring at -5°C for 1 hour and at ambient temperature for 3 hours, the mixture was added to a stirred suspension of sodium bicarbonate (300 g) in ethyl acetate (360 ml) and ice. After 30 minutes with periodic additions of saturated sodium bicarbonate solution to render the mixture basic, it was filtered and the solids were washed thrice with ethyl acetate. The combined filtrate and washings were washed with water, dried over sodium sulfate and evaporated in vacuo to yield 7.79 g, (79%) of amorphous product which was used in the succeeding step without further purification.

Step C: Preparation of trans and cis-5,6-dihydro-6-methoxymethyl-4-(n-propylamino)-4H-thieno[2,3-b]thiopyran-2-sulfoanmide-7,7-dioxide (α-Isomer, and β-Isomer)

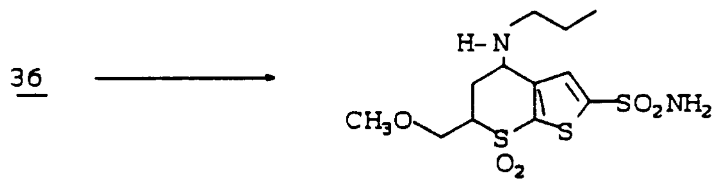

37

A stirred solution of 5,6-dihydro-4-hydroxy-6-methoxymethyl-4H-thieno[2,3-b]thiopyran-2-sulfonamide-7,7-dioxide (3.50 g, 0.011 m) in dry pyridine (25 ml) was cooled to -10°C under nitrogen while p-toluenesulfonyl chloride (4.77 g, 0.025 m) was added in one portion. After stirring at -10°C for 5 hours, the mixture was further cooled to -20°C and n-propylamine (18.3 g, 0.31 m) was added dropwise while maintaining temperature below 0°C. The mixture was stirred at ambient temperature for 1.5 hours and then at 50°C for 19 hours. The reaction mixture was concentrated in vacuo and the residue was distributed between ethyl acetate (200 ml) and saturated sodium bicarbonate solution (100 ml). The aqueous layer was separated and extracted with ethyl acetate (2 x 100 ml). The combined ethyl acetate extracts were washed twice with water and concentrated to approximately 75 ml in vacuo. The solution was extracted with 3N hydrochloric acid (2 x 50 ml) and washed with water (50 ml). The combined acid extracts and water wash were basified with sodium bicarbonate and extracted with ethyl acetate (3 x 250 ml). The combined extracts were washed thrice with water, dried over sodium sulfate and evaporated in vacuo to yield a tan solid residue of the mixture of isomers weighing 2.20 g (54%).

The residue was chromtographed on silica gel on a 100 mm diameter Still column, eluting initially with 2900 ml chloroform/methanol/ammonium hydroxide, 95:5:0.5, followed by chloroform/methanol/ammonium hydroxide, 90:10:1 to yield 0.39 g, of the α-isomer (trans-) and 1.00 g of the β-isomer (cis). The trans-isomer (0.39, 0.0011 m) was dissolved in absolute ethanol (10 ml), 4.8 N ethanolic hydrochloric acid (0.3 ml) was added and the solution was diluted to incipient turbidity with anhydrous ether to yield 0.26 g of the hydrochloride salt melting with decomposition at approximately 150°C.

| Anal. Calcd. for $C_{12}H_{20}N_2O_5S_3 \cdot HCl$: | | | |
|---|---|---|---|
| | C, 35.39; | H, 5.23; | N, 6.92; |
| Found | C, 35.70; | H, 5.28; | N, 6.64. |

The cis-siomer (1.00 g, 0.0027 m) was dissolved in absolute ethanol (25 ml) and absolute methanol(10 ml) with warming, the solution was concentrated to approximately 15 ml. 4.8 N ethanolic hydrochloric acid (0.8 ml) was added and the solution was diluted to incipient cloudiness with anhdyrous ether to yield 0.81 g of the hydrochloride salt melting at 218-221°C.

| Anal. Calcd. for $C_{12}H_{20}N_2O_5S_3 \cdot HCl$: | | | |
|---|---|---|---|
| | C, 35.39; | H, 5.23; | N, 6.92; |
| Found | C, 35.25; | H, 5.26; | N, 6.90. |

PMR studies showed the α-isomer to have a trans configuration and the β-isomer to be cis

EXAMPLE 7

5,6-Dihydro-6-ethoxymethyl-4-(n-propylamino)-4H-thieno[2,3-b]thiopyran-2-sulfonamide-7,7-dioxide trans-isomer, and cis-isomer)

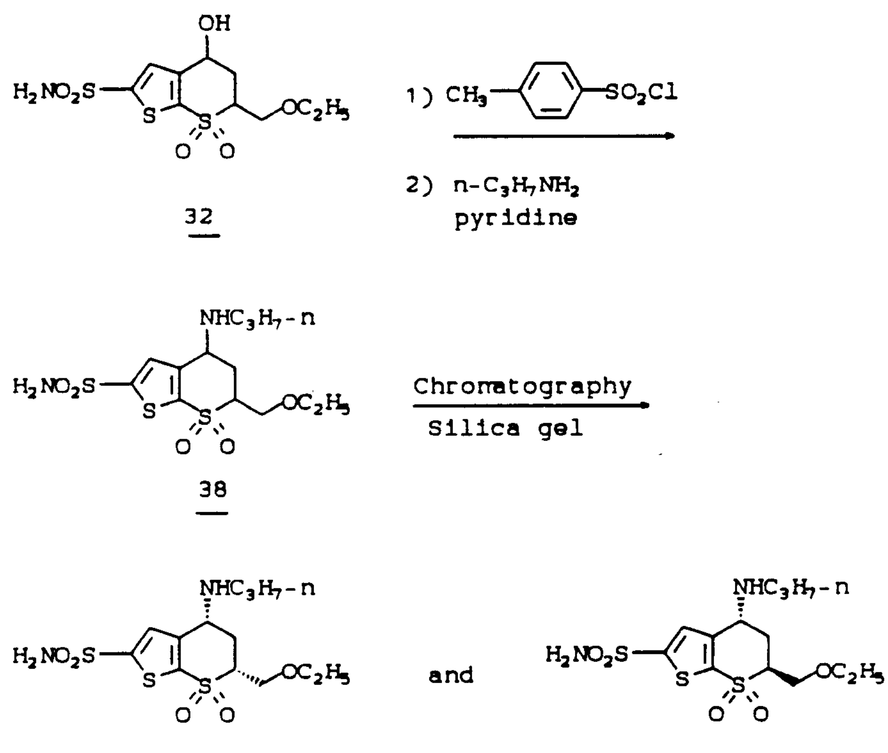


32

38

β-Isomer and 38 α-Isomer

A stirred solution of 5,6-dihydro-6-ethoxymethyl-4-hydroxy-4H-thieno[2,3-b]thiopyran-2-sulfonamide-7,7-dioxide prepared as described in Example 6 for preparation of the corresponding 6-methoxyethyl analog (4.00 g, 0.0017 m) in dry pyridine (25 ml) was cooled to -10°C under nitrogen while p-toluene-sulfonyl chloride (4.96 g. 0.026 m) was added in one portion. After stirring at -10°C for 5 hours, the mixture was further cooled to -20°C and n-propylamine (19.6 g, 0.33 m) was added dropwise while maintaining the temperature below 0°C. The mixture was stirred at ambient temperature for 1 hour and then at 50°C for 18 hours. The reaction mixture was concentrated in vacuo and the residue was distributed between ethyl acetate (200 ml) and saturated sodium bicarbonate solution (100 ml). The aqueous layer was separated and extracted with ethyl acetate (2 x 100 ml). The combined ethyl acetate extracts were washed with water and concentrated to approximately 75 ml in vacuo. The solution was extracted with 3N hydrochloric acid (2 x 50 ml) and washed once with water. The combined acid extracts and water wash were basified with sodium bicarbonate and extracted with ethyl acetate (3 x 250 ml). After washing thrice with water and drying over sodium sulfate, the solvent was evaporated in vacuo to yield a red oily residue of the mixture of isomers weighing 3.18 g (71%).

The oily residue was chromatographed on silica gel on a 100 mm. diameter Still column, eluting initially with 2600 ml of chlorofrom/methanol/ammonium hydroxide, 95:5:0.5, followed by chloroform/methanol/ammonium hydroxide, 90:10;1. The fractions that contained a mixture of isomers were rechromatographed on an 80 mm, diameter Still column, eluting with chloroform/methanol/ammonium hydroxide, 95:5:0.5, to ultimately yield 0.69 g of the α-isomer and 1.53 g of the β-isomer.

The α-isomer (0.69 g, 0.0018 m) was dissolved in absolute ethanol (5 ml), 4.8 N ethanolic hydrochloric acid (0.5 ml) was added and the solution was diluted to incipient turbidity with anhydrous ether to yield 0.32 g of the hydrochloride salt melting at 123°C (d).

| Anal. Calcd. for $C_{13}H_{22}N_2O_5S_3 \cdot HCl$: | | | |
|---|---|---|---|
| | C, 37.27; | H, 5.53; | N, 6.69; |
| Found | C, 37.19; | H, 5.68; | N, 6.57. |

The β-isomer (1.53 g, 0.0040 m) was dissolved in absolute ethanol (10 ml), 4.8 N ethanolic hydrochloric acid (1.3 ml) was added and the solution was diluted to incipient turbidity with anhydrous ether to yield 1.52 g of the hydrochloride salt melting at 110°C (d).

| Anal. Calcd. for $C_{13}H_{22}N_2O_5S_3 \cdot HCl$: | | | |
|---|---|---|---|
| | C, 37.27; | H, 5.53; | N, 6.69; |
| Found | C, 36.95; | H, 5.63; | N, 6.59; |

PMR studies showed the α-isomer to have a trans configuration and the β-isomer to be cis.

The following compounds in Table I were prepared by employing essentially the same procedures as described in the examples indicated in Table I.

TABLE I

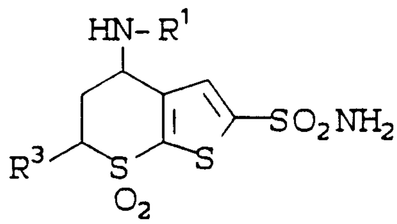

39

| Examples | $R^3$ | $R^1$ | Isomer | mp(°C) |
|---|---|---|---|---|
| 6 | $HOCH_2CH_2NHCH_2$- | $-CH_2CH_3$ | trans(+/-) | 190-192 |
| 6 | $HOCH_2CH_2NHCH_2$- | $-CH_2CH_3$ | cis(+/-) | 145-155 |
| 2,3 | $CH_3CH_2OCH_2CH_2$- | $-CH_2CH_2CH_3$ | trans (-) | 244.5-247 |
| 2,3 | $CH_3CH_2OCH_2CH_2$- | $-CH_2CH_2CH_3$ | trans (+) | 244-247 |
| 10 | $CH_3CH_2OCH_2CH_2CH_2$- | $-CH_2CH_3$ | trans (+/-) | 235-236 |
| 10 | $CH_3CH_2OCH_2CH_2CH_2$- | $-CH_2CH_3$ | cis (+/-) | 226-227 |
| 3 | $CH_3OCH_2CH_2CH_2$- | $-CH_2CH_3$ | cis (+) | 229-230 |
| 3 | $CH_3OCH_2CH_2CH_2$- | $-CH_2CH_3$ | cis (-) | 229-230 |
| 10 | $CH_3OCH_2CH_2CH_2$- | $-CH_3$ | trans (+/-) | 214-216 |
| 7 | $CH_3OCH_2CH_2CH_2$- | $-CH_3$ | cis (+/-) | 149-151 |
| 10 | $CH_3CH_2OCH_2CH_2CH_2$- | $-CH_2CH_3$ | trans (-) | 193-194 |
| 5 | $CH_3OCH_2CH_2OCH_2$- | $-CH_2CH_3$ | cis (+/-) | 205-207 |
| 5 | $CH_3OCH_2CH_2OCH_2$- | $-CH_2CH_3$ | trans (+/-) | 135-137 |
| 3 | $CH_3OCH_2CH_2OCH_2$- | $-CH_2CH_3$ | trans (+) | 207-209 |

| Examples | $R^3$ | $R^1$ | Isomer | mp(°C) |
|---|---|---|---|---|
| 3 | $CH_3OCH_2CH_2OCH_2-$ | $-CH_2CH_3$ | trans (-) | 208-210 |
| 3 | $CH_3CH_2OCH_2CH_2CH_2-$ | $-CH_2CH_3$ | trans (+) | 194-195 |
| 5, 3 | $CH_3CH_2CH_2OCH_2-$ | $-CH_2CH_3$ | trans (-) | 217-218 |
| 5, 3 | $CH_3CH_2CH_2OCH_2-$ | $-CH_2CH_3$ | trans (+) | 217.5-218.5 |
| 5 | $CH_3OCH_2CH_2OCH_2-$ | $-CH_2CH_2CH_3$ | trans (+/-) | 240-242 |
| 5 | $CH_3OCH_2CH_2OCH_2-$ | $-CH_2CH_2CH_3$ | cis (+/-) | 215-217 |
| 3 | $CH_3OCH_2CH_2OCH_2-$ | $-CH_2CH_2CH_3$ | trans (S,S) | 201-203 |

| Examples | $R^3$ | $R^1$ | Isomer | mp(°C) |
|---|---|---|---|---|
| 2 | $CH_3OCH_2CH_2OCH_2CH_2-$ | $-CH_2CH_3$ | trans (+/-) | 138-140 (maleate) |
| 3 | $CH_3OCH_2CH_2OCH_2CH_2-$ | $-CH_2CH_3$ | trans (-) | 98-105 (maleate) |
| 2 | $CH_3OCH_2CH_2OCH_2CH_2-$ | $-CH_2CH_3$ | trans (+) | 70-80 (maleate) |
| 2 | $CH_3OCH_2CH_2OCH_2CH_2-$ | $-CH_2CH_3$ | cis (+/-) | 155-158 (maleate) |
| 5 | $CH_3CH_2CH_2OCH_2-$ | $-CH_2CH_2CH_3$ | trans (+/-) | ~140 |
| 3 | $CH_3CH_2CH_2OCH_2-$ | $-CH_2CH_2CH_3$ | trans (-) | 201-206 |
| 3 | $CH_3CH_2CH_2OCH_2-$ | $-CH_2CH_2CH_3$ | trans (+) | --- |
| 20 | $CH_3CH_2OCH_2CH_2O-CH_2CH_2CH_2-$ | $-CH_2CH_2CH_3$ | trans (-) | 184-186 |
| 10 | $CH_3OCH_2CH_2O-CH_2CH_2CH_2-$ | $-CH_2CH_3$ | trans (+/-) | 184-182 |
| 10 | $CH_3OCH_2CH_2O-CH_2CH_2CH_2-$ | $-CH_2CH_3$ | cis (+/-) | 200-202 |
| 20 | $CH_3OCH_2CH_2O-CH_2CH_2CH_2-$ | $-CH_2CH_2CH_3$ | trans(-) | 180-182 |
| 20 | $CH_3OCH_2CH_2CH_2O-CH_2CH_2CH_2-$ | $-CH_2CH_2CH_3$ | trans(-) | 160-162 |

| $R^3$ | $R^1$ |
|---|---|
| $HO(CH_2)_2O(CH_2)_5$ | $-CH_2CH_2CH_3$ |
| $HOCH_2CH_2OCH_2-$ | $-CH_2CH_2CH_3$ |
| $HOCH_2CH_2S-CH_2-$ | $-CH_2CH_3$ |
| $CH_3CH_2CH_2OCH_2CH_2-$ | $-CH_2CH_3$ |
| $HOCH_2CH_2OCH_2CH_2-$ | $-CH_2CH_2CH_3$ |

EXAMPLE 8

Enantiomers of cis-5,6-dihydro-6-ethoxymethyl-4-(N-propylamino)-4H-thieno[2,3-b]thiopyran-2-sulfonamide-7,7-dioxide.

Step A: Preparation of the cis(+) enantiomer

To a solution of 5,6-dihydro-6-ethoxymethyl-4-(n-propylamino)-4H-thieno[2,3-b]thiopyran-2-sulfonamide-7,7-dioxide (cis,β-isomer),(3.5 g, 0.0092 m), in acetonitrile (60 ml) was added di-p-toluoyl-D-tartaric acid monohydrate (0.93, 0.0023 m). After standing overnight at ambient temperature, the white solid product was collected, dried and recrystallized twice more from acetonitrile-methanol to yield 2.1 g of resolved salt. The product was converted to the free base by treatment with saturated bicarbonate solution and extraction four times with ethyl acetate. The combined ethyl acetate extracts were washed with saturated sodium chloride, dried over sodium sulfate and concentrated in vacuo to yield 1.8 g of the base. Conversion to the hydrochloride salt utilizing ethanolic hydrogen chloride yielded a crystalline analytical sample melting at 160-161°C(d).

| Anal. Calc'd For $C_{13}H_{22}N_2O_5S_3$, HCl; | | | |
|---|---|---|---|
| | C, 37.27; | H, 5.53; | N, 6.69 |
| Found | C, 37.48; | H, 5.60; | N, 6.71 |

$[\alpha]_D^{25}$ = +59.24°, for the hydrochloride salt in methanol

Step B: Preparation of the cis(-) enantiomer

The combined mother liquors from the separation of the dextrorotatory enantiomer were concentrated in vacuo.

The residue was treated with saturated sodium bicarbonate solution and extracted five times with ethyl acetate. The combined extracts were washed with water, dried over sodium sulfate and evaporated in vacuo to yield 1.9g, of the free base. A solution of the base in acetonitrile (60 ml) was treated with di-p-toluoyl-L-tartaric acid (0.93g, 0.0024 m). After standing overnight at ambient temperature, the white solid product was collected, dried and recrystallized twice more from acetonitrile-methanol to yield 2.0g of resolved salt. The salt was converted to the free base by treatment with saturated sodium bicarbonate solution and extraction four times with ethyl acetate. After washing with water and drying over sodium sulfate, the solvent was evaporated in vacuo to yield 1.2g. of base. Conversion to the crystalline hydrochloride salt using ethanolic hydrogen chloride gave an analytical sample melting at 157-90°C (d).

| Anal. Calc'd for $C_{13}H_{22}N_2O_5S_3$. HCL: | | | |
| --- | --- | --- | --- |
| | C, 37.27; | H, 5.53; | N, 6.69 |
| Found | C, 37.41; | H, 5.86; | N, 6.34 |

$[\alpha]_D^{25}$ = -65.23°, for the hydrochloride salt in methanol

EXAMPLE 9

5,6-Dihydro-4-ethylamino-6-(2-methoxyethyl)-4H-thieno[2,3-b]thiopyran-2-sulfonamide-7,7-dioxide

Step A: Preparation of 5,6-Dihydro-4-(methoxyethoxymethoxy)-6-(2-methoxyethyl)-4H-thieno[2,3-b] thiopyran

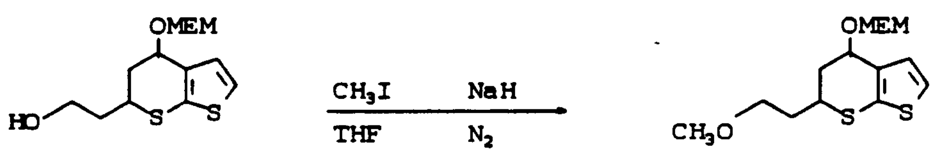


40

41

Sodium hydride (2.4g, 0.05 mol of 50% dispersion in mineral oil) was washed free of mineral oil using pet. ether under nitrogen and was suspended in dry tetrahydrofuran (25 ml). The suspension was cooled to 0°C and stirred as 11.2g (0.037 mol) of 5,6-dihydro-6-(2-hydroxyethyl)-4-(methoxyethoxymethoxy)-4H-thieno[2,3-b]thiopyran dissolved in dry tetrahydrofuran (25 ml) was added rapidly. Finally, 14.2g (0.10 mol) of methyl iodide was added and the mixture was stirred at ambient temperature overnight. The resulting white suspension was concentrated in vacuo at room temperature to remove the tetrahydrofuran. The pale yellow residue was taken up in ethyl acetate (150 ml) and water (50 ml). The ethyl acetate solution was separated, washed with saturated NaCl, dried, filtered and concentrated in vacuo to give the product as a pale yellow oil (11.2g, 95% yield).

Step B: Preparation of 5,6-Dihydro-4-(methoxyethoxymethoxy-6-(2-methoxyethyl)-4H-thieno[2,3-b]-thiopyran-2-sulfonamide

41

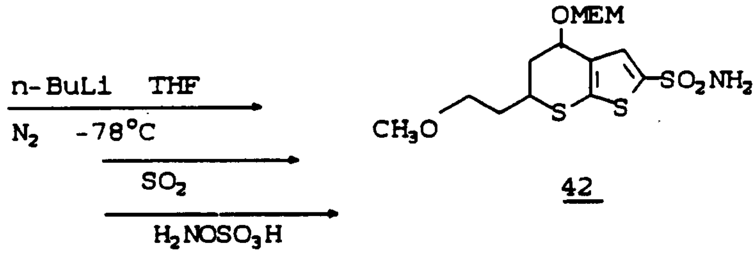


42

5,6-Dihydro-4-(methoxyethoxyethoxy)-6-(2-methoxyethyl)-4H-thieno[2,3-b]thiopyran (11.2g, 0.035 mol) was dissolved in dry tetrahydrofuran (100 ml) and the solution was cooled to -78°C under nitrogen. Then n-butyl lithium (16.8 mol, 0.042 mol of 2.5 m in hexane) was added dropwise over 1/2 hour. The solution was stirred at -78°C for an additional hour. Anhydrous sulfur dioxide was bubbled over the surface of the solution at -78°C to -40°C until the mixture became acidic. Stirring at -40 to -78°C was continued for 2 1/4 hours and then to room temperature over 1/2 hour. The solution was concentrated in vacuo. The residual lithio salt was dissolved in water (75 ml) containing sodium acetate (8.2g, 0.10 mol) and the solution was cooled to 0°C. Hydroxylamine-O-sulfonic acid (9.5g, 0.084 mol) was added and the mixture was stirred at room temperature overnight. The reaction mixture was diluted with saturated sodium bicarbonate (100 ml) and ethyl acetate (200 ml). The aqueous layer was separated and extracted with ethyl acetate. The ethyl acetate solutions were combined, washed with water, dried, filtered and concentrated in vacuo, to an amber liquid (13.1g 94%).

Step C: 5,6-Dihydro-4-(methoxyethoxymethoxy)-6-(2-methoxyethyl)-4H-thieno[2,3-b]thiopyran-2-sulfonamide-7,7-dioxide

42

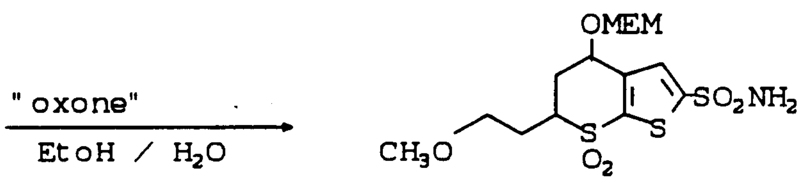


43

5,6-Dihydro-4-(methoxyethoxymethoxy)-6-(2-methoxyethyl)-4H-thieno[2,3-b]thiopyran-2-sulfonamide (13.1g. 0.033 mol) was dissolved in ethanol (150 ml) and water (75 ml) was added. To this stirred cloudy solution was added Oxone® (30.7g, 0.05 mol). After stirring at ambient temperature for 3 hours another 7.7g of Oxone® was added. Stirring was continued for an additional 1 1/2 hours and then the mixture was basified by adding excess sodium bicarbonate in small portions. The mixture was filtered and the solids were washed with ethyl acetate and ethanol. The filtrate and washings were concentrated in vacuo at room temperature. The residual yellow gum was taken up in ethyl acetate (250 ml) and water (50 ml). The ethyl acetate solution was separated and washed with saturated NaCl, dried, filtered and concentrated in vacuo at room temperature to give 12.3g (86%) of pale yellow oil.

Step D: Preparation of 5,6-Dihydro-4-hydroxy-6-(2-methoxyethyl)-4H-thieno[2,3-b]thiopyran2-sulfonamide-7,7-dioxide

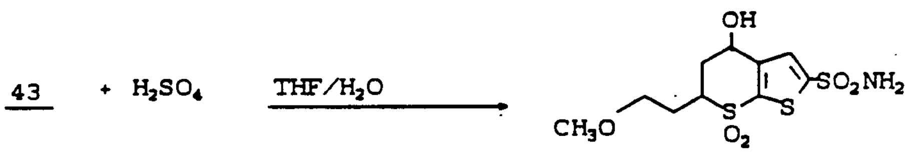


44

5,6-Dihydro-4-(methoxyethoxymethoxy)-6-(2-methoxyethyl)-4H-thieno[2,3-b]thiopyran-2-sulfonamide-7,7-dioxide (12.0g, 0.028 mol) was dissolved in 25 ml of tetrahydrofuran and 50 ml of sulfuric acid-water (50-50, volume-volume) was added. The solution was stirred at ambient temperature for 4 hours. Then the solution was poured into a suspension of sodium bicarbonate (200g) in ethyl acetate (300 ml). After the mixture was neutralized it was filtered. The solids were washed with ethyl acetate and tetrahydrofuran and the filtrate and washings were concentrated in vacuo at room temperature. The residual oil was taken up in ethyl acetate (150 ml), washed with saturated NaCl, dried, filtered and concentrated in vacuo at room temperature to give 9.16g (95%) of white solid foam.

Step E: Preparation of 4-acetamido-5,6-dihydro-6-(2-methoxyethyl)-4H-thieno[2,3-b]-thiopyran-2-sulfonamide-7,7-dioxide

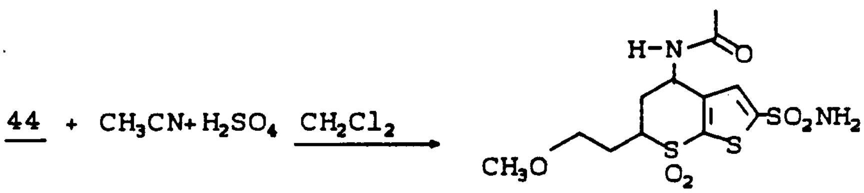


45

5,6-Dihydro-4-hydroxy-6-(2-methoxyethyl)-4H-thieno[2,3-b]thiopyran-2-sulfonamide-7,7-dioxide (8.86g, 0.026 mol) was dissolved in dry acetonitrile (85 ml) and methylene chloride (40 ml). Concentrated sulfuric acid (24 ml, 0.45 mol) was added dropwise over 1 hour at -5 to -10°C. The solution was left stirring overnight as the ice melted in the cooling bath and the temperature rose to room temperature. After 24 hours the reaction solution was poured into 300 ml of ice and water. Solid sodium bicarbonate was then added until the mixture was slightly basic. Then 300 ml of water was added to dissolve the salts and the mixture was extracted with 150 and 3 x 100 ml of ethyl acetate. The combined extracts were washed with saturated NaCl solution, dried, filtered and concentrated in vacuo at room temperature to give 4.4g (44%) of pale yellow solid.

Step F: 5,6-Dihydro-4-ethylamino-6-(2-methoxyethyl)-4H-thieno[2,3-b]thiopyran-2-sulfonamide-7,7-dioxide (cis and trans isomers)

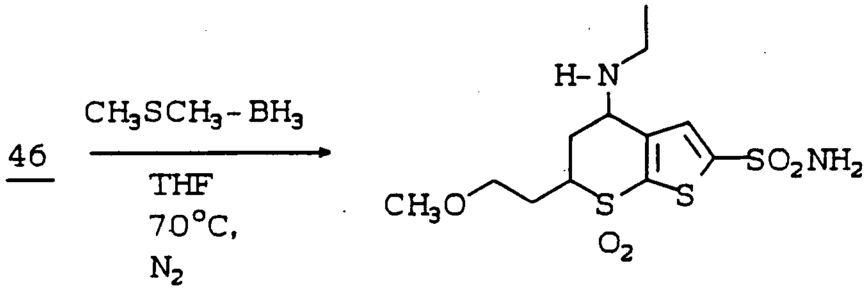


47

To a stirred solution of 4-acetamido-5,6-dihydro-6-(2-methoxyethyl)-4H-thieno[2,3-b]thiopyran2-sulfonamide-7,7-dioxide (4.4 g, 0.0115 mol) in tetrahydrofuran (45 ml) under nitrogen and heated to reflux was added borane dimethylsulfide complex (5 ml, 0.05 mol of a 10 M complex) over about 20 minutes. Stirring was continued at reflux for 3 hours, then at room temperature overnight and another 5 hours and then concentrated in vacuo at room temperature. Methanol (50 ml) was carefully added to the white residue. The resulting solution was saturated with anhydrous HCl gas and then was stirred at reflux for 1 hour. The solution was concentrated in vacuo and the residue was taken up in ethyl acetate (50 ml) and saturated sodium bicarbonate (20 ml). The ethyl acetate solution was separated and washed with saturated NaCl, dried, filtered and concentrated in vacuo. The procedure gave a mixture of cis and trans isomers (3.2 g) as a white gummy foam which was chromatographed on silica gel (300 g) using 7.5% methanol-chloroform. Mixed fractions (0.87 g) were chromatographed again using 5% methanol-ether. There was obtained 1.62 g of the α-isomer, mp 132-134°C and 0.35 g of the β-isomer, mp 167-169°C. The hydrochloride salts of the α (trans) and β-isomer (cis) racemates were prepared using ethanolic-HCl and diluting with ether. MP's were 173-175°C and 264.5-266°C, respectively.

EXAMPLE 10

5,6-Dihydio-4-ethylamino-6-(3-methoxypropyl)-4H-thieno[2,3-b]thiopyran-2-sulfonamide-7,7-dioxide

Step A: Preparation of 5-carbomethoxy-3-thienylthiopentanoic acid

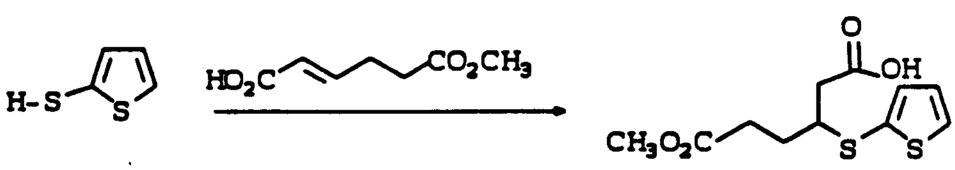

48

A solution of 2-mercaptothiophene (29 g, 0.25 mol), 5-carbomethoxypent-2-enoic acid (40 g, 0.25 mol) and triethylamine (18.6 ml, 0.134 mol) in dry THF (430 ml) was heated at reflux for 3 hours. The THF was evaporated at reduced pressure, the residue dissolved in ethyl acetate, washed with cold 2N HCl, $H_2O$, brine, dried over $Na_2SO_4$ and evaporated at reducer pressure to give 63 g of the title compound which was used in Step B without further purification.

Step B: Preparation of 6-(2-carbomethoxyethyl)-5,6-dihydro-4-oxo-4H-thieno[2,3-b]thiopyran

48 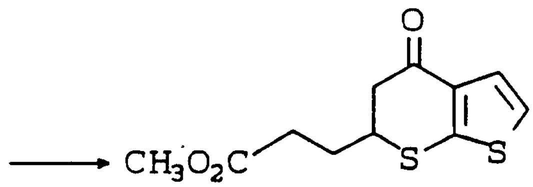

49

A solution of compound from Step A (63 g, 0.252 mol) in $CH_2Cl_2$ (600 ml) and dimethylformamide (1 ml) in a $N_2$ atmosphere was treated over a 20 minute period with oxalyl chloride (22.4 ml, 0.26 mol) in $CH_2Cl_2$ (50 ml). The reaction mixture was stirred at 25°C for 2 hours, cooled to -10°C and treated with $SnCl_4$ (14.5 ml, 0.125 mol) in $CH_2Cl_2$ (50 ml) over a 20 minute period. The reaction was stirred at 0°C for 1 hour then poured into 300 ml of vigorously stirred ice and $H_2O$. The $CH_2Cl_2$ layer was washed with saturated $NaHCO_3$, brine then dried over $Na_2SO_4$ and evaporated at reduced pressure. Purification was effected by flash chromatography on $SiO_2$ eluting with ethyl acetate-hexane (1:3 v/v).

Step C: Preparation of 6-(2-carboethoxyethyl)-5,6-dihydro-4-hydroxy-4H-thieno[2,3-b]thiopyran

49 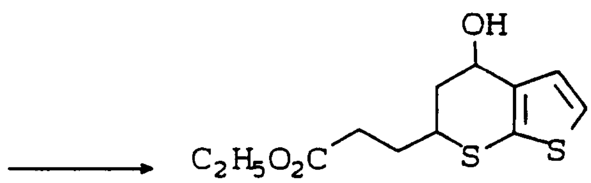

50

A solution of compound from Step B (40.0 g 0.156 mol) in ethanol (460 ml) was cooled to 0°C in a $N_2$ atmosphere and treated with sodium borohydride (1.90 g 0.05 mole). The reaction mixture was stirred at 25°C for 18 hours then the ethanol was evaporated at reduced pressure. The residue was dissolved in ethyl acetate, washed with ice-$H_2O$, saturated $NaHCO_3$, brine, then dried over $Na_2SO_4$. Evaporation of the ethyl acetate left 42 g of title compound as an oil.

Step D: Preparation of 6-(2-carboethoxyethyl)-5,6-dihydro-4-methoxyethoxymethoxy-4H-thieno[2,3-b]thiopyran

50 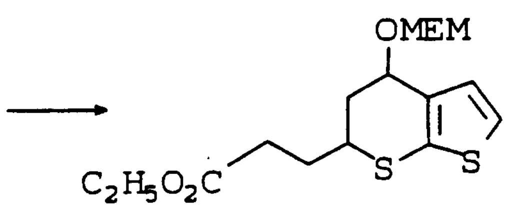

51

A stirred solution of product from Step C (46.8 g, 0.172 mol) in $CH_2Cl_2$ (350 ml) was cooled in an ice bath and

treated with N,N-diisopropylethylamine (47.4 ml, 0.273 mol) and methoxyethoxymethyl chloride (31.2 ml, 0.273 mol). The reaction mixture was stirred at 25°C for 18 hours washed with ice-$H_2O$, saturated $NaHCO_3$ and brine then dried over $Na_2SO_4$. Evaporation of the $CH_2Cl_2$ left 58.6 of title comound as a yellow oil.

Step E: Preparation of 5,6-Dihydro-6-(3-hydroxypropyl)4-methoxyethoxymethoxy-4H-thieno-[2,3-b]thiopyran

51

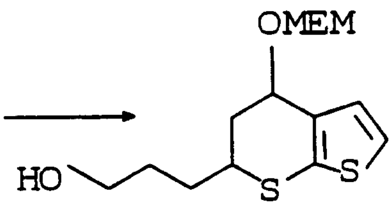

52

Diethyl ether (55 ml) was cooled in an ice bath in a $N_2$ atmosphere. Lithium aluminum hydride (3.93 g, 0.104 mol) was suspended with stirring and a solution of product from Step D (29.3 g, 0.0814 mol) in THF (78 ml) was added over a 1.5 hour period at 0°-3°C. The reaction mixture was stirred at 25°C for 18 hours, cooled in ice then cautiously treated dropwise with $H_2O$ (4.15 ml) over 1/2 hour, then with 20% NaOH (3.1 ml) and finally with more $H_2O$ (14.5 ml). Stirring was continued for 1/2 hour, the precipitated salts filtered and rinsed with ether. The combined organic phase was dried over $Na_2SO_4$ and evaporated at reduced pressure to leave 25 g of product as a yellow oil.

Step F: Preparation of 5,6-Dihydro-4-methoxyethoxymethoxy-6-(3-methoxypropyl)-4H-thieno[2,3-b]thiopyran

52

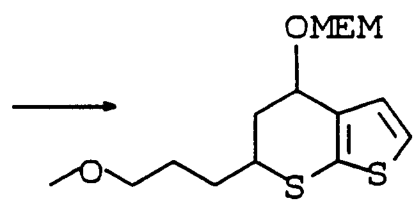

53

Sodium hydride (50% in mineral oil, 4.8 g, 0.10 mole) was rinsed with petroleum ether then transferred under $N_2$ to a 500 ml flask with THF. The suspension was cooled in ice with stirring and compound from Step E (25 g, 0.079 mol) in THF (80 ml) was added over 20 minutes. The reaction mixture was stirred for 1/2 hour then methyl iodide (30.2 g, 0.21 mol) was added and stirring was continued for 18 hours at 25°C. Most of the THF was evaporated. The residue was dissolved in ethyl acetate, washed with $H_2O$ brine, dried over $Na_2SO_4$ and the solvent evaporated at reduced pressure to leave 25 g of product as a yellow oil.

Step G: Preparation of 5,6-Dihydro-4-methoxyethoxymethoxy-6-(3-methoxypropyl)-4H-thieno[2,3-b]-thiopyran-2-sulfonamide

53

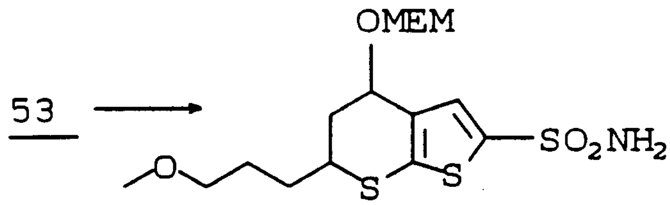

54

Under $N_2$ a solution of compound from Step F (25 g, 0.075 mol) in THF (220 ml) was cooled to -78°C with stirring. A solution of butyl lithium (2.5 M in hexane, 39 ml, 0.098 mol) was added over 15 minutes then stirring was continued at -78°C for 1 hour. The nitrogen inlet was replaced by an inlet for $SO_2$ gas which was introduced over the surface of the reaction for 15 minutes during which time the temperature rose to -55°C then dropped back to -78°C. Stirring was continued at -78°C for 2 hours then at 20°C for 1/2 hour. The solvents were evaporated in vacuo and the residue was stirred in ice while a solution of sodium acetate (19.2 g, 0.234 mole) in water (175 ml) was added followed by hydroxylamine-O-sulfonic acid (22.2 g, 0.197 mol). The reaction was stirred at 25°C for one hour then treated with saturated $NaHCO_3$ until slightly basic, extracted with ethyl acetate, washed with brine, dried over $Na_2SO_4$ and evaporated at reduced pressure to give 28 g of product which melted at 114°C after crystallization from ethyl acetatehexane.

| Anal. Cal'd for $C_{16}H_{25}NO_6S_3$: | | | |
|---|---|---|---|
| | C, 43.78; | H, 6.12; | N, 3.40 |
| Found: | C, 43.35; | H, 5.88; | N, 3.45 |

Step H: Preparation of 4-Acetamido-5,6-dihydro-6-(3-methoxypropyl)-4H-thieno[2,3-b]thiopyran-2-sulfonamide

54

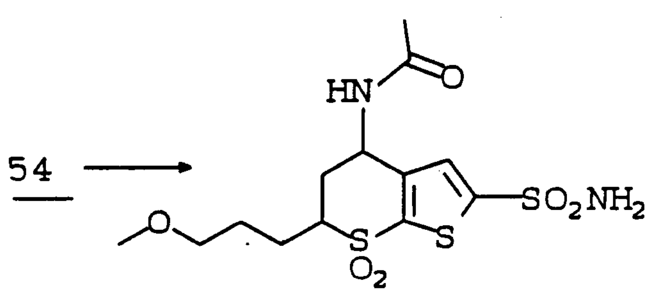

55

A stirred suspension of product from Step G (14.0 g, 0.034 mol) in acetonitrile (125 ml) was cooled to 15°C and treated over 15 minutes with concentrated sulfuric acid (18.1 ml). The reaction mixture was stirred at 25°C for 4 hours then poured into ice $H_2O$ (500 ml) and made basic by the addition of solid $NaHCO_3$. Ethyl acetate (700 ml) was added, the precipitated salt removed by filtration and the ethyl acetate solution was washed with brine, dried over $Na_2SO_4$ and evaporated at reduced pressure to give 13 g of product as a reddish-brown gum.

Step I: Preparation of 4-Acetamido-5,6-dihydro-6-(3-methoxypropyl)-4H-thieno[2,3-b]thiopyran-2-sulfonamide-7,7-dioxide

55

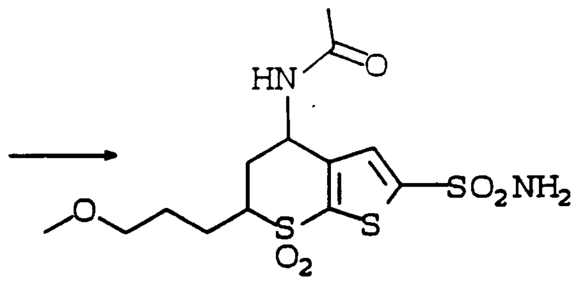

56

Water (66 ml) was added to a solution of product from Step H (13 g, 0.035 mole) in warm ethanol (130 ml). Oxone® (32.8 g, 0.053 mol) was added and the mixture stirred at 25°C for 18 hours. Solid $NaHCO_3$ was added to neutrality, the salts filtered, rinsed with ethanol and the solvents evaporated at reduced pressure. The residue was dissolved in ethyl acetate (150 ml) washed with saturated $NaHCO_3$ and brine, dried over $Na_2SO_4$ and evaporated at reduced pressure. Chromatography on $SiO_2$ eluted with $CHCl_3$-$CH_3OH$ (10:1) gave 8.7 g of product.

Step J: Preparation of 5,6-Dihydro-4-ethylamino-6-(3-methoxypropyl)-4H-thieno[2,3-b]thiopyran-2-sulfonamide-7,7-dioxide

56

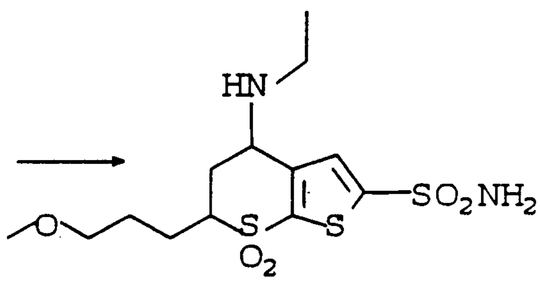

57

A stirred solution of product from Step I (8.7 g, 0.022 mole) in dry THF (45 ml) was heated to reflux in a $N_2$ atmosphere. Borane-methylsulfide complex (22 ml, 0.22 mol) was added over 15 minutes, the reaction refluxed 4 hours then cooled in an ice bath. Methanol (22 ml) was added dropwise over 1/2 hour then the solvents were evaporated at reduced pressure. The flask was again cooled in ice and 6N HCl (82 ml) was slowly added. The acidic solution was heated on a steam bath for 1/2 hour, evaporated to dryness, dissolved in ethyl acetate (700 ml) washed with brine, dried over $Na_2SO_4$ and evaporated at reduced pressure to give 8 g of a mixture of cis and trans products which were separated by chromatography eluting with $CHCl_3$-$CH_3OH$ (10:1). Each isomer was converted to its hydrochloride salt with ethanolic HCl.
mp (trans • HCl): 212-214°C
mp (cis • HCl): 251-252°C.

| Anal. Calc'd for $C_{13}H_{22}N_2O_5S_3 \cdot HCO$: | | | |
|---|---|---|---|
| | C, 37.27; | H, 5.53; | N, 6.69 |
| Found: (trans) | C, 37.49; | H, 5.63; | N, 6.74 |
| Found: (cis) | C, 37.58; | H, 5.45; | N, 6.68. |

Step K: Resolution of the trans diastereomer

To a warm stirred solution of the trans product from Step J (3.67 g, 9.6 mM) in acetonitrile (40 ml) was added di-p-toluoyl-D-tartaric acid hydrate (960 mg, 2.4 mM). The mixture was cooled, the salt filtered, slurried in fresh warm acetonitrile (40 ml) cooled and filtered. The salt thus obtained was treated with saturated $NaHCO_3$ and ethyl acetate. The ethyl acetate was washed with water and brine, dried over $Na_2SO_4$ and evaporated at reduced pressure. The residual base was dissolved in hot ethanol (50 ml) and treated with an excess of ethanolic HCl. The solution was cooled and filtered to give 1.3 g of trans (+) product hydrochloride which melted at 233-235°C as the hemi-hydrate.

| Anal. Cal'd for $C_{13}H_{22}N_2O_5S_3 \cdot HCl \cdot 1/2H_2O$ | | | |
|---|---|---|---|
| | C, 36.48; | H, 5.65; | N, 6.35 |
| Found: | C, 36.67; | H, 5.56; | H, 6.56. |

$[\alpha]_D^{25}$ = +12.1° ($CH_3OH$)

The acetonitrile filtrates from the resolution of the trans (+) isomer were evaporated to dryness and distributed between ethyl acetate and saturated $NaHCO_3$. The ethyl acetate layer was washed with brine, dried over $Na_2SO_4$ and evaporated in vacuo to give 2.3 g (6 mM) of free base. The base was dissolved in 50 ml of hot acetonitrile and treated with di-p-toluoyl-L-tartaric acid hydrate (1.21 g, 3 mMol) then refrigerated overnight. The salt was filtered, slurried in warn acetonitrile (30 ml) cooled, filtered and dried. The resulting salt was distributed between ethyl acetate and saturated $NaHCO_3$; the ethyl acetate was washed with brine, dried over $Na_2SO_4$ and evaporated in vacuo. The resultant base was dissolved in ethanol (50 ml) and treated with an excess of ethanolic hydrochloric acid and filtered to give 1.6 g of trans (-) enantiomer hydrochloride hemiethanolate.

| Anal. Calc'd for $C_{13}H_{22}N_2O_5S_3 \cdot HCl \cdot 1/2EtOH$ | | | |
|---|---|---|---|
| | C, 38.04; | H, 5.93; | N, 6.34 |
| Found: | C, 38.26; | H, 6.11; | N, 6.15. |

$[\alpha]_D^{25}$ = -12.2° ($CH_3OH$).

A 190 mg sample of trans (-) base was dissolved in ethanol (5 ml) to which was added a slight excess of isethionic acid. Treatment with ether gave the trans (-) enantiomer isethionate.

| Anal. Calc'd for $C_{13}H_{22}N_2O_5S_3 \cdot C_2H_6O_4S$ | | | |
|---|---|---|---|
| | C, 35.42; | H, 5.55; | N, 5.51 |
| Found: | C, 35.51; | H, 5.47; | N, 5.28. |

EXAMPLE 11

5,6-Dihydro-6-(3-methoxypropyl)-4-propylamino-4H-thieno[2,3-b]thiopyran-2-sulfonamide-7,7-dioxide

Step A: Preparation of 5,6-Dihydro-6-(3-methoxypropyl)-4-propronamido-4H-thieno[2,3-b]-thiopyran-2-sulfonamide

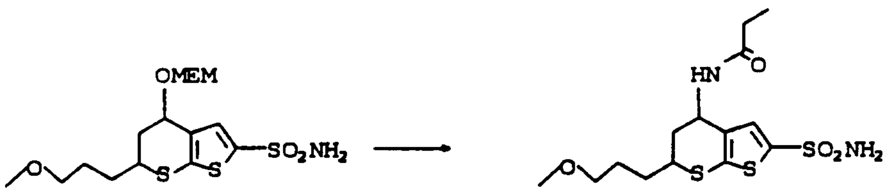

58 59

A stirred suspension of 5,6-Dihydro-4-methoxyethoxymethoxy)-6-(3-methoxypropyl)-4H-thieno[2,3-b]thiopyran-2-sulfonamide (12.6 g, 0.307 mol) in propionitrile (125 ml) was cooled to 15°C and treated with concentrated $H_2SO_4$ (16.3 ml) over a 5 minute period. The reaction mixture was stirred at 25°C for 3 hours, poured into ice $H_2O$, made basic with solid $NaHCO_3$ and extracted with ethyl acetate which was washed with brine, dried over $Na_2SO_4$ and evaporated in vacuo to give 12.4 g of product.

Step B: 5,6-Dihydro-6-(3-methoxypropyl)-4-proponamido-4H-thieno[2,3-b]thiopyran-2-sulfonamide-7,7-dioxide

59

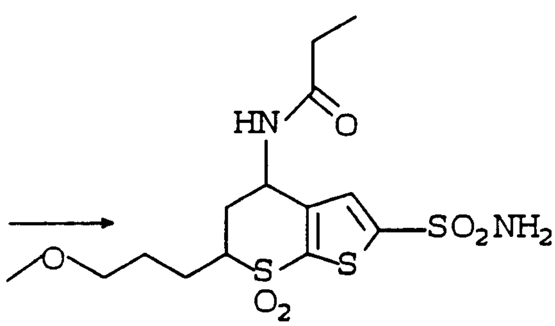

60

Water (60 ml) was added to a stirred solution of product from Step A in hot ethanol (120 ml) then Oxone® (30 g, 0.049 mol) was added over 5 minutes. The reaction mixture was stirred at 25°C for 3 hours and neutralized with solid $NaHCO_3$. The salts were filtered, rinsed with ethanol and ethyl acetate and the organic solvents were evaporated at reduced pressure. The residue was dissolved in ethyl acetate, washed with saturated $NaHCO_3$, brine and dried over $Na_2SO_4$. The ethyl acetate was evaporated in vacuo and the residue chromatographed on $SiO_2$ eluted with $CHCl_3$-$CH_3OH$ (10:1) to give 9 g of product.

Step C: Preparation of 5,6-Dihydro-6-(3-methoxypropyl)-4-propylamino-4H-thieno[2,3-b]-thiopyran-2-sulfonamide-7,7-dioxide

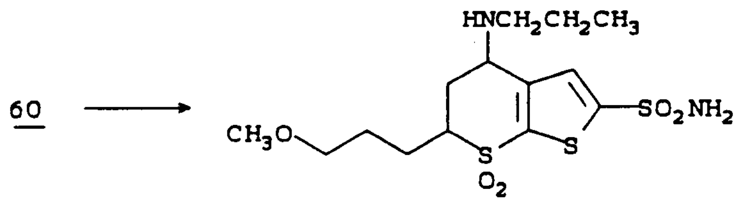

61

Under $N_2$ compound from Step B (9.0 g, 0.022 mole) was dissolved in THF (45 ml), heated to reflux and treated with borane-methyl sulfide complex (22 ml, 0.22 mol) over 15 minutes. The reaction mixture was heated at reflux for 4 hours, cooled in an ice bath and slowly treated with $CH_3OH$ (27 ml) over 50 minutes. After 1 hour the solvents were evaporated in vacuo, the residue cooled in ice and slowly treated with 6N HCl (82 ml). The HCl solution was heated on a steam bath for 1/2 hour then evaporated to dryness at reduced pressure. The residue was made basic with saturated $NaHCO_3$, extracted into ethyl acetate, washed with brine, dried over $Na_2SO_4$ and evaporated in vacuo. The cis and trans isomers were separated by chromatography on $SiO_2$ eluting with $CHCl_3$-$CH_3OH$ (10:1) then converted to their hydrochloride salts by treatment with ethanolic HCl and ether.
m.p. (trans) = 231-232°C m.p. (cis) = 274-275°C.

| Anal. Calc'd for $C_{14}H_{24}N_2O_5S_3 \cdot HCl$ | | | |
|---|---|---|---|
| | C, 38.83; | H, 5.82; | N, 6.47 |
| Found: (trans) | C, 38.73; | H, 5.67; | N, 6.38 |
| (cis) | C, 38.92; | H, 5.79; | N, 6.38 |

Step D: Resolution of trans 5,6-Dihydro-6-(3-methoxypropyl)-4-propylamino-4H-thieno[2,3-b]-thiopyran-2-sulfonamide-7,7-dioxide

To a warm solution of trans product from Step C (1.4 g, 3.54 mMol) in 2-propanol (20 ml) was added di-p-toluoyl-D-tartaric acid hydrate (0.71 g, 1.76 mMol). The solution was refrigerated and the salt thus obtained was thrice recrystallized from 2-propanol (3 x 30 ml). The resulting salt was treated with saturated $NaHCO_3$, extracted with ethyl acetate, washed with brine, dried over $Na_2SO_4$ and evaporated in vacuo. The resulting base was treated with ethanolic HCl and ether to give 520 mg of trans (+) product hydrochloride, m.p. = 215-217°C.

| Analysis Calc'd for $C_{14}H_{24}N_2O_5S \cdot HCl$ | | | |
|---|---|---|---|
| | C, 38.83; | H, 5.82; | N, 6.47 |
| Found: | C, 38.99; | H, 5.71; | N, 6.44 |

$[\alpha]_D^{25}$ = + 12.2° ($CH_3OH$).

The 2-propanol filtrates were combined and evaporated to dryness. The residue was distributed between saturated $NaHCO_3$ and ethyl acetate. The latter was washed with brine, dried over $Na_2SO_4$ and evaporated in vacuo. The base (3.8 g, 7.1 mMol) was dissolved in hot 2-propanol and treated with di-p-toluoyl-2-tartaric acid (1.43 g, 3.55 mMol) and cooled. The resulting salt was thrice recrystallized from 2-propanol (3 x 40 ml) converted to the base and then to the hydrochloride as previously described to give 320 mg of trans (-) enantiomer hydrochloride.

mp = 218-220°C.

| Analysis for $C_{14}H_{24}N_2O_5S_3 \cdot HCl$ | | | |
|---|---|---|---|
| | C, 38.83; | H, 5.82; | N, 6.47 |
| Found: (trans (-)) | C, 38.56; | H, 5.71; | N, 6.41 |

$[\alpha]_D^{25}$ = -12.3° ($CH_3OH$).

EXAMPLE 12

Enantiomers of trans -5,6-dihydro-4 ethylamino-6-[3-(2-methoxyethoxy)propyl]-4H-thieno[2,3-b]-thiopyran-2-sulfonamide-7,7-dioxide

Step A: Preparation of the trans, (+) rotary enantiomer hydrochloride

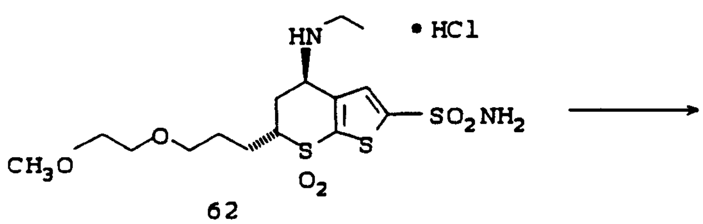

62

(trans-isomer)

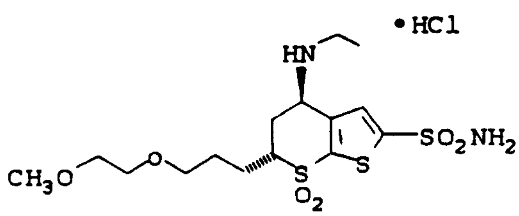

62

(trans-isomer)

(+) rotary enantiomer

A sample of the title compound (2.8g, 6.6 mM) was dissolved in hot ethyl acetate (120 ml) along with di-p-toluoyl-D-tartaric acid (1.27g, 3.3mM). After standing for 7 days the white crystalline salt was filtered and recrystallized from ethyl acetate (100 ml) to give 960 mg of salt which was partitioned between aqueous $NaHC0_3$ and ethyl acetate, the latter washed with brine, dried over $Na_2SO_4$ and evaporated in vacuo. The residue was dissolved in ethanol (8 ml) treated with a slight excess of 6N ethanolic HCl then ether until cloudy then refrigerated overnight. The white crystals were filtered, rinsed with ether and dried to give 530 mg of (+) title compound which melts at 177-9°C.

| Analysis Calc'd for $C_{15}H_{26}N_2O_6S_3 \cdot HCl$ | | | |
|---|---|---|---|
| | C, 38.91, | H, 5.88, | N, 6.05 |
| Found: | C, 38.90, | H, 5.96, | N, 6.39 |

Step B: Preparation of the trans, (-) rotary enantiomer hydrochloride

62 62

(trans-isomer) (trans-isomer)

(-) rotary enantiomer

The ethyl acetate filtrates from the (+) salt were combined and washed with aqueous $NaHCO_3$ and brine, dried over $Na_2SO_4$ and evaporated in vacuo providing 2.0 g of the (-) enriched free base which was dissolved in hot ethyl acetate along with di-p-toluoyl-L-tartaric acid (953 mg). After cooling the crystals were filtered then recrystallized from EtOAc (70 ml). The salt was partitioned between aqueous sodium bicarbonate and EtOAc, the latter washed with brine dried over sodium sulfate and evaporated in vacuo. The residue was dissolved in ethanol (5 ml), treated with a slight excess of 6N ethanolic HCl then with ether until cloudy then refrigerated. The white crystals were filtered, rinsed with ether and dried to give 380 mg of (-) title compound which melts at 177-9°C

| Analysis Calc'd for $C_{15}H_{26}N_2O_6S_3 \cdot HCl$ | | | |
|---|---|---|---|
| | C, 38.91, | H, 5.88, | N, 6.05 |
| Found: | C, 38.83, | H, 5.94, | N, 5.93 |

EXAMPLE 13

5,6-Dihydro-4-ethylamino-6-(2-hydroxyethoxy)-methyl-4H-thieno[2,3-b]thiopyran-2-sulfonamide-7,7-dioxide hydrochloride, trans isomer

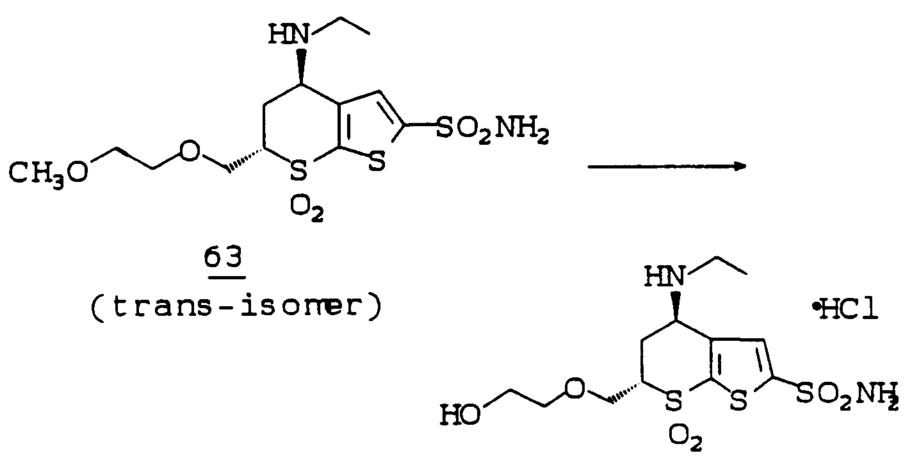

63

(trans-isomer)

64

(trans-isomer)

Under $N_2$, 18-crown 6 (7.92 g, 0.03 mol) in $CH_2Cl_2$ (50 ml) and NaI (5 g, 0.033 mol) was stirred at room temperature. After 5 minutes, the reaction was cooled to 0-4°C and an ether solution of trans-5,6-dihydro-4-ethylamino-6-(2-methoxyethoxy) methyl-4H-thieno[2,3-b]thiopyran-2-sulfonamide-7,7-dioxide (2.1g, 0.005 mol) was added. After addition, the reaction was stirred at room temperature for 0.5 hours and then cooled to -78°C and $BBr_3$ in $CH_2Cl_2$ (1M, 25 ml, 0.025 mol) was added. After the addition, the reaction was allowed to warm to 0°C over 3 hours. The mixture was then concentrated to dryness and the residue was treated with 6N HCl (40 ml). After heating for 5 minutes in a steam bath, the reaction was cooled and neutralized to pH 8.5 with $NaHCO_3$. The resulting mixture was extracted with EtOAc(3x) and the organic extracts were dried, filtered and concentrated to dryness. The residue was chromatographed on a Still silica gel column and the product eluted with 25-50% methanol-chloroform with 2.5 to 5.0% aqueous $NH_3$ to yield crude product. The material was crystallized as the HCl salt from EtOH to yield 280 mg (13%) of the title compound; m.p. 157-9°C.

| Analysis Calc'd for $C_{12}H_{20}N_2O_6S_3 \cdot HCl$ | | | |
|---|---|---|---|
| | C, 34.24, | H, 5.03, | N, 6.66 |
| Found: | C, 34.57, | H, 5.25, | N, 6.49 |

PREPARATION 17

5,6-Dihydro-4-ethylamino-6-(2-furfurylthioethyl)-4H-thieno[2,3-b]thiopyran-2-sulfonamide-7,7-dioxide hydrochloride, trans isomer

Step A: 6-Bromoethyl-5,6-dihydro-4-ethylamino-4H-thieno[2,3-b]thiopryan-2-sulfonamide-7,7-dioxide hydrobromide, trans isomer

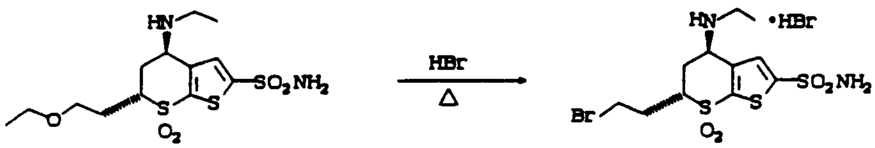


21 (trans-isomer) 67 (trans-isomer)

The trans isomer of 5,6-dihydro-6-(2-ethoxyethyl)-4-ethylamino-4H-thieno[2,3-b]-thiopyran-2-sulfonamide-7,7-dioxide (5.9 g, 0.0154 mol) (prepared as in Example 2) was stirred at steam bath temperature with 48% hydrobromic acid (100 ml) until the intermediate 6-(2-hydroxyethyl) compound was completely converted to the 6-(2-bromoethyl) product as shown by thin layer chromatography (3 to 5 days). The reaction mixture was concentrated *in vacuo* at 60°C bath temperature. A quantitative yield of the title compound was obtained as a pale tan solid (7.7g).

EXAMPLE 18

5,6-Dihydro-4-ethylamino-6-(2-hydroxyethylthioethyl)-4H-thieno[2,3-b]thiopyran-2-sulfonamide-7,7-dioxide hydrochloride, trans isomer

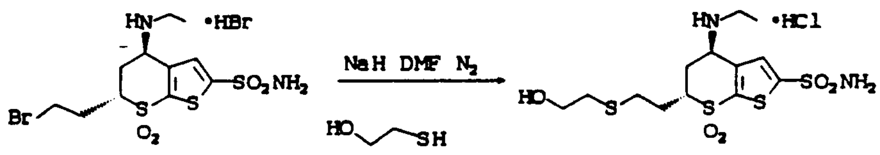


67 (trans-isomer) → 69 (trans-isomer)

The title compound was prepared using substantially the same procedure described in Step B of Preparation 17, except that mercaptoethanol (0.84 ml, 0.012 mol) was substituted in place of furfuryl mercaptan. Chromatography on silica gel gave 400 mg of pure product as an oil which solidified to a white solid with m.p.= 140-142°C. The hydrochloride salt was prepared using ethanolic hydrogen chloride and was recrystallized from methanol-ether, with m.p.= 130-140°C with decomposition.

| Analysis calc'd for $C_{13}H_{22}N_2O_5S_4$ • HCl; | | | |
|---|---|---|---|
| | C, 34.62; | H, 5.14; | N, 6.21 |
| Found: | C, 35.04; | H, 5.44; | N, 6.00 |

PREPARATION 19

5,6-Dihydro-4-propylamino-6-(3-bromopropyl)-4H-thieno[2,3-b]thiopyran-2-sulfonamide-7,7-dioxide hydrobromide, trans isomer

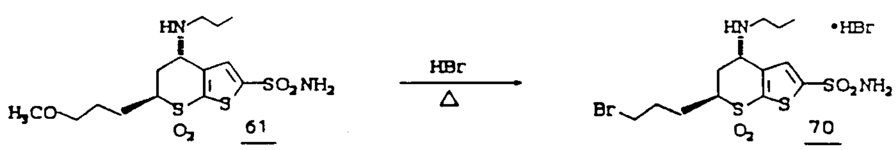


61 (trans-isomer) (S,S) enantiomer → 70 (trans-isomer) (S,S) enantiomer

Employing the procedure substantially as described in Step A of Preparation 17, but substituting trans 5,6-dihydro-6-(3-methoxypropyl)-4-propylamino-4H-thieno[2,3-b]thiopyran-2-sulfonamide-7,7-dioxide as the starting material, the title compound was prepared in quantitative yield. Melting point=223-225°C.

| Analysis Calc'd for: $C_{13}H_{21}N_2BrO_4S_3$ • HBr | | | |
|---|---|---|---|
| | C, 32.40, | H, 4.60, | N, 5.81 |
| Found: | C, 32.50, | H, 3.73, | N, 5.71 |

EXAMPLE 20

Employing the procedure substantially as described in Step B of Preparation 17, but substituting trans(S,S) 5,6-dihydro-4-propylamino-6-(3-bromopropyl)-4H-thieno[2,3-b]thiopyran-2-sulfonamide-7,7-dioxide hydrobromide (prepared as described in Preparation 19) for the 6-bromoethyl compound used therein, and substituting the furfuryl mercaptan used therein with the compounds described in Table II, there were produced the corresponding (S,S)6-(3-substituted-propyl) compounds also described in Table II:

TABLE II

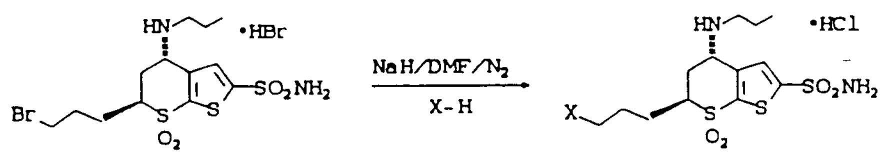


(trans-isomer)
(S, S) enantiomer

70
(trans-isomer)
(S, S) enantiomer

| X | Isomer | MP (°C) |
|---|---|---|
| $HOCH_2CH_2S-$ | trans (S, S) | 154-156** |

** = Free base

EXAMPLE 39

5,6-Dihydro-6-allyloxymethyl-4-ethylamino-4H-thieno[2,3-b]thiopyran-2-sulfonamide-7,7-dioxide hydrochloride

Step A: 5,6-Dihydro-6-allyloxymethyl-4-methoxyethoxymethoxy-4H-thieno[2,3-b]-thiopyran-2-sulfonamide-7,7-dioxide

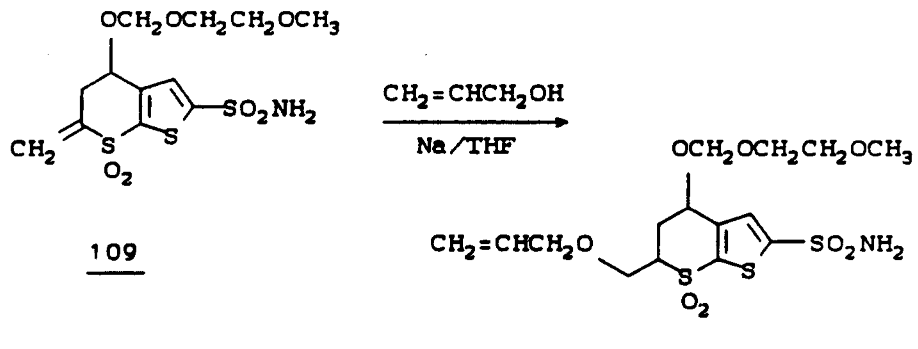


109

110

Sodium (4.6g., 0.20m) was added portionwise to allyl alcohol (110 ml.) with stirring under nitrogen. After solution was effected, it was added to 5,6-dihydro-4-methoxyethoxymethoxy-6-methylene-4H-thieno[2,3-b]thiopyran-2-sulfonamide-7,7-dioxide, (32.5g, 0.085m), dissolved in tetrahydrofuran (75 ml.). After stirring at ambient temperature for 21 hours, the reaction mixture was cooled in ice, acidified with 6N hydrochloric acid (90 ml., 0.24m) and then basified with saturated sodium bicarbonate solution. The mixture was concentrated in vacuo and the residue distributed between ethyl acetate (600 ml) and $H_20$ (250 ml.). The aqueous layer was separated and extracted with ethyl acetate (2 x 250 ml.), the combined ethyl acetate layers were washed twice with brine, dried over sodium sulfate, and concentrated in vacuo to yield 33.1g. (88%) of viscuous oily product.

Step B: 5,6-Dihydro-6-allyloxymethyl-4-hydroxy-4H-thieno[2,3-b]thiopyran-sulfonamide-7,7-dioxide

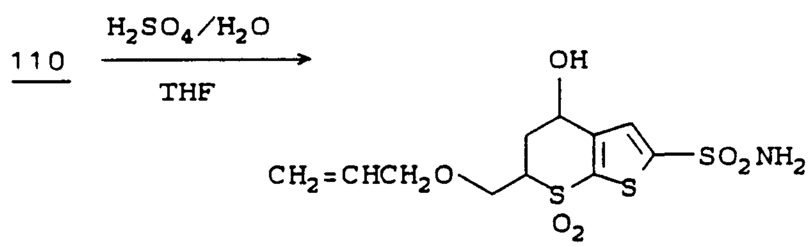


111

A solution of 5,6-dihydro-6-allyloxymethyl-4-methoxyethoxymethoxy-4H-thieno[2,3-b]thiopyran-2-sulfonamide-7,7-dioxide (33.0 g., 0.075 m) in tetrahydrofuran (230 ml.) was cooled to -5°C and stirred while a solution of concentrated sulfuric acid (230 ml.) in water (230 ml.) was added dropwise over 45 min. while maintaining the temperature below 5°C. After stirring at -5°C for 1 hour and at ambient temperature for 3 hours, the mixture was added slowly to a stirred suspension of sodium bicarbonate (750 g) in ethyl acetate (900 ml.) and ice. After 30 minutes with periodic additions of saturated sodium bicarbonate to render the mixture basic, it was filtered and the solids were washed with ethyl acetate. The combined filtrate and washings were washed twice with $H_20$, dried over sodium sulfate and concentrated in vacuo to afford 25.9 g (98%) of an amorphous product.

Step C: 5,6-Dihydro-6-allyloxymethyl-4-ethylamino-4H-thieno[2,3-b]thiopyran-2-sulfonamide-7,7-dioxide

111

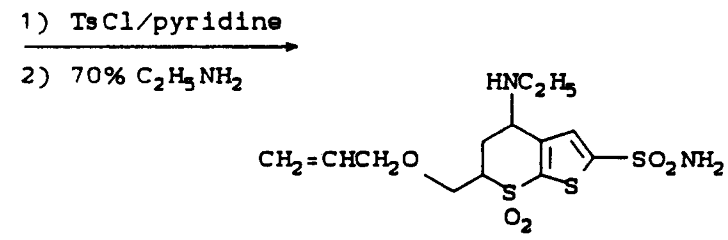


112

A stirred solution of 5,6-dyhydro-6-allyloxymethyl-4-hydroxy-4H-thieno[2,3-b]thiopyran-2-sulfonamide-7,7-dioxide (12.5 g, 0.035 m) in dry pyridine (65 ml.) was cooled to -10°C under nitrogen while p-toluenesulfonyl chloride (14.7 g., 0.077m) was added in one portion. After stirring at -10°C for 5 hours, the mixture was further cooled to -20°C and 70% aqueous ethylamine (150 ml.) was added over 45 minutes while maintaining the temperature between -20°C and -10°C. The mixture was stirred at ambient temperature for 1.5 hours and then at 50°C for 16.5 hours, after which it was concentrated in vacuo. The residue was distributed between ethyl acetate (600 ml) and saturated sodium bicarbonate solution (300 ml.), the aqueous layer was separated and extracted with ethyl acetate (2 x 300 ml.), the combined ethyl acetate extracts were washed twice with $H_2O$ and concentrated to approximately 250 ml. in vacuo. The solution was extracted with 3N hydrochloric acid (2 x 150 ml.) and washed with $H_2O$ (150 ml.), the combined acid extracts and $H_2O$ wash were basified with sodium bicarbonate and extracted with ethyl acetate (3 x 350 ml.). The combined extracts were washed twice with $H_2O$, dried over sodium sulfate and concentrated in vacuo to yield 6.3 g (47%) of product as an isomeric mixture.

The isomeric mixture of 5,6-dihydro-6-allyloxymethyl-4-ethylamino-4H-thieno[2,3-b]thiopyran-2-sulfonamide-7,7-dioxide (6.2 g.) was chromatographed on silica gel on a 100 mm. diameter Still column, eluting with chloroform/methane/ammonium hydroxide, 95:5:0.5. After re-chromatographing the fractions containing a mixture of isomers, a total of 1.2 g. of the pure α-isomer was obtained, along with 1.6 g of the pure β-isomer.

The α-isomer (1.2 g., 0.0032 m) was dissolved in absolute ethanol (10 ml.), 5.95 N ethanolic hydrogen chloride (1.0 ml., 0.0060 m) was added and the solution was diluted to incipient turbidity with anhydrous ether. The resultant hydrochloride salt was recrystallized from absolute ethanol (10 ml.) - anhydrous ether (8 ml.) to yield 0.81g of an analytical sample of the α-isomer melting at 148-150°C.

| Anal. Calc'd for $C_{13}H_{20}N_2O_5S_3 \cdot HCl$: | | | |
|---|---|---|---|
| | C, 37.45; | H, 5.08; | N, 6.72 |
| Found: | C, 37.21; | H, 5.04; | N, 6.69 |

The β-isomer (1.6 g., 0.0042m) was dissolved in absolute ethanol (10 ml.), 5.95 N ethanolic hydrogen chloride (1.2 ml., 0.0071 m) was added and the solution diluted to incipient cloudiness with anhydrous ether. The product was recrystallized from absolute ethanol (10 ml.)- anhydrous ether (12 ml.) to yield 1.13 g of the analytically pure hydrochloride salt of the β-isomer, melting at 204-205.5°C.

| Anal. Calc'd for $C_{13}H_{20}N_2O_5S_3 \cdot HCl$: | | | |
|---|---|---|---|
| | C, 37.45; | H, 5.08; | N, 6.72 |
| Found: | C, 37.75; | H, 4.72; | N, 6.89 |

EXAMPLE 40

5,6 Dihydro-4-ethylamino-6-(3-hydroxypropoxy)methyl-4H-thieno[2,3-b]thiopyran-2-sulfonamide-7,7-dioxide hydrochloride (α-isomer)

Step A: 5,6-Dihydro-6-allyloxymethyl-4-methoxyethoxymethoxy-4H-thieno[2,3-b]-thiopyran

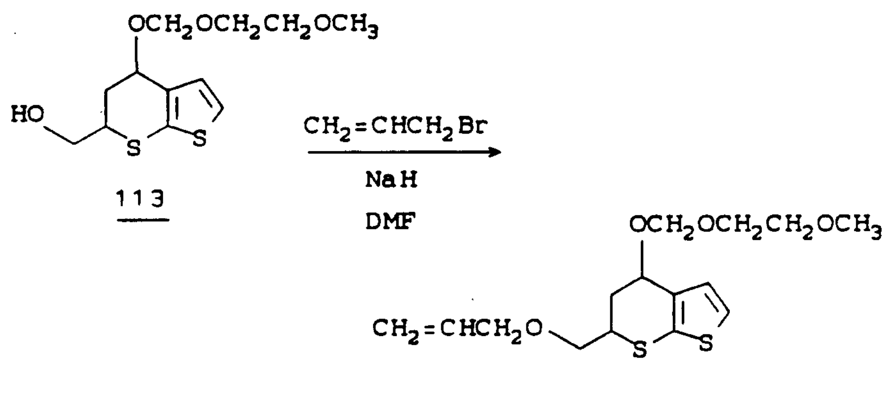


114

Sodium hydride, 50% suspension in mineral oil (7.2 g, 0.15m), was washed with hexane to remove mineral oil. The washed solid was suspended in dry dimethylformamide, 100 ml., and stirred at ambient temperature in a nitrogen atmosphere while a solution of 5,6-dihydro-6-hydroxymethyl-4-methoxyethoxymethoxy-4H-thieno[2,3-b]thiopyran, 35.0 g. (0.12 m), in dry dimethylformamide, 100 ml, was added over 10 minutes. After stirring at ambient temperature for 1.5 hours, allyl bromide, 29.0g. (0.24 m), was added over 15 minutes and the mixture was stirred at ambient termperature for 16 hours. The reaction mixture was concentrated in vacuo and the residue was suspended in 250 ml. $H_2O$ containing 30 ml. of 6N hydrochloric acid. The mixture was extracted with ethyl acetate (300 ml. and 2 x 250 ml.), the combined extracts were washed successively with brine, saturated sodium bicarbonate, 10% sodium bisulfite, brine, and then dried over sodium sulfate. After filtering, the filtrate was concentrated in vacuo to yield 36.94g. (93%) of product as a brown, fluid oil.

Step B: 5,6-Dihydro-6-allyloxymethyl-4-methoxyethoxymethoxy-4H-thieno[2,3-b] thiopyran-2-sulfonamide

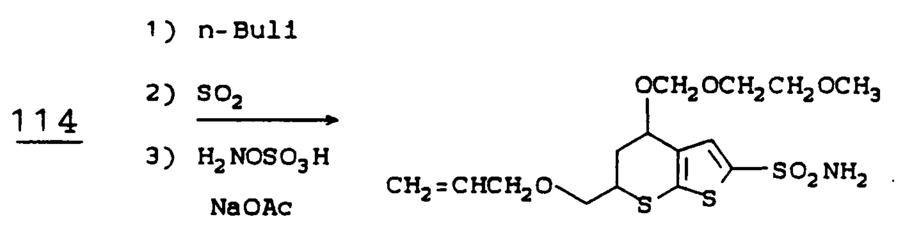


115

A solution of 5.6-dihydro-6-allyloxymethyl-4-methoxyethoxymethoxy-4H-thieno[2,3-b]thiopyran, 13.2 g (0.040m), in dry tetrahydrofuran, 75 ml., was cooled to -78°C with stirring under nitrogen and 1.6 M n-butyllithium, 30ml. (0.048m), was added over 30 minutes keeping the temperature below -60°C. After stirring at -78°C for one hour, sulfur dioxide was passed over the surface of the reaction mixture intermittently over one hour while maintaining the temperature below -45°. The mixture was stirred at -78°C for one hour and then allowed to warm to ambient temperature over 30 minutes. The mixture was concentrated in vacuo and the residue treated with $H_20$ containing sodium acetate, 9.0 g. (0.11 m), and stirred with ice-bath cooling while hydroxylamine-O-sulfonic acid, 11.3 g. (0.10 m) was added over 10 minutes. An additional 3.1 g. (0.038m) sodium acetate was added and the mixture was stirred at ambient temperature for 17 hours. Saturated sodium bicarbonate solution, 80 ml., was added and the mixture was extracted with chloroform (200 ml. and 2 x 150 ml.). The combined extracts were washed twice with brine, dried over sodium sulfate and concentrated in vacuo to yield 15.43 g. (94%) of viscous, brown oily product, which was used in the subsequent reaction without further purification.

Step C: 5,6-Dihydro-4-acetamido-6-allyloxymethyl-4H-thieno[2,3-b]thiopyran-2-sulfonamide

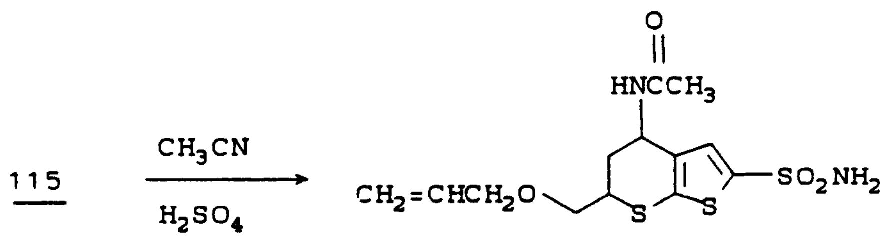


116

A solution of 5,6-dihydro-6-allyloxymethyl-4-methoxyethoxymethoxy-4H-thieno[2,3-b]thiopyran-2-sulfonamide, 12.6 g (0.031 m) in acetonitrile, 105 ml., was cooled to 0°C with stirring under nitrogen while concentrated sulfuric acid, 30.4 g. (0.31 m) was added dropwise over 30 minutes. The mixture was stirred at 0°C for 30 minutes and then at ambient temperature for 22 hours. The mixture was poured into ice and $H_2O$ (225 ml.), rendered basic by the slow addition of sodium bicarbonate, 135 g., and extracted with ethyl acetate (300 ml. and 2 x 150 ml.). The combined extracts were washed with brine, dried over sodium sulfate and concentrated in vacuo to yield tan solid product weighing 8.59 g. (70%).

Step D: 5,6-Dihydro-4-acetamido-6-(3-hydroxypropoxy)methyl-4H-thieno[2,3-b]thiopyran-2-sulfonamide

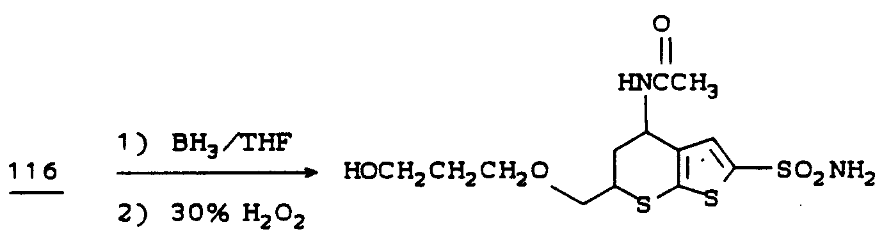


117

A solution of 5,6-dihydro-4-acetamido-6-allyloxymethyl-4H-thieno[2,3-b]thiopyran-2-sulfonamide, 2.5 g. (0.0069 m), in dry tetrahydrofuran, 125 ml., was cooled to -5°C with stirring under nitrogen while 1.0 M borane-tetrahydrofuran complex, 30 ml. ( 0.030m) was added over 5 minutes. After stirring at ambient temperature for 30 minutes, an additional 6 ml. of 1.0 M borane-tetrahydrofuran complex was added in one portion and reaction at ambient temperature was continued for 30 minutes more. The reaction mixture was cooled to -5°C and 5N sodium hydroxide, 14 ml. (0.07 m), was added dropwise, followed by the slow addition of 10% hydrogen peroxide, 3.97 g. (0.035 m). After stirring at ambient temperature for 16 hours, 10% sodium sulfite, (15 ml.), was added. The mixture was acidified with 6N hydrochloric acid, 12 ml., and then basified with saturated sodium bicarbonate. After concentration in vacuo to remove tetrahydrofuran below 30°C, the aqueous suspension was extracted with ethyl acetate (3 x 75 ml.), the combined extracts were washed twice with brine, dried over sodium sulfate and evaporated in vacuo to yield 1.74 g. (66%) of product.

Step E: 5,6-Dihydro-4-acetamido-6-(3-hydroxypropoxy)methyl-4H-thieno[2,3-b]thiopyran-2-sulfonamide-7,7-dioxide

117

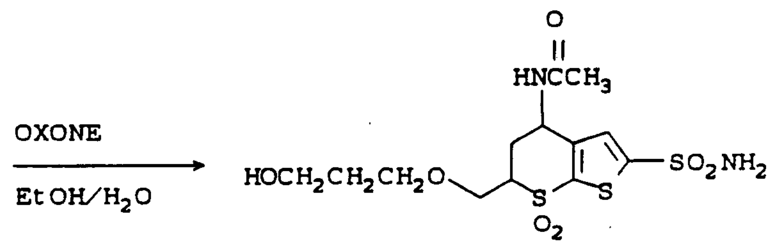


118

A solution of 5,6-dihydro-4-acetamido-6-(3-hydroxypropoxy)methyl-4H-thieno[2,3-b]thiopyran-2-sulfonamide, 1.60 g. (0.0042 m), in ethanol, 15 ml., and $H_20$, 6 ml., was stirred with OXONE®, 3.07 g. (0.0050 m), at ambient temperture for 3 hours. An additional 0.65 g. of OXONE® was added and stirring was continued for 1.5 hours more. The mixture was rendered basic wih sodium bicarbonate and filtered. The filtrate was concentrated in vacuo to remove ethanol and leave an aqueous suspension. The filtered solid was washed with ethyl acetate, 100 ml, and the washings were used to extract the above aqueous suspension, the aqueous layer was separated and extracted with ethyl acetate (2 x 75 ml.) the combined extracts were washed with brine and dried over sodium sulfate. Evaporation in vacuo afforded 0.77 g. (45%) of product.

Step F: 5,6-Dihydro-4-ethylamino-6-(3-hydroxy-propoxy)methyl-4H-thieno[2,3-b]thiopyran-2-sulfonamide-7,7-dioxide (α-Isomer)

118

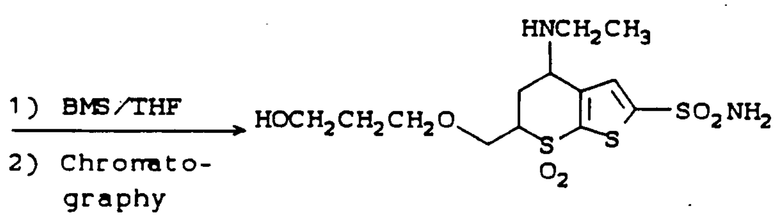


119

α-isomer

A solution of 5,6-dihydro-4-acetamido-6-(3-hydroxypropoxy)methyl-4H-thieno[2,3-b]thiopyran-2-sulfonamide-7,7-dioxide, 0.75 g. (0.0018 m) in dry tetrahydrofuran, 10 ml., was stirred at ambient temperature under nitrogen while 10 M borane-methylsulfide complex, 1.8 ml. (0.018 m.), was added over 20 minutes. The mixture was refluxed over 2 hours, and then dimethylsulfide was distilled through a Vigreaux column with continual heating for 2 hours more. With cooling in an ice-acetone bath, excess borane-methylsulfide was decomposed by the dropwise addition of absolute methanol, 2 ml., followed by 6N hydrochloric acid, 9 ml. The mixture was heated on a steam bath with stirring for 30 minutes and then concentrated in vacuo to remove tetrahydrofuran and methanol. Ethyl acetate, 15 ml., was added to the aqueous suspension which was rendered basic with sodium bicarbonate. The aqueous layer was re-extracted with ethyl acetate (2 x 25 ml.), the combined extracts were washed successively with saturated sodium bicarbonate and with brine, dried over sodium sulfate and concentrated in vacuo to yield 0.56 g (78%) of oily product.

Chromotography on silica gel on a 40 mm. diameter Still column, eluting with chloroform/methanol/ammonium hydroxide, 90:10:1, afforded 0.18 g. of the α-isomer which was dissolved in absolute ethanol, 3 ml., treated with 5.95 N

ethanolic hydrogen chloride, 0.1 ml., and diluted to incipient turbidity with anhydrous ether. The resultant crystalline hydrochloride salt weighed 0.14 g. and melts at approximately 120°C (with decomposition).

| Anal. Calcd. for $C_{13}H_{22}N_2O_6S_3$ • HCl: | | | |
|---|---|---|---|
| | C, 35.89; | H, 5.33; | N,6.44 |
| Found: | C, 35.63; | H, 5.40; | N, 6.26 |

EXAMPLE 42

5,6-Dihydro-4-ethylamino-6-methoxymethyl-6-propyl-4H-thieno[2,3-b]thiopyran-2-sulfonamide-7,7-dioxide hydrochloride

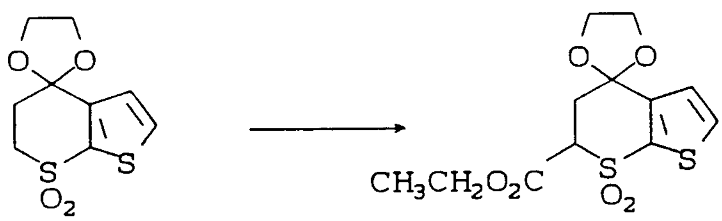

94 121

Step A: 5,6-Dihydro-7,7-dioxo-6-ethoxycarbonyl-4H-thieno[2,3-b]thiopyran-4-one, ethylene ketal

To a stirred solution of 5,6-dihydro-7,7-dioxo-4H-thieno[2,3-b]thiopyran-4-one, ethylene ketal (12.3g, 50 mmol) in dry THF (250 mL) under nitrogen at -30°C, was added a solution of lithium bis(trimethylsilyl)amide in hexane (1M, 110 mL, 110 mmols) over 5-10 minutes. After 0.5 hour at -30°C, a solution of diethyl carbonate (9.8g, 83 mmols) in THF (25 mL) was added, and the reaction mixture was allowed to warm to 10°C. The reaction mixture was mixed with 10% $NH_4Cl$ solution (500 mL) and the THF was removed under reduced pressure. The resulting aqueous solution was extracted with EtOAc (3 x 300 mL), and the combined organic extracts were washed with $H_2O$ (2 x 100 mL), brine (2 x 150 mL) and dried ($Na_2SO_4$). Removal of the dried filtered solvent and recrystallization of the residue (11.3g) from 1-chlorobutane gave pure title compound, mp 131°C.

| Anal., Calcd. for $C_{12}H_{14}O_6S_2$: | | |
|---|---|---|
| | C, 45.27; | H, 4.43 |
| Found: | C, 44.91; | H, 4.32 |

Step B: 5,6-Dihydro-7,7-dioxo-6-ethoxycarbonyl-6-propyl-4H-thieno[2,3-b]thiopyran-4-one, ethylene ketal

121

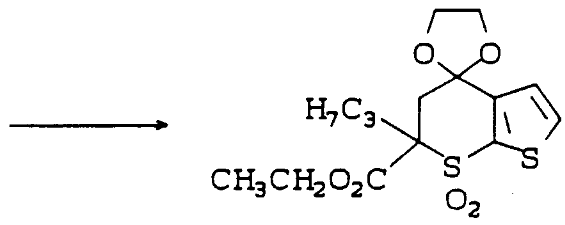

122

A solution of product from Step A (3.18g 10 mmols) in DMF (10 mL) was added to a stirred mixture of sodium hydride (0.50g, 60% dispersion, 12.5 mmols) in DMF (30 mL). After complex addition the reaction mixture was stirred for 0.5 hour and propyl bromide (1.35g, 11 mmols) was added by pipette. After an additional two hours at ambient temperature, another charge of propyl bromide was added (1.35g, 11 mmols). After stirring for an additional 18 hours, the reaction mixture was diluted with $H_2O$ (1.50 mL) and extracted with ether (3 x 75 mL). The combined ether extracts were washed with $H_2O$ (2 x 50 mL), brine (2 x 50 mL) and dried ($Na_2SO_4$). Removal of the filtered dried solvent under reduced pressure gave an opaque white gum. Trituration with hexane gave 2.6g of the title compound as a white solid, used directly in the next step.

Step C: 5,6-Dihydro-7,7-dioxo-6-hydroxymethyl-6-propyl-4H-thieno[2,3-b]thiopyran-4-one, ethylene ketal

122

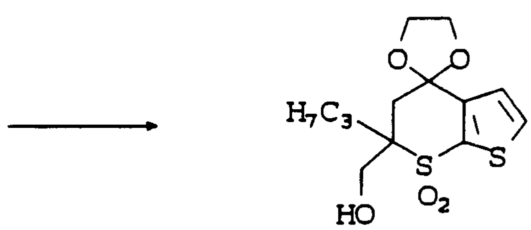

123

To a stirred, cooled suspension of lithium aluminum hydride (356 mg) in ether (15 mL) was added a solution of the product from step B (2.6g) in THF (15 ml) over a 3/4 hour period. The reaction mixture was stirred at 25°C overnight, cooled in ice, then slowly treated with $H_2O$ (0.357 ml), 6N NaOH (0.446 ml), then more $H_2O$ (1.25 ml). The salts were filtered, the solution dried over $Na_2SO_4$ and evaporated *in vacuo* to leave 1.2 g of the title compound as a yellow oil.

Step D: 5,6-Dihydro-7,7-dioxo-6-methoxymethyl-6-propyl-4H-thieno[2,3-b]thiopyran-4-one, ethylene ketal

123

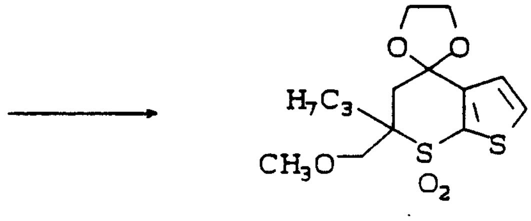

124

Under nitrogen, sodium hydride (50% in oil, 230 mg) was suspended with stirring in cold THF (8 ml). A solution of 5,6-dihydro-7,7-dioxo-6-hydroxymethyl-6-propyl-4H-thieno[2,3-b]thiopyran-4-one, ethylene ketal (1.3g) in THF (5 ml) was added over a 1/2 hour period. The reaction mixture was stirred for 1/2 hour at 25°C then methyl iodide (1.2g) was added. After 1 hour the reaction mixture was cooled in ice, and methanol (1 ml) was added over 5 minutes. The solvents were evaporated, the residue dissolved in ethyl acetate, washed with $H_2O$ then brine, dried over $Na_2SO_4$ and evaporated in vacuo. Crystallization of the residue from ethyl acetate-hexane gave 1.2g of the title compound which melts at 90-92°C.

Step E: 5,6-Dihydro-7,7-dioxo-6-methoxymethyl-6-propyl-4H-thieno[2,3-b]thiopyran-2-sulfonamide-4-one, ethylene ketal

124

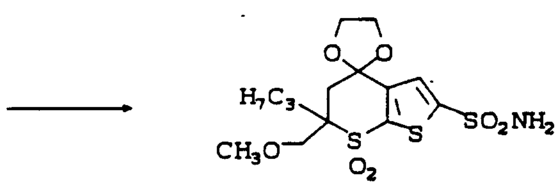

125

Under nitrogen a stirred solution of 5,6-dihydro-7,7-dioxo-6-methoxymethyl-6-propyl-4H-thieno[2,3-b]thiopyran-4-one, ethylene ketal (10.3g) in THF (90 ml) was cooled to -78°C. A solution of 2.5N butyl lithium in THF (16.2 ml) was added over 20 minutes. Stirring was continued for 20 minutes then the nitrogen inlet was replaced with an $SO_2$ inlet and $SO_2$ was added at such a rate that the temperature rose to -57° then dropped back to -78°C. The cooling bath was removed and stirring was continued for 1 hour. The solvents were removed in vacuo, the residue stirred in an ice bath and treated with a solution of sodium acetate (7.9g) in $H_2O$ (72 ml) then hydroxylamine-O-sulfonic acid (9.2g). Stirring was continued for 45 minutes, the solution made basic with aqueous $NaHCO_3$, extracted with ethyl acetate which was washed with brine, dried over $Na_2SO_4$ and evaporated to give 11g of the title compound which was used without purification in the next step.

Step F: 5,6-Dihydro-7,7-dioxo-6-methoxymethyl-6-propyl-4H-thieno[2,3-b]thiopyran-2-sulfonamide-4-one

125

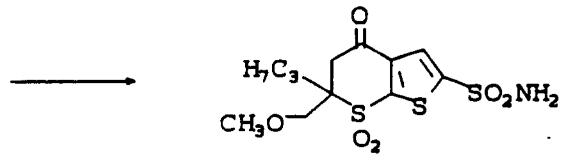

126

A solution of 5,6-dihydro-7,7-dioxo-6-methoxymethyl-6-propyl-4H-thieno[2,3-b]thiopyran-2-sulfonamide-4-one, ethylene ketal (11g), THF (150 ml) and 6N HCl (150 ml) was heated at reflux for 45 minutes. The THF was evaporated in vacuo and the acid solution was extracted with ethyl acetate. The organic phase was washed with brine, dried over $Na_2SO_4$ and evaporated in vacuo. Trituration of the residue with butyl chloride gave 8g of the title compound as a white solid which melts at 197°C.

Step G: 5,6-Dihydro-4-hydroxy-6-methoxymethyl-6-propyl-4H-thieno[2,3-b]thiopyran-2-sulfonamide-7,7-dioxide

126

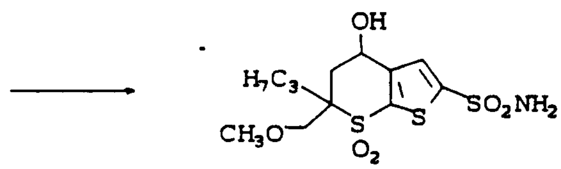

127

To a stirred suspension of 5,6-dihydro-7,7-dioxo-6-methoxymethyl-6-propyl-4H-thieno[2,3-b]thiopyran-2-sulfonamide-4-one (8g) in methanol (400 ml) was added sodium borohydride (0.83g) over a 5 minute period. After 1/2 hour the methanol was evaporated, the residue dissolved in ethyl acetate, washed with 2N HCl, $H_2O$, brine, dried over $Na_2SO_4$ and evaporated in vacuo to provide 8.6g the title compound as a foam.

Step H: N'-(5,6-Dihydro-4-hydroxy-6-methoxymethyl-6-propyl-4H-thieno[2,3-b]thiopyran-2-sulfonyl)-N,N-dimethylformamidine-7,7-dioxide

127

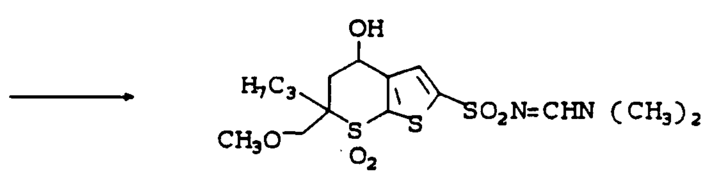

128

A solution of 5,6-dihydro-4-hydroxy-6-methoxymethyl-6-propyl-4H-thieno[2,3-b]thiopyran-2-sulfonamide-7,7-dioxide (8.6g) and dimethylformamide dimethylacetal (4.3 ml) in acetonitrile (400 ml) was stirred for 1 1/2 hours and evaporated in vacuo. The residue was dissolved in ethyl acetate, washed with 1N HCl $H_2O$, and brine, then dried over

$Na_2SO_4$ to give 9.6g of the title compound as an oil.

<u>Step I</u>: <u>N'-(5,6-Dihydro-4-methanesulfonyloxy-6-methoxymethyl-6-propyl-4H-thieno[2,3-b]thiopyran-2-sulfonyl)-N,N-dimethylformamidine-7,7-dioxide</u>

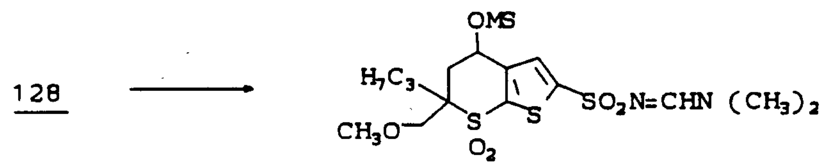

129

To a stirred solution of N'-(5,6-dihydro-4-hydroxy-6-methoxymethyl-6-propyl-4H-thieno[2,3-b]thiopyran-2-sulfonyl)-N,N-dimethylformamidine-7,7-dioxide (9.6g) in THF (175 ml) was added triethylamine (3.9 m) and methanesulfonic anhydride. After 3 hours the THF was evaporated, the residue treated with $H_2O$ (100 ml) and extracted with ethyl acetate (2 x 100 ml). The organic phase was washed with brine, dried over $Na_2SO_4$, and evaporated <u>in vacuo</u> to give 9g of the title compound as an oil.

<u>Step J</u>: <u>N'-(4-Azido-5,6-dihydro-6-methoxymethyl-6-propyl-4H-thieno[2,3-b]thiopyran-2-sulfonyl)-N,N-dimethylformamidine-7,7-dioxide</u>

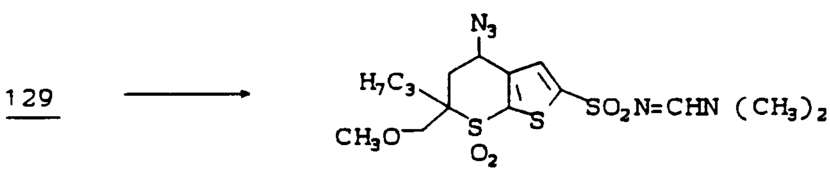

130

Under nitrogen a solution of N'-(5,6-dihydro-4-methanesulfonyloxy-6-methoxymethyl-6-propyl-4H-thieno[2,3-b]thiopyran-2-sulfonyl)-N N-dimethylformamidine-7,7-dioxide (9g) in DMSO (230 ml) was treated with sodium azide (1.62g) and stirred at 25°C for 3 hours. Additional sodium azide (0.6g) was added and stirring continued for 2 hours. The reaction mixture was poured into ice $H_2O$ (300 ml) and extracted with ethyl acetate (2 x 150 ml), the extract was washed with $H_2O$ and brine, dried over $Na_2SO_4$ and evaporated <u>in vacuo</u> to give 7.7g of the title compound as a foam.

Step K: N'-(4-Amino-5,6-dihydro-6-methoxymethyl-6-propyl-4H-thieno[2,3-b]thiopyran-2-sulfonyl)-N,N-dimethylformamidine-7,7-dioxide

130

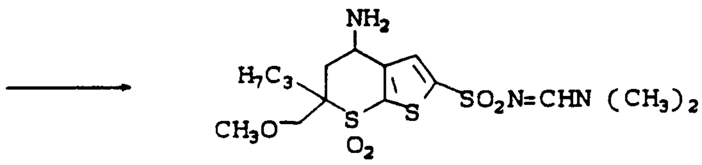

131

A solution of N'-(4-azido-5,6-dihydro-6-methoxymethyl-6-propyl-4H-thieno[2,3-b]thiopyran-2-sulfonyl)-N,N-dimethylformamidine-7,7-dioxide (7.7g) and triphenylphosphine (4.7g) in THF (230 ml) was stirred at 25°C for 20 hours. Water (70 ml) was added and the reaction mixture was heated at reflux for 5 hours. The THF was evaporated in vacuo and the aqueous phase extracted with ethyl acetate which was washed with brine, dried over $Na_2SO_4$ and evaporated in vacuo to give 7.4g of a mixture of α and β isomers. The isomers were separated by chromatography on silica eluting with $CHCl_3$-$CH_3OH$ 22:1.

Step L: 4-Amino-5,6-dihydro-6-methoxymethyl-6-propyl-4H-thieno[2,3-b]thiopyran-2-sulfonamide-7,7-dioxide hydrochloride

131

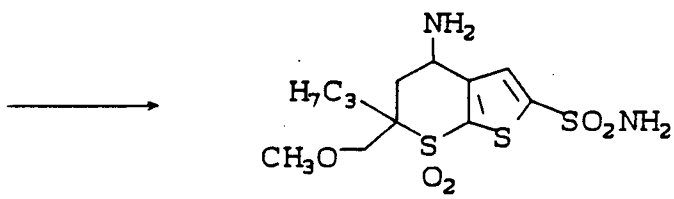

132

A solution of N'-(4-amino-5,6-dihydro-6-methoxymethyl-6-propyl-4H-thieno[2,3-b]thiopyran-2-sulfonyl)-N,N-dimethylformamidine-7,7-dioxide (300 mg) in THF (25 ml) and 6N HCl (10 ml) was heated at reflux for 5 hours. The THF was evaporated in vacuo and the residue made basic with $NaHCO_3$, extracted with ethyl acetate, washed with brine, dried over $Na_2SO_4$ and evaporated in vacuo. The residue was dissolved in ethanol (3 ml), treated with a slight excess of ethanolic HCl, then with ether and refrigerated overnight to give the title compound.

| Analysis calc'd for $C_{12}H_{20}N_2O_5S_3$ • HCl • 1/4 $C_2H_5OH$, | | | |
|---|---|---|---|
| | C, 36.05; | H, 5.44; | N, 6.73; |
| Found (α-isomer) | C, 36.15, | H, 5.10; | N, 6.76 |

| Analysis calc'd for $C_{12}H_{20}N_2O_5S_3 \cdot HCl$, | | | |
|---|---|---|---|
| | C, 35.59; | H, 5.23; | N, 6.92; |
| Found (β-isomer) | C, 35.61; | H, 5.12; | N, 6.94 |

EXAMPLE 43

5,6-Dihydro-4-ethylamino-6-methoxymethyl-6-propyl-4H-thieno[2,3-b]thiopyran-2-sulfonamide-7,7-dioxide hydrochloride

131

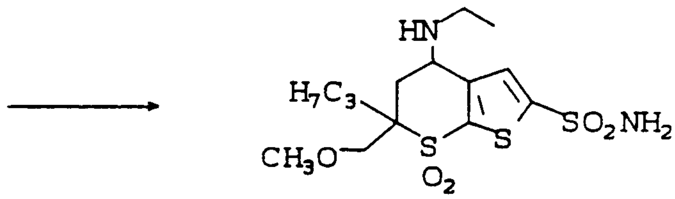

133

Acetaldehyde (0.42 ml) was added under nitrogen to a solution of N'-(4-amino-5,6-dihydro-6-methoxymethyl-6-propyl-4H-thieno[2,3-b]thiopyran-2-sulfonyl)-N,N-dimethylformamidine-7,7-dioxide (2.3g) in THF (28 ml). After 45 minutes the reaction mixture was added dropwise to a stirred suspension of sodium borohydride (1.07g) in ethanol (28 ml) which had been cooled to 0°C. After 30 minutes the reaction was quenched with 3N HCl and the THF and ethanol were evaporated in vacuo. The residue was made basic with $NaHCO_3$, and extracted with ethyl acetate which was washed with brine, dried over $Na_2SO_4$, evaporated in vacuo and purified by column chromatography on silica ($CHCl_3$-$CH_3OH$ 18:1). The pertinent fractions were evaporated, the residue was dissolved in ethanol (8 ml) and treated with a slight excess of ethanolic HCl then ether to give 1.2g of the title compound.

| Analysis calc. for $C_{14}H_{24}N_2O_5S_3 \cdot HCl \cdot 1/2\ C_2H_5OH$, | | | |
|---|---|---|---|
| | C, 39.50; | H, 6.18; | N, 6.14; |
| Found (α-isomer) | C, 39.83; | H, 5.81; | N, 6.43 |
| Found (β-isomer) | C, 39.41; | H, 5.80; | N, 6.42 |

EXAMPLE 45

Pharmaceutical Formulations

| | | |
|---|---|---|
| Active compound | 1 mg | 15 mg |
| Monobasic sodium phosphate $2H_2O$ | 9.38 mg | 6.10 mg |
| Dibasic sodium phosphate $.12H_2O$ | 28.48 mg | 16.80 mg |
| Benzalkonium chloride | 0.10 mg | 0.10 mg |
| Water for injection q.s. and. | 1.0 mg | 1.0 mg |

The active compound, phosphate buffer salts, and benzalkonium chloride are added to and dissolved in water. The pH of the composition is adjusted to 6.8 and diluted to volume. The composition is rendered sterile by ionizing radiation.

| | |
|---|---|
| Active compound | 5 mg |
| petrolatium q.s. and. | 1 gram |

The compound and the petrolatum are aseptically combined.

| | |
|---|---|
| Active compound | 1 mg |
| Hydroxypropylcellulose q.s. | 12 mg |

Ophthalmic inserts are manufactured from compression molded films which are prepared on a Carver Press by subjecting the powdered mixture of the above ingredients to a compressional force of 12,000 lbs. (gauge) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then capped. The vials containing the hydrate insert are then autoclaved at 250°F for 1/2 hour.

**Claims**

**Claims for the following Contracting States : AT, BE, CH, DE, DK, FR, GB, IT, LI, LU, NL, SE**

**1.** A compound of the structural formula:

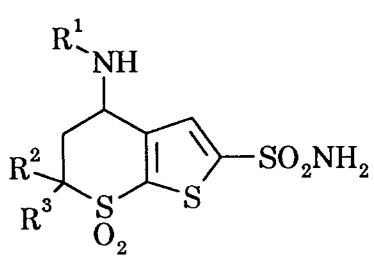

its isomers, or a pharmaceutically acceptable salt thereof wherein:

$R^1$ is $C_{1-6}$alkyl;

$R^2$ is hydrogen or $C_{1-6}$alkyl;
$R^3$ is

(a) $C_{1-5}$alkoxy-$C_{1-5}$alkyl,
(b) hydroxy-$C_{1-5}$alkoxy-$C_{1-5}$alkyl,
(c) $C_{1-5}$alkoxy-$C_{1-5}$alkoxy-$C_{1-5}$alkyl,
(d) hydroxy-$C_{1-5}$alkylamino-$C_{1-5}$alkyl,
(e) hydroxy-$C_{1-5}$alkylthio-$C_{1-5}$alkyl, or
(f) $C_{2-5}$alkenyloxy-$C_{1-5}$alkyl.

**2.** The compound of Claim 1 which is

a) 5,6-dihydro-6-(2-ethoxyethyl)-4-ethylamino-4H-thieno[2,3-b]thiopyran-2-sulfonamide-7,7-dioxide;
b) 5,6-dihydro-6-ethoxymethyl-4-ethylamino-4H-thieno[2,3-b]thiopyran-2-sulfonamide-7,7-dioxide;
c) 5,6-dihydro-6-methoxymethyl-4-(n-propylamino)-4H-thieno[2,3-b]thiopyran-2-sulfonamide-7,7-dioxide;
d) 5,6-dihydro-6-(3-methoxypropyl)-4-(methylamino)-4H-thieno[2,3-b]thiopyran-2-sulfonamide-7,7-dioxide;
e) 5,6-dihydro-6-(3-methoxypropyl)-4-(n-propylamino)-4H-thieno[2,3-b]thiopyran-2-sulfonamide-7,7-dioxide;
f) 5,6-dihydro-4H-4-ethylamino-6-(3-methoxypropyl)-thieno[2,3-b]thiopyran-2-sulfonamide-7,7-dioxide;
g) 5,6-dihydro-6-(3-methoxypropyl)-4-propylamino-4H-thieno[2,3-b]thiopyran-2-sulfonamide-7,7-dioxide;
h) 5,6-dihydro-4-ethylamino-4H-6-(2-hydroxyethylamino)methylthieno[2,3-b]thiopyran-2-sulfonamide-7,7-dioxide;
i) 5,6-dihydro-6-allyloxyymethyl-4-ethylamino-4H-thieno[2,3-b]thiopyran-2-sulfonamide-7,7-dioxide;
j) 5,6-dihydro-6-(3-allyloxy)propyl-4-propylamino-4H-thieno[2,3-b]thiopyran-2-sulfonamide-7,7-dioxide;
k) 5,6-dihydro-6-[3-(2-methoxy)ethoxy]propyl-4-propylamino-4H-thieno[2,3-b]thiopyran-2-sulfonamide-7,7-dioxide;
l) 5,6-dihydro-4-ethylamino-6-[3-(2-methoxy)ethoxy]-propyl-4H-thieno[2,3-b]thiopyran-2-sulfonamide-7,7-dioxide;
m) 5,6-dihydro-6-[3-(2-ethoxy)ethoxy]propyl-4-propylamino-4H-thieno[2,3-b]thiopyran-2-sulfonamide-7,7-dioxide;
n) 5,6-dihydro-4-ethylamino-6-[2-(2-hydroxyethylthio)ethyl]-4H-thieno[2,3-b]thiopyran-2-sulfonamide-7,7-dioxide;
o) 5,6-dihydro-4-propylamino-6-(2-methoxyethoxy)methyl-4H-thieno[2,3-b]thiopyran-2-sulfonamide-7,7-dioxide;
p) 5,6-dihydro-4-ethylamino-4H-6-(2-hydroxyethyl)aminomethylthieno[2,3-b]thiopyran-2-sulfonamide-7,7-dioxide;
s) 5,6-dihydro-6-[2-(2-methoxy)ethoxy]ethyl-4-ethylamino-4H-thieno[2,3-b]thiopyran-2-sulfonamide-7,7-dioxide;
t) 5,6-dihydro-6-[3-(3-methoxy)propoxy]propyl-4-propylamino-4H-thieno[2,3-b]thiopyran-2-sulfonamide-7,7-dioxide; or
u) 5,6-dihydro-4-propylamino-6-[3-(2-hydroxyethylthio)propyl]-4H-thieno[2,3-b]thiopyran-2-sulfonamide-7,7-dioxide;

or a pharmaceutically acceptable salt thereof.

**3.** The compound of Claim 2 which is the
trans diastereomer of 5,6-dihydro-6-(2-ethoxyethyl)-4-ethylamino-4H-thieno[2,3-b]thiopyran-2-sulfonamide-7,7-dioxide; 5,6-dihydro-4H-4-ethylamino-6-(3-methoxypropyl)thieno[2,3-b]thiopyran-2-sulfonamide-7,7-dioxide; 5,6-dihydro-6-(3-methoxypropyl)-4-propylamino-4H-thieno[2,3-b]thiopyran-2-sulfonamide-7,7-dioxide; 5,6-dihydro-4-ethylamino-4H-6-(2-hydroxyethyl)aminomethylthieno[2,3-b]thiopyran-2-sulfonamide-7,7-dioxide; 5,6-dihydro-6-allyloxymethyl-4-ethylamino-4H-thieno[2,3-b]thiopyran-2-sulfonamide-7,7-dioxide; 5,6-dihydro-6-(3-allyloxy)propyl-4-propylamino-4H-thieno[2,3-b]thiopyran-2-sulfonamide-7,7-dioxide; 5,6-dihydro-6-[3-(2-methoxy)ethoxy]propyl-4-propylamino-4H-thieno[2,3-b]thiopyran-2-sulfonamide-7,7-dioxide; 5,6-dihydro-4-ethylamino-6-[3-(2-methoxy)ethoxy]propyl-4H-thieno[2,3-b]-thiopyran-2-sulfonamide-7,7-dioxide; 5,6-dihydro-6-[3-(2-ethoxy)ethoxy]propyl-4-propylamino-4H-thieno[2,3-b]thiopyran-2-sulfonamide-7,7-dioxide; 5,6-dihydro-4-ethylamino-6-[2-(2-hydroxyethylthio)ethyl]-4H-thieno[2,3-b]-thiopyran-2-sulfonamide-7,7-dioxide; or 5,6-dihydro-4-propylamino-6-(2-methoxyethoxy)methyl-4H-thieno[2,3-b]thiopyran-2-sulfonamide-7,7-dioxide.

**4.** The compound of Claim 2 which is the
trans-(S,S)-enantiomer of 5,6-dihydro-6-(2-ethoxyethyl)-4-ethylamino-4H-thieno[2,3-b]thiopyran-2-sulfonamide-

7,7-dioxide;
5,6-dihydro-4H-4-ethylamino-6-(3-methoxypropyl)-thieno[2,3-b]thiopyran-2-sulfonamide-7,7-dioxide;
5,6-dihydro-6-(3-methoxypropyl)-4-propylamino-4H-thieno[2,3-b]thiopyran-2-sulfonamide-7,7-dioxide;
5,6-dihydro-4-ethylamino-4H-6-(2-hydroxyethylamino)methylthieno-[2,3-b]thiopyran-2-sulfonamide-7,7-dioxide;
5,6-dihydro-6-(3-allyloxy)propyl-4-propylamino-4H-thieno[2,3-b]-thiopyran-2-sulfonamide -7,7-dioxide; 5,6-dihydro-6-[3-(2-methoxy)ethoxy]propyl-4-propylamino-4H-';[ o[2,3-b]thiopyran-2-sulfonamide-7,7-dioxide;
5,6-dihydro-6-[3-(2-ethoxy)ethoxy]propyl-4-propylamino-4H-thieno[2,3-b]thiopyran-2-sulfonamide-7,7-dioxide; 5,6-dihydro-6-(3-hydroxypropyl)-4-propylamino-4H-thieno[2,3-b]thiopyran-2-sulfonamide-7,7-dioxide;
5,6-dihydro-6-[2-(2-methoxy)ethoxy]ethyl-4-ethylamino-4H-thieno[2,3-b]thiopyran-2-sulfonamide-7,7-dioxide;
5,6-dihydro-6-[3-(2-methoxy)ethoxy]propyl-4-ethylamino-4H-thieno[2,3-b]thiopyran-2-sulfonamide-7,7-dioxide;
5,6-dihydro-6-[3-(3-methoxy)propoxy]propyl-4-propylamino-4H-thieno[2,3-b]thiopyran-2-sulfonamide-7,7-dioxide;
or 5,6-dihydro-4-propylamino-6-[3-(2-hydroxyethyithio)propyl]-4H-thieno[2,3-b]thiopyran-2-sulfonamide-7,7-dioxide.

5. The compound of Claim 2 which is the
cis-diasteriomer of 5,6-dihydro-6-(2-ethoxyethyl)-4-ethylamino-4H-thieno[2,3-b]thiopyran -2-sulfonamide-7,7-dioxide; 5,6-dihydro-4H-4-ethylamino-6-(3-methoxypropyl)thieno[2,3-b]thiopyran-2-sulfonamide-7,7-dioxide;
5,6-dihydro-6-(3-methoxypropyl)-4-propylamino-4H-thieno[2,3-b]thiopyran-2-sulfonamide-7,7-dioxide; 5,6-dihydro-4-ethylamino-4H-6-(2-hydroxyethylamino)methylthieno-[2,3-b]thiopyran-2-sulfonamide-7,7-dioxide; 5,6-dihydro-6-allyloxymethyl-4-ethylamino-4H-thieno[2,3-b]thiopyran-2-sulfonamide-7,7-dioxide; or
5,6-dihydro-4-propylamino-6-(2-methoxyethoxy)methyl-4H-thieno[2,3-b]thiopyran-2-sulfonamide-7,7-dioxide.

6. The compound of Claim 4 which is
5,6-dihydro-6-(3-methoxypropyl)-4-propylamino-4H-thieno[2,3-b]thiopyran-2-sulfonamide-7,7-dioxide.

7. An ophthalmological formulation for the treatment of ocular hypertension comprising an ophthalmological carrier and an effective ocular antihypertensive amount of the compound of Claim 1.

8. The use of a compound of Claim 1 for the preparation of a medicament for treating ocular hypertension.

9. The use of a compound of Claim 1 for the preparation of a medicament for treating psoriasis.

10. The use as claimed in Claim 9 wherein the medicament is adapted for administration by topical or oral means.

**Claims for the following Contracting States : ES, GR**

1. A process for preparing of a compound of structural formula:

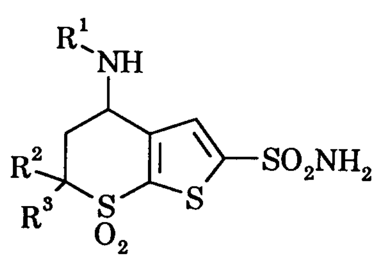

its isomers, or a pharmaceutically acceptable salt thereof wherein:

$R^1$ is $C_{1-6}$alkyl;
$R^2$ is hydrogen or $C_{1-6}$alkyl;
$R^3$ is

(a) $C_{1-5}$alkoxy-$C_{1-5}$alkyl,
(b) hydroxy-$C_{1-5}$alkoxy-$C_{1-5}$alkyl,
(c) $C_{1-5}$alkoxy-$C_{1-5}$alkoxy-$C_{1-5}$alkyl,
(d) hydroxy-$C_{1-5}$alkylamino-$C_{1-5}$alkyl,
(e) hydroxy-$C_{1-5}$alkylthio-$C_{1-5}$alkyl, or

(f) $C_{2-5}$alkenyloxy-$C_{1-5}$alkyl,

which comprises:
reducing the 4-N-acyl group with borane-dimethyl-sulfide complex in an ethereal solvent to provide a compound of formula I,
or alternatively,

a) treating the 4-hydroxy compound with toluene sulfonyl chloride in pyridine at about -20 degrees C to 5 degrees C, and
b) adding an alkylamine at a temperature below about 15 degrees C, followed by warming to about 30 to 60 degrees C for about 5 to 16 hours to provide a compound of formula I,

or alternatively

a) treating a solution of the keto compound with an amine of formula $R^1$ $NH_2$, followed by titanium tetrachloride dropwise, and
b) treating the compound in step a) with a complex metal hydride in excess in a $C_{1-3}$alkanol to obtain a compound of formula I.

**2.** A process as claimed in claim 1 for preparing the compound of Claim 1 which is

a) 5,6-dihydro-6-(2-ethoxyethyl)-4-ethylamino-4H-thieno[2,3-b]thiopyran-2-sulfonamide-7,7-dioxide;
b) 5,6-dihydro-6-ethoxymethyl-4-ethylamino-4H-thieno[2,3-b]thiopyran-2-sulfonamide-7,7-dioxide;
c) 5,6-dihydro-6-methoxymethyl-4-(n-propylamino)-4H-thieno[2,3-b]thiopyran-2-sulfonamide-7,7-dioxide;
d) 5,6-dihydro-6-(3-methoxypropyl)-4-(methylamino)-4H-thieno[2,3-b]thiopyran-2-sulfonamide-7,7-dioxide;
e) 5,6-dihydro-6-(3-methoxypropyl)-4-(n-propylamino)-4H-thieno[2,3-b]thiopyran-2-sulfonamide-7,7-dioxide;
f) 5,6-dihydro-4H-4-ethylamino-6-(3-methoxypropyl)-thieno[2,3-b]thiopyran-2-sulfonamide-7,7-dioxide;
g) 5,6-dihydro-6-(3-methoxypropyl)-4-propylamino-4H-thieno[2,3-b]thiopyran-2-sulfonamide-7,7-dioxide;
h) 5,6-dihydro-4-ethylamino-4H-6-(2-hydroxyethylamino)methylthieno[2,3-b]thiopyran-2-sulfonamide-7,7-dioxide;
i) 5,6-dihydro-6-allyloxymethyl-4-ethylamino-4H-thieno[2,3-b]thiopyran-2-sulfonamide-7,7-dioxide;
j) 5,6-dihydro-6-(3-allyloxy)propyl-4-propylamino-4H-thieno[2,3-b]thiopyran-2-sulfonamide-7,7-dioxide;
k) 5,6-dihydro-6-[3-(2-methoxy)ethoxy]propyl-4-propylamino-4H-thieno[2,3-b]thiopyran-2-sulfonamide-7,7-dioxide;
l) 5,6-dihydro-4-ethylamino-6-[3-(2-methoxy)ethoxy]-propyl-4H-thieno[2,3-b]thiopyran-2-sulfonamide-7,7-dioxide;
m) 5,6-dihydro-6-[3-(2-ethoxy)ethoxy]propyl-4-propylamino-4H-thieno[2,3-b]thiopyran-2-sulfonamide-7,7-dioxide;
n) 5,5-dihydro-4-ethylamino-6-[2-(2-hydroxyethylthio)ethyl]-4H-thieno[2,3-b]thiopyran-2-sulfonamide-7,7-dioxide;
o) 5,6-dihydro-4-propylamino-6-(2-methoxyethoxy)methyl-4H-thieno[2,3-b]thiopyran-2-sulfonamide-7,7-dioxide;
p) 5,6-dihydro-4-ethylamino-4H-6-(2-hydroxyethyl)aminomethylthieno[2,3-b]thiopyran-2-sulfonamide-7,7-dioxide;
s) 5,6-dihydro-6-[2-(2-methoxy)ethoxy]ethyl-4-ethylamino-4H-thieno[2,3-b]thiopyran-2-sulfonamide-7,7-dioxide;
t) 5,6-dihydro-6-[3-(3-methoxy)propoxy]propyl-4-propylamino-4H-thieno[2,3-b]thiopyran-2-sulfonamide-7,7-dioxide; or
u) 5,6-dihydro-4-propylamino-6-[3-(2-hydroxyethylthio)propyl]-4H-thieno[2,3-b]thiopyran-2-sulfonamide-7,7-dioxide;

or a pharmaceutically acceptable salt thereof.

**3.** A process as claimed in claim 1 for preparing the compound of formula (I) which is the trans diastereomer of 5,6-dihydro-6-(2-ethoxyethyl)-4-ethylamino-4H-thieno[2,3-b]thiopyran-2-sulfonamide-7,7-dioxide; 5,6-dihydro-4H-4-ethylamino-6-(3-methoxypropyl)thieno[2,3-b]thiopyran-2-sulfonamide-7,7-dioxide; 5,6-dihydro-6-(3-methoxypropyl)-4-propylamino-4H-thieno[2,3-b]thiopyran-2-sulfonamide-7,7-dioxide; 5,6-dihydro-4-ethylamino-4H-6-(2-hydroxyethyl)aminomethylthieno[2,3-b]thiopyran-2-sulfonamide-7,7-dioxide; 5,6-dihydro-6-allyloxymethyl-4-ethylamino-4H-thieno[2,3-b]thiopyran-2-sulfonamide-7,7-dioxide;

5,6-dihydro-6-(3-allyloxy)propyl-4-propylamino-4H-thieno[2,3-b]thiopyran-2-sulfonamide-7,7-dioxide; 5,6-dihydro-6-[3-(2-methoxy)ethoxy]propyl-4-propylamino-4H-thieno[2,3-b]thiopyran-2-sulfonamide-7,7-dioxide; 5,6-dihydro-4-ethylamino-6-[3-(2-methoxy)ethoxy]propyl-4H-thieno[2,3-b]-thiopyran-2-sulfonamide-7,7-dioxide; 5,6-dihydro-6-[3-(2-ethoxy)ethoxy]propyl-4-propylamino-4H-thieno[2,3-b]thiopyran-2-sulfonamide-7,7-dioxide; 5,6-dihydro-4-ethylamino-6-[2-(2-hydroxyethylthio)ethyl]-4H-thieno[2,3-b]-thiopyran-2-sulfonamide-7,7-dioxide; or 5,6-dihydro-4-propylamino-6-(2-methoxyethoxy)methyl-4H-thieno[2,3-b]thiopyran-2-sulfonamide-7,7-dioxide.

**4.** A process as claimed in claim 1 for preparing the compound of formula (I) which is the trans-(S,S)-enantiomer of 5,6-dihydro-6-(2-ethoxyethyl)-4-ethylamino-4H-thieno[2,3-b]thiopyran-2-sulfonamide-7,7-dioxide; 5,6-dihydro-4H-4-ethytamino-6-(3-methoxypropyl)-thieno[2,3-b]thiopyran-2-sulfonamide-7,7-dioxide; 5,6-dihydro-6-(3-methoxypropyl)-4-propylamino-4H-thieno[2,3-b]thiopyran-2-sulfonamide-7,7-dioxide; 5,6-dihydro-4-ethylamino-4H-6-(2-hydroxyethylamino)-methylthieno-[2,3-b]thiopyran-2-sulfonamide-7,7-dioxide; 5,6-dihydro-6-(3-allyloxy)propyl-4-propylamino-4H-thieno[2,3-b]-thiopyran-2-sulfonamide -7,7-dioxide; 5,6-dihydro-6-[3-(2-methoxy)ethoxy]propyl-4-propylamino-4H-thieno[2,3-b]thiopyran-2-sulfonamide-7,7-dioxide; 5,6-dihydro-6-[3-(2-ethoxy)ethoxy]propyl-4-propylamino-4H-thieno[2,3-b]thiopyran-2-sulfonamide-7,7-dioxide; 5,6-dihydro-6-(3-hydroxypropyl)-4-propylamino-4H-thieno[2,3-b]thiopyran-2-sulfonamide-7,7-dioxide; 5,6-dihydro-6-[2-(2-methoxy)ethoxy]ethyl-4-ethylamino-4H-thieno[2,3-b]thiopyran-2-sulfonamide-7,7-dioxide; 5,6-dihydro-6-[3-(2-methoxy)ethoxy]propyl-4-ethylamino-4H-thieno[2,3-b]thiopyran-2-sulfonamide-7,7-dioxide; 5,6-dihydro-6-[3-(3-methoxy)propoxy]propyl-4-propylamino-4H-thieno[2,3-b]thiopyran-2-sulfonamide-7,7-dioxide; or 5,6-dihydro-4-propylamino-6-[3-(2-hydroxyethylthio)propyl]-4H-thieno[2,3-b]thiopyran-2-sulfonamide-7,7-dioxide.

**5.** A process as claimed in claim 1 for preparing the compound of formula (I) which is the cis-diasteriomer of 5,6-dihydro-6-(2-ethoxyethyl)-4-ethylamino-4H-thieno[2,3-b]thiopyran -2-sulfonamide-7,7-dioxide; 5,6-dihydro-4H-4-ethylamino-6-(3-methoxypropyl)thieno[2,3-b]thiopyran-2-sulfonamide-7,7-dioxide; 5,6-dihydro-6-(3-methoxypropyl)-4-propylamino-4H-thieno[2,3-b]thiopyran-2-sulfonamide-7,7-dioxide; 5,6-dihydro-4-ethylamino-4H-6-(2-hydroxyethylamino)methylthieno-[2,3-b]thiopyran-2-sulfonamide-7,7-dioxide; 5,6-dihydro-6-allyloxymethyl-4-ethylamino-4H-thieno[2,3-b]thiopyran-2-sulfonamide-7,7-dioxide; or 5,6-dihydro-4-propylamino-6-(2-methoxyethoxy)methyl-4H-thieno[2,3-b]thiopyran-2-sulfonamide-7,7-dioxide.

**6.** A process as claimed in claim 1 for preparing the compound of formula (I) which is the 5,6-dihydro-6-(3-methoxypropyl)-4-propylamino-4H-thieno[2,3-b]thiopyran-2-sulfonamide-7,7-dioxide.

## Patentansprüche

**Patentansprüche für folgende Vertragsstaaten : AT, BE, CH, DE, DK, FR, GB, IT, LI, LU, NL, SE**

**1.** Eine Verbindung der Strukturformel:

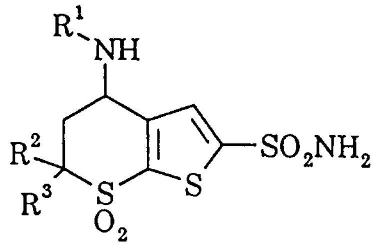

,

ihre Isomere oder ein pharmazeutisch annehmbares Salz davon, worin

$R^1$ $C_{1-6}$-Alkyl ist,
$R^2$ Wasserstoff oder $C_{1-6}$-Alkyl ist,
$R^3$

(a) $C_{1-5}$-Alkoxy-$C_{1-5}$-alkyl,
(b) Hydroxy-$C_{1-5}$-alkoxy-$C_{1-5}$-alkyl,

(c) $C_{1-5}$-Alkoxy-$C_{1-5}$-alkoxy-$C_{1-5}$-alkyl,
(d) Hydroxy-$C_{1-5}$-alkylamino-$C_{1-5}$-alkyl,
(e) Hydroxy-$C_{1-5}$-alkylthio-$C_{1-5}$-alkyl oder
(f) $C_{2-5}$-Alkenyloxy-$C_{1-5}$-alkyl ist.

**2.** Die Verbindung nach Anspruch 1, die

a) 5,6-Dihydro-6-(2-ethoxyethyl)-4-ethylamino-4H-thieno[2,3-b]thiopyran-2-sulfonamid-7,7-dioxid,
b) 5,6-Dihydro-6-ethoxymethyl-4-ethylamino-4H-thieno[2,3-b]thiopyran-2-sulfonamid-7,7-dioxid,
c) 5,6-Dihydro-6-methoxymethyl-4-(n-propylamino)-4H-thieno[2,3-b]thiopyran-2-sulfonamid-7,7-dioxid,
d) 5,6-Dihydro-6-(3-methoxypropyl)-4-(methylamino)-4H-thieno[2,3-b]-thiopyran-2-sulfonamid-7,7-dioxid,
e) 5,6-Dihydro-6-(3-methoxypropyl)-4-(n-propylamino)-4H-thieno[2,3-b]-thiopyran-2-sulfonamid-7,7-dioxid,
f) 5,6-Dihydro-4H-4-ethylamino-6-(3-methoxypropyl)thieno[2,3-b]thiopyran-2-sulfonamid-7,7-dioxid,
g) 5,6-Dihydro-6-(3-methoxypropyl)-4-propylamino-4H-thieno[2,3-b]thiopyran-2-sulfonamid-7,7-dioxid,
h) 5,6-Dihydro-4-ethylamino-4H-6-(2-hydroxyethylamino)methylthieno[2,3-b]-thiopyran-2-sulfonamid-7,7-dioxid,
i) 5,6-Dihydro-6-allyloxymethyl-4-ethylamino-4H-thieno[2,3-b]thiopyran-2-sulfonamid-7,7-dioxid,
j) 5,6-Dihydro-6-(3-allyloxy)propyl-4-propylamino-4H-thieno[2,3-b]thiopyran-2-sulfonamid-7,7-dioxid,
k) 5,6-Dihydro-6-[3-(2-methoxy)ethoxy]propyl-4-propylamino-4H-thieno-[2,3-b]thiopyran-2-sulfonamid-7,7-dioxid,
l) 5,6-Dihydro-4-ethylamino-6-[3-(2-methoxy)ethoxy]propyl-4H-thieno[2,3-b]-thiopyran-2-sulfonamid-7,7-dioxid,
m) 5,6-Dihydro-6-[3-(2-ethoxy)ethoxy]propyl-4-propylamino-4H-thieno[2,3-b]-thiopyran-2-sulfonamid-7,7-dioxid,
n) 5,6-Dihydro-4-ethylamino-6-[2-(2-hydroxyethylthio)ethyl]-4H-thieno-[2,3-b]thiopyran-2-sulfonamid-7,7-dioxid,
o) 5,6-Dihydro-4-propylamino-6-(2-methoxyethoxy)methyl-4H-thieno[2,3-b]-thiopyran-2-sulfonamid-7,7-dioxid,
p) 5,6-Dihydro-4-ethylamino-4H-6-(2-hydroxyethyl)aminomethylthieno[2,3-b]-thiopyran-2-sulfonamid-7,7-dioxid,
s) 5,6-Dihydro-6-[2-(2-methoxy)ethoxy]ethy]-4-ethylamino-4H-thieno[2,3-b]-thiopyran-2-sulfonamid-7,7-dioxid,
t) 5,6-Dihydro-6-[3-(3-methoxy)propoxy]propyl-4-propylamino-4H-thieno-[2,3-b]thiopyran-2-sulfonamid-7,7-dioxid oder
u) 5,6-Dihydro-4-propylamino-6-[3-(2-hydroxyethylthio)propyl]-4H-thieno-[2,3-b]thiopyran-2-sulfonamid-7,7-dioxid

oder ein pharmazeutisch annehmbares Salz davon ist.

**3.** Die Verbindung nach Anspruch 2, die das trans-Diastereomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)thieno[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethyl)aminomethylthieno-[2,3-b]thiopyran-2-sulfonamid-7,7-dioxid, 5,6-Dihydro-6-allyloxymethyl-4-ethylamino-4H-thieno[2,3-b]thiopyran-2-sulfonamid-7,7-dioxid, 5,6-Dihydro-6-(3-allyloxy)propyl-4-propylamino-4H-thieno[2,3-b]thiopyran-2-sulfonamid-7,7-dioxid, 5,6-Dihydro-6-[3-(2-methoxy)ethoxy]propyl-4-propylamino-4H-thieno[2,3-b]thiopyran-2-sulfonamid-7,7-dioxid, 5,6-Dihydro-4-ethylamino-6-[3-(2-methoxy)ethoxy]propyl-4H-thieno[2,3-b]thiopyran-2-sulfonamid-7,7-dioxid, 5,6-Dihydro-6-[3-(2-ethoxy)ethoxy]propyl4-propylamino-4H-thieno[2,3-b]thiopyran-2-sulfonamid-7,7-dioxid, 5,6-Dihydro-4-ethylamino-6-[2-(2-hydroxyethylthio)ethyl]-4H-thieno[2,3-b]thiopyran-2-sulfonamid-7,7-dioxid oder 5,6-Dihydro-4-propylamino-6-(2-methoxyethoxy)methyl-4H-thieno[2,3-b]thiopyran-2-sulfonamid-7,7-dioxid ist.

**4.** Die Verbindung nach Anspruch 2, die das trans-(S,S)-Enantiomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)-thieno[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethylamino)methythieno[2,3-b]thiopyran-2-sulfonamid-7,7-dioxid, 5,6-Dihydro-6-(3-allyloxy)propyl-4-propylamino-4H-thieno[2,3-b]thiopyran-2-sulfonamid-7,7-dioxid, 5,6-Dihydro-6-[3-(2-methoxy)ethoxy]propyl-4-propylamino-4H-thieno[2,3-b]thiopyran-2-sulfonamid-7,7-dioxid, 5,6-Dihydro-6-[3-(2-ethoxy)ethoxy]propyl-4-propylamino-4H-thieno[2,3-b]thiopyran-2-sulfonamid-7,7-dioxid, 5,6-Dihydro-6-(3-hydroxypropyl)-4-propylamino-4H-thieno[2,3-b]thiopyran-2-sulfonamid-7,7-dioxid, 5,6-Dihydro-6-[2-(2-methoxy)ethoxy]ethyl-4-ethylamino-4H-thieno-[2,3-b]thiopyran-2-sulfonamid-7,7-dioxid, 5,6-Dihydro-6-[3-(2-methoxy)ethoxy]propyl-4-ethylamino-4H-thieno[2,3-b]thiopyran-2-sulfonamid-7,7-dioxid, 5,6-Dihydro-6-[3-(3-methoxy)propoxy]propyl-4-propylamino-4H-thieno[2,3-b]thiopyran-2-sulfonamid-7,7-dioxid oder 5,6-Dihydro-4-propylamino-6-[3-(2-hydroxyethylthio)propyl]-4H-thieno[2,3-b]thiopyran-2-sulfonamid-7,7-dioxid ist.

5. Die Verbindung nach Anspruch 2, die das cis-Diastereomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)thieno-[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethylamino)methylthieno[2,3-b]thiopyran-2-sulfonamid-7,7-dioxid, 5,6-Dihydro-6-allyloxymethyl-4-ethylamino-4H-thieno[2,3-b]thiopyran-2-sulfonamid-7,7-dioxid oder 5,6-Dihydro-4-propylamino-6-(2-methoxyethoxy)methyl-4H-thieno[2,3-b]thiopyran-2-sulfonamid-7,7-dioxid ist.

6. Die Verbindung nach Anspruch 4, die 5,6-Dihydro-6-(3-methoxypropyl)-4-propylamino-4H-thieno[2,3-b]thiopyran-2-sulfonamid-7,7-dioxid ist.

7. Eine ophthalmologische Formulierung zur Behandlung von erhöhtem Augeninnendruck, die einen ophthalmologischen Träger und eine wirksame Menge der Verbindung nach Anspruch 1 zur Senkung des erhöhten Augeninnendrucks enthält.

8. Die Verwendung einer Verbindung nach Anspruch 1 zur Herstellung eines Medikaments zur Behandlung von erhöhtem Augeninnendruck.

9. Die Verwendung einer Verbindung nach Anspruch 1 zur Herstellung eines Medikaments zur Behandlung von Psoriasis.

10. Die wie in Anspruch 9 beanspruchte Verwendung, bei der das Medikament hergerichtet ist zur Verabreichung durch topische oder orale Mittel.

**Patentansprüche für folgende Vertragsstaaten : ES, GR**

1. Ein Verfahren zur Herstellung einer Verbindung der Strukturformel:

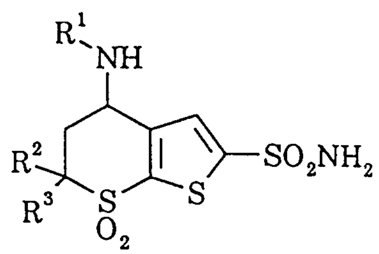

,

ihrer Isomere oder eines pharmazeutisch annehmbaren Salzes davon, worin

$R^1$ $C_{1-6}$-Alkyl ist,
$R^2$ Wasserstoff oder $C_{1-6}$-Alkyl ist,
$R^3$

(a) $C_{1-5}$-Alkoxy-$C_{1-5}$-alkyl,
(b) Hydroxy-$C_{1-5}$-alkoxy-$C_{1-5}$-alkyl,
(c) $C_{1-5}$-Alkoxy-$C_{1-5}$-alkoxy-$C_{1-5}$-alkyl,
(d) Hydroxy-$C_{1-5}$-alkylamino-$C_{1-5}$-alkyl,
(e) Hydroxy-$C_{1-5}$-alkylthio-$C_{1-5}$-alkyl oder
(f) $C_{2-5}$-Alkenyloxy-$C_{1-5}$-alkyl ist,

das umfaßt:

Reduktion der 4-N-Acylgruppe mit Boran-Dimethylsulfid-Komplex in einem Etherlösungsmittel, um eine Verbindung der Formel I zu ergeben,
oder alternativ

a) Behandeln der 4-Hydroxyverbindung mit Toluolsulfonylchlorid in Pyridin bei etwa -20°C bis 5°C und

b) Zugabe eines Alkylamins bei einer Temperatur unter etwa 15°C, gefolgt von Erwärmen auf etwa 30°C bis 60°C für etwa 5 bis 16 Stunden, um eine Verbindung der Formel I zu ergeben,

oder alternativ

a) Behandeln einer Lösung der Ketoverbindung mit einem Amin der Formel $R^1NH_2$, gefolgt von tropfenweise Titantetrachlorid, und

b) Behandeln der Verbindung in Schritt a) mit einem Metallhydridkomplex im Überschuß in einem $C_{1-3}$-Alkanol, um eine Verbindung der Formel I zu erhalten.

**2.** Ein Verfahren wie in Anspruch 1 beansprucht zur Herstellung der Verbindung nach Anspruch 1, die

a) 5,6-Dihydro-6-(2-ethoxyethyl)-4-ethylamino-4H-thieno[2,3-b]thiopyran-2-sulfonamid-7,7-dioxid,
b) 5,6-Dihydro-6-ethoxymethyl-4-ethylamino-4H-thieno[2,3-b]thiopyran-2-sulfonamid-7,7-dioxid,
c) 5,6-Dihydro-6-methoxymethyl-4-(n-propylamino)-4H-thieno[2,3-b]thiopyran-2-sulfonamid-7,7-dioxid,
d) 5,6-Dihydro-6-(3-methoxypropyl)-4-(methylamino)-4H-thieno[2,3-b]-thiopyran-2-sulfonamid-7,7-dioxid,
e) 5,6-Dihydro-6-(3-methoxypropyl)-4-(n-propylamino)-4H-thieno[2,3-b]-thiopyran-2-sulfonamid-7,7-dioxid,
f) 5,6-Dihydro-4H-4-ethylamino-6-(3-methoxypropyl)thieno[2,3-b]thiopyran-2-sulfonamid-7,7-dioxid,
g) 5,6-Dihydro-6-(3-methoxypropyl)-4-propylamino-4H-thieno[2,3-b]thiopyran-2-sulfonamid-7,7-dioxid,
h) 5,6-Dihydro-4-ethylamino-4H-6-(2-hydroxyethylamino)methylthieno[2,3-b]-thiopyran-2-sulfonamid-7,7-dioxid,
i) 5,6-Dihydro-6-allyloxymethyl-4-ethylamino-4H-thieno[2,3-b]thiopyran-2-sulfonamid-7,7-dioxid,
j) 5,6-Dihydro-6-(3-allyloxy)propyl-4-propylamino-4H-thieno[2,3-b]thiopyran-2-sulfonamid-7,7-dioxid,
k) 5,6-Dihydro-6-[3-(2-methoxy)ethoxy]propyl-4-propylamino-4H-thieno-[2,3-b]thiopyran-2-sulfonamid-7,7-dioxid,
l) 5,6-Dihydro-4-ethylamino-6-[3-(2-methoxy)ethoxy]propyl-4H-thieno[2,3-b]-thiopyran-2-sulfonamid-7,7-dioxid,
m) 5,6-Dihydro-6-[3-(2-ethoxy)ethoxy]propyl-4-propylamino-4H-thieno[2,3-b]-thiopyran-2-sulfonamid-7,7-dioxid,
n) 5,6-Dihydro-4-ethylamino-6-[2-(2-hydroxyethylthio)ethyl]-4H-thieno-[2,3-b]thiopyran-2-sulfonamid-7,7-dioxid,
o) 5,6-Dihydro-4-propylamino-6-(2-methoxyethoxy)methyl-4H-thieno[2,3-b]-thiopyran-2-sulfonamid-7,7-dioxid,
p) 5,6-Dihydro-4-ethylamino-4H-6-(2-hydroxyethyl)aminomethylthieno[2,3-b]-thiopyran-2-sulfonamid-7,7-dioxid,
s) 5,6-Dihydro-6-[2-(2-methoxy)ethoxy]ethyl-4-ethylamino-4H-thieno[2,3-b]-thiopyran-2-sulfonamid-7,7-dioxid,
t) 5,6-Dihydro-6-[3-(3-methoxy)propoxy]propyl-4-propylamino-4H-thieno-[2,3-b]thiopyran-2-sulfonamid-7,7-dioxid oder
u) 5,6-Dihydro-4-propylamino-6-[3-(2-hydroxyethylthio)propyl]-4H-thieno-[2,3-b]thiopyran-2-sulfonamid-7,7-dioxid

oder ein pharmazeutisch annehmbares Salz davon ist.

**3.** Ein Verfahren wie in Anpruch 1 beansprucht zur Herstellung der Verbindung der Formel (I), die das trans-Diastereomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)thieno[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethyl)aminomethylthieno[2,3-b]thiopyran-2-sulfonamid-7,7-dioxid, 5,6-Dihydro-6-allyloxymethyl-4-ethylamino-4H-thieno[2,3-b]thiopyran-2-sulfonamid-7,7-dioxid, 5,6-Dihydro-6-(3-allyloxy)propyl-4-propylamino-4H-thieno[2,3-b]thiopyran-2-sulfonamid-7,7-dioxid, 5,6-Dihydro-6-[3-(2-methoxy)ethoxy]propyl-4-propylamino-4H-thieno[2,3-b]thiopyran-2-sulfonamid-7,7-dioxid, 5,6-Dihydro-4-ethylamino-6-[3-(2-methoxy)ethoxylpropyl-4H-thieno[2,3-b]thiopyran-2-sulfonamid-7,7-dioxid, 5,6-Dihydro-6-[3-(2-ethoxy)ethoxy]propyl-4-propylamino-4H-thieno[2,3-b]thiopyran-2-sulfonamid-7,7-dioxid, 5,6-Dihydro-4-ethylamino-6-[2-(2-hydroxyethylthio)ethyl]-4H-thieno[2,3-b]thiopyran-2-sulfonamid-7,7-dioxid oder 5,6-Dihydro-4-propylamino-6-(2-methoxyethoxy)methyl-4H-thieno[2,3-b]-thiopyran-2-sulfonamid-7,7-dioxid ist.

**4.** Ein Verfahren wie in Anspruch 1 beansprucht zur Herstellung der Verbindung der Formel (I), die das trans-(S,S)-Enantiomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)thieno[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethylamino)methylthieno[2,3-b]thiopyran-2-sulfonamid-7,7-dioxid, 5,6-Dihydro-6-(3-allyloxy)propyl-4-propylamino-4H-thieno-[2,3-b]thiopyran-2-sulfonamid-7,7-dioxid, 5,6-Dihydro-6-[3-(2-methoxy)-ethoxyl propyl-4-propylamino-4H-thieno[2,3-b]thiopyran-2-sulfonamid-7,7-dioxid, 5,6-Dihydro-6-[3-(2-ethoxy)ethoxy]propyl-4-propylamino-4H-thieno-[2,3-b]thiopyran-2-sulfonamid-7,7-dioxid, 5,6-Dihydro-6-(3-hydroxypropyl)-4-propylamino-4H-thieno[2,3-b]thiopyran-2-sulfonamid-7,7-dioxid, 5,6-Dihydro-6-[2-(2-methoxy)ethoxy]ethyl-4-ethylamino-4H-thieno[2,3-b]thiopyran-2-sulfonamid-7,7-dioxid, 5,6-Dihydro-6-[3-(2-methoxy)ethoxy]propyl-4-ethylamino-4H-thieno[2,3-b]thiopyran-2-sulfonamid-7,7-dioxid, 5,6-Dihydro-6-[3-(3-methoxy)propoxy]propyl-4-propylamino-4H-thieno[2,3-b]thiopyran-2-sulfonamid-7,7-dioxid, oder 5,6-Dihydro-4-propylamino-6-[3-(2-hydroxyethylthio)propyl]-4H-thieno[2,3-b]thiopyran-2-sulfonamid-7,7-dioxid ist.

5. Ein Verfahren wie in Anspruch 1 beansprucht zur Herstellung der Verbindung der Formel (I), die das cis-Diastereomer von 5,6-Dihydro-6-(2-ethoxyethyl)-4-ethylamino-4H-thieno[2,3-b]thiopyran-2-sulfonamid-7,7-dioxid, 5,6-Dihydro-4H-4-ethylamino-6-(3-methoxypropyl)thieno[2,3-b]thiopyran-2-sulfonamid-7,7-dioxid, 5,6-Dihydro-6-(3-methoxypropyl)-4-propylamino-4H-thieno[2,3-b]thiopyran-2-sulfonamid-7,7-dioxid, 5,6-Dihydro-4-ethylamino-4H-6-(2-hydroxyethylamino)methylthieno[2,3-b]thiopyran-2-sulfonamid-7,7-dioxid, 5,6-Dihydro-6-allyloxymethyl-4-ethylamino-4H-thieno[2,3-b]-thiopyran-2-sulfonamid-7,7-dioxid oder 5,6-Dihydro-4-propylamino-6-(2-methoxyethoxy)methyl-4H-thieno[2,3-b]thiopyran-2-sulfonamid-7,7-dioxid ist.

6. Ein Verfahren wie in Anspruch 1 beansprucht zur Herstellung der Verbindung der Formel (I), die das 5,6-Dihydro-6-(3-methoxypropyl)-4-propylamino-4H-thieno[2,3-b]thiopyran-2-sulfonamid-7,7-dioxid ist.

**Revendications**

**Revendications pour les Etats contractants suivants : AT, BE, CH, DE, DK, FR, GB, IT, LI, LU, NL, SE**

1. Composé de formule développée :

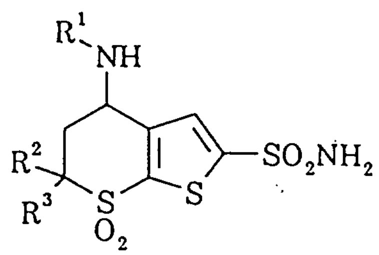

ses isomères, ou un de ses sels acceptables d'un point de vue pharmaceutique, où :

$R^1$ est un radical alkyle en $C_1$-$C_6$ ;
$R^2$ est un hydrogène ou un radical alkyle en $C_1$-$C_6$ ;
$R^3$ est un radical

(a) (alcoxy en $C_1$-$C_5$)-(alkyle en $C_1$-$C_5$),
(b) hydroxy-(alcoxy en $C_1$-$C_5$)-(alkyle en $C_1$-$C_5$),
(c) (alcoxy en $C_1$-$C_5$)-(alcoxy en $C_1$-$C_5$)-(alkyle en $C_1$-$C_5$),
(d) hydroxy-(alkylamino en $C_1$-$C_5$)-(alkyle en $C_1$-$C_5$),
(e) hydroxy-(alkylthio en $C_1$-$C_5$)-(alkyle en $C_1$-$C_5$), ou
(f) (alcényloxy en $C_2$-$C_5$)-(alkyle en $C_1$-$C_5$).

2. Composé selon la revendication 1, qui est l'un des composés suivants :

a) 7,7-dioxyde de 5,6-dihydro-6-(2-éthoxyéthyl)-4-éthylamino-4H-thiéno[2,3-b]thiopyranne-2-sulfonamide ;
b) 7,7-dioxyde de 5,6-dihydro-6-éthoxyméthyl-4-éthylamino-4H-thiéno[2,3-b]thiopyranne-2-sulfonamide ;
c) 7,7-dioxyde de 5,6-dihydro-6-méthoxyméthyl-4-(n-propylamino)-4H-thiéno[2,3-b]thiopyranne-2-sulfonamide ;
d) 7,7-dioxyde de 5,6-dihydro-6-(3-méthoxypropyl)-4-(méthylamino)-4H-thiéno[2,3-b]thiopyranne-2- sulfonamide ;

e) 7,7-dioxyde de 5,6-dihydro-6-(3-méthoxypropyl)-4-(n-propylamino)-4H-thiéno[2,3-b]thiopyranne-2-sulfonamide ;

f) 7,7-dioxyde de 5,6-dihydro-4H-4-éthylamino-6-(3-méthoxypropyl)-thiéno[2,3-b]thiopyranne-2-sulfonamide ;

g) 7,7-dioxyde de 5,6-dihydro-6-(3-méthoxypropyl)-4-propylamino-4H-thiéno[2,3-b]thiopyranne-2-sulfonamide ;

h) 7,7-dioxyde de 5,6-dihydro-4-éthylamino-4H-6-(2-hydroxyéthylamino)méthylthiéno[2,3-b]thiopyranne-2-sulfonamide ;

i) 7,7-dioxyde de 5,6-dihydro-6-allyloxyméthyl-4-éthylamino-4H-thiéno[2,3-b]thiopyranne-2-sulfonamide ;

j) 7,7-dioxyde de 5,6-dihydro-6-(3-allyloxy)-propylamino-4H-thiéno[2,3-b]thiopyranne-2-sulfonamide ;

k) 7,7-dioxyde de 5,6-dihydro-6-[3-(2-méthoxy)-éthoxy]propyl-4-propylamino-4H-thiéno[2,3-b]thiopyranne-2-sulfonamide ;

l) 7,7-dioxyde de 5,6-dihydro-4-éthylamino-6-[3-(2-méthoxy)éthoxy]propyl-4H-thiéno[2,3-b]thiopyranne-2-sulfonamide ;

m) 7,7-dioxyde de 5,6-dihydro-6-[3-(2-éthoxy)-éthoxy]propyl-4-propylamino-4H-thiéno[2,3-b]thiopyranne-2-sulfonamide ;

n) 7,7-dioxyde de 5,6-dihydro-6-[2-(2-hydroxyéthylthio)éthyl]-4H-thiéno[2,3-b]thiopyranne-2-sulfonamide ;

o) 7,7-dioxyde de 5,6-dihydro-4-propylamino-6-(2-méthoxyéthoxy)méthyl-4H-thiéno[2,3-b]thiopyranne-2-sulfonamide ;

p) 7,7-dioxyde de 5,6-dihydro-4-éthylamino-4H-6-(2-hydroxyéthyl)aminométhylthiéno[2,3-b]thiopyranne-2-sulfonamide ;

s) 7,7-dioxyde de 5,6-dihydro-6-[2-(2-méthoxy)-éthoxy]éthyl-4-éthylamino-4H-thiéno[2,3-b]thiopyranne-2- sulfonamide ;

t) 7,7-dioxyde de 5,6-dihydro-6-[3-(3-méthoxy)-propoxy]propyl-4-propylamino-4H-thiéno[2,3-b]thiopyranne-2-sulfonamide ;

u) 7,7-dioxyde de 5,6-dihydro-4-propylamino-6-[3-(2-hydroxyéthylthio)propyl]-4H-thiéno[2,3-b]thiopyranne-2-sulfonamide ;

ou l'un de ses sels acceptables d'un point de vue pharmaceutique.

3. Composé selon la revendication 2, qui est le diastéréoisomère trans du 7,7-dioxyde de 5,6-dihydro-6-(2-éthoxyéthyl)-4-éthylamino-4H-thiéno[2,3-b]thiopyranne-2-sulfonamide ; du 7,7-dioxyde de 5,6-dihydro-4H-4-éthylamino-6-(3-méthoxypropyl)-thiéno[2,3-b]thiopyranne-2-sulfonamide ; du 7,7-dioxyde de 5,6-dihydro-6-(3-méthoxypropyl)-4-propylamino-4H-thiéno[2,3-b]thiopyranne-2-sulfonamide ; du 7,7-dioxyde de 5,6-dihydro-4-éthylamino-4H-6-(2-hydroxyéthyl)aminométhylthiéno[2,3-b]thiopyranne-2-sulfonamide ; du 7,7-dioxyde de 5,6-dihydro-6-allyloxyméthyl-4-éthylamino-4H-thiéno[2,3-b]thiopyranne-2-sulfonamide ; du 7,7-dioxyde de 5,6-dihydro-6-(3-allyloxy)propyl-4-propylamino-4H-thiéno-[2,3-b]thiopyranne-2-sulfonamide ; du 7,7-dioxyde de 5,6-dihydro-6-[3(2-méthoxy)éthoxy]propyl-4-propylamino-4H-thiéno[2,3-b]thiopyranne-2-sulfonamide ; du 7,7-dioxyde de 5,6-dihydro-4-éthylamino-6-[3-(2-méthoxy)-éthoxy]propyl-4H-thiéno[2,3-b]thiopyranne-2-sulfonamide ; du 7,7-dioxyde de 5,6-dihydro-6-[3-(2-éthoxy)éthoxy]-propyl-4-propylamino-4H-thiéno[2,3-b]thiopyranne-2-sulfonamide ; du 7,7-dioxyde de 5,6-dihydro-4-éthylamino-6-[2-(2-hydroxyéthylthio)éthyl]-4H-thiéno[2,3-b]thiopyranne-2-sulfonamide ; ou du 7,7-dioxyde de 5,6-dihydro-4-propylamino-6-(2-méthoxyéthoxy)méthyl-4H-thiéno[2,3-b]thiopyranne-2-sulfonamide.

4. Composé selon la revendication 2, qui est l'énantiomère trans-(S,S) du 7,7-dioxyde de 5,6-dihydro-6-(2-éthoxyéthyl)-4-éthylamino-4H-thiéno[2,3-b]thiopyranne-2-sulfonamide ; du 7,7-dioxyde de 5,6-dihydro-4H-4-éthylamino-6-(3-méthoxypropyl)thiéno[2,3-b]thiopyranne-2-sulfonamide ; du 7,7-dioxyde de 5,6-dihydro-6-(3-méthoxypropyl)-4-propylamino-4H-thiéno[2,3-b]thiopyranne-2-sulfonamide ; du 7,7-dioxyde de 5,6-dihydro-4-éthylamino-4H-6-(2-hydroxyéthylamino)méthyl-thiéno[2,3-b]thiopyranne-2-sulfonamide ; du 7,7-dioxyde de 5,6-dihydro-6-(3-allyloxy)propyl-4-propylamino-4H-thiéno-[2,3-b]thiopyranne-2-sulfonamide ; du 7,7-dioxyde de 5,6-dihydro-6-[3(2-méthoxy)éthoxy]propyl-4-propylamino-4H-thiéno[2,3-b]thiopyranne-2-sulfonamide ; du 7,7-dioxyde de 5,6-dihydro-6-[3-(2-éthoxy)éthoxy]-propyl-4-propylamino-4H-thiéno[2,3-b]thiopyranne-2-sulfonamide ; du 7,7-dioxyde de 5,6-dihydro-6-(3-hydroxypropyl)-4-propylamino-4H-thiéno[2,3-b]thiopyranne-2-sulfonamide ; du 7,7-dioxyde de 5,6-dihydro-6-[2-(2-méthoxy)éthoxy]-éthyl-4-éthylamino-4H-thiéno[2,3-b]thiopyranne-2-sulfonamide ; du 7,7-dioxyde de 5,6-dihydro-6-[3-(2-méthoxy)éthoxy]propyl-4-éthylamino-4H-thiéno[2,3-b]thiopyranne-2-sulfonamide ; du 7,7-dioxyde de 5,6-dihydro-6-[3-(3-méthoxy)propoxy]-propyl-4-propylamino-4H-thiéno[2,3-b]thiopyranne-2-sulfonamide ; ou du 7,7-dioxyde de 5,6-dihydro-4-propylamino-6-[3-(2-hydroxyéthylthio)propyl]-4H-thiéno[2,3-b]thiopyranne-2-sulfonamide.

5. Composé selon la revendication 2, qui est le diastéréoisomère cis du 7,7-dioxyde de 5,6-dihydro-6-(2-éthoxyéthyl)-4-éthylamino-4H-thiéno[2,3-b]thiopyranne-2-sulfonamide ; 7,7-dioxyde de 5,6-dihydro-4H-4-éthylamino-6-(3-méthoxypropyl)thiéno[2,3-b]thiopyranne-2-sulfonamide ; 7,7-dioxyde de 5,6-dihydro-6-(3-méthoxypropyl)-4-propylamino-4H-thiéno[2,3-b]thiopyranne-2-sulfonamide ; 7,7-dioxyde de 5,6-dihydro-4-éthylamino-4H-6-(2-hydroxyéthylamino)-méthylthiéno[2,3-b]thio pyranne-2-sulfonamide ; 7,7-dioxyde de 5,6-dihydro-6-allyloxyméthyl-4-éthylamino-4H-thiéno[2,3-b]thiopyranne-2-sulfonamide ; ou du 7,7-dioxyde de 5,6-dihydro-4-propylamino-6-(2-méthoxyéthoxy)méthyl-4H-thiéno[2,3-b]thiopyranne-2-sulfonamide.

**6.** Composé selon la revendication 4, qui est le 7,7-dioxyde de 5,6-dihydro-6-(3-méthoxypropyl)-4-propylamino-4H-thiéno[2,3-b]thiopyranne-2-sulfonamide.

**7.** Formulation ophtalmologique pour le traitement de l'hypertension oculaire, qui comprend un support ophtalmologique et une quantité à effet antihypertenseur oculaire du composé selon la revendication 1.

**8.** Utilisation d'un composé selon la revendication 1 pour préparer un médicament destiné à traiter l'hypertension oculaire.

**9.** Utilisation d'un composé selon la revendication 1 pour préparer un médicament destiné au traitement du psoriasis.

**10.** Utilisation selon la revendication 9, dans lequel le médicament est adapté à une administration par des moyens topiques ou oraux.

**Revendications pour les Etats contractants suivants : ES, GR**

**1.** Procédé pour préparer un

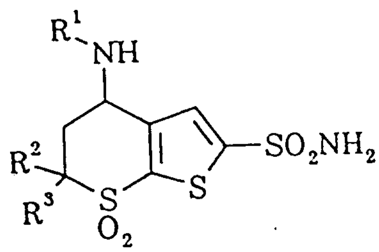

ses isomères, ou un de ses sels acceptables d'un point de vue pharmaceutique, ou :

$R^1$ est un radical alkyle en $C_1$-$C_6$ ;
$R^2$ est un hydrogène ou un radical alkyle en $C_1$-$C_6$ ;
$R^3$ est un radical

(a) (alcoxy en $C_1$-$C_5$)-(alkyle en $C_1$-$C_5$),
(b) hydroxy-(alcoxy en $C_1$-$C_5$)-(alkyle en $C_1$-$C_5$),
(c) (alcoxy en $C_1$-$C_5$)-(alcoxy en $C_1$-$C_5$)-(alkyle en $C_1$-$C_5$),
(d) hydroxy-(alkylamino en $C_1$-$C_5$)-(alkyle en $C_1$-$C_5$),
(e) hydroxy-(alkylthio en $C_1$-$C_5$)-(alkyle en $C_1$-$C_5$), ou
(f) (alcényloxy en $C_2$-$C_5$)-(alkyle en $C_1$-$C_5$),

qui consiste à réduire le groupe 4-N-acyle avec un complexe borane-sulfure de diméthyle dans un solvant de type éther pour obtenir un composé de formule I, ou bien encore,

a) à traiter le composé 4-hydroxy avec du chlorure de toluènesulfonyle dans de la pyridine à une température d'environ -20 à 5°C, et
b) à ajouter une alkylamine à une température inférieure à 15°C, puis à chauffer à environ 30 à 60°C pendant environ 5 à 16 heures pour obtenir un composé de formule I, ou bien encore,

a) à traiter une solution du composé céto avec une amine de formule $R^1NH_2$, puis, goutte-à-goutte, avec du tétrachlorure de titane, et
b) à traiter le composé de l'Etape A avec un hydrure métallique complexe en excès dans un alcanol en $C_1$-$C_3$,

pour obtenir un composé de formule I.

2. Procédé selon la revendication 1, pour préparer le composé selon la revendication 1, qui est l'un des composés suivants :

   a) 7,7-dioxyde de 5,6-dihydro-6-(2-éthoxyéthyl)-4-éthylamino-4H-thiéno[2,3-b]thiopyranne-2-sulfonamide ;
   b) 7,7-dioxyde de 5,6-dihydro-6-éthoxyméthyl-4-éthylamino-4H-thiéno[2,3-b]thiopyranne-2-sulfonamide ;
   c) 7,7-dioxyde de 5,6-dihydro-6-méthoxyméthyl-4-(n-propylamino)-4H-thiéno[2,3-b]thiopyranne-2-sulfonamide ;
   d) 7,7-dioxyde de 5,6-dihydro-6-(3-méthoxypropyl)-4-(méthylamino)-4H-thiéno[2,3-b]thiopyranne-2-sulfonamlde ;
   e) 7,7-dioxyde de 5,6-dihydro-6-(3-méthoxypropyl)-4-(n-propylamino)-4H-thiéno[2,3-b]thiopyranne-2-sulfonamide ;
   f) 7,7-dioxyde de 5,6-dihydro-4H-4-éthylamino-6-(3-méthoxypropyl)-thiéno[2,3-b]thiopyranne-2-sulfonamide ;
   g) 7,7-dioxyde de 5,6-dihydro-6-(3-méthoxypropyl)-4-propylamino-4H-thiéno[2,3-b]thiopyranne-2-sulfonamide ;
   h) 7,7-dioxyde de 5,6-dihydro-4-éthylamino-4H-6-(2-hydroxyéthylamino)méthylthiéno[2,3-b]thiopyranne-2-sulfonamide ;
   i) 7,7-dioxyde de 5,6-dihydro-6-allyloxyméthyl-4-éthylamino-4H-thiéno[2,3-b]thiopyranne-2-sulfonamide ;
   j) 7,7-dioxyde de 5,6-dihydro-6-(3-allyloxy)propylamino-4H-thiéno[2,3-b]thiopyranne-2-sulfonamide ;
   k) 7,7-dioxyde de 5,6-dihydro-6-[3-(2-méthoxy)-éthoxy]propyl-4-propylamino-4H-thiéno[2,3-b]thiopyranne- 2-sulfonamide ;
   l) 7,7-dioxyde de 5,6-dihydro-4-éthylamino-6-[3-(2-méthoxy)éthoxy]propyl-4H-thiéno[2,3-b]thiopyranne-2-sulfonamide ;
   m) 7,7-dioxyde de 5,6-dihydro-6-[3-(2-éthoxy)-éthoxy]propyl-4-propylamino-4H-thiéno[2,3-b]thiopyranne-2-sulfonamide ;
   n) 7,7-dioxyde de 5,6-dihydro-6-[2-(2-hydroxyéthylthio)éthyl]-4H-thiéno[2,3-b]thiopyranne-2-sulfonamide ;
   o) 7,7-dioxyde de 5,6-dihydro-4-propylamino-6-(2-méthoxyéthoxy)méthyl-4H-thiéno[2,3-b]thiopyranne-2-sulfonamide ;
   p) 7,7-dioxyde de 5,6-dihydro-4-éthylamino-4H-6-(2-hydroxyéthyl)aminométhylthiéno[2,3-b]thiopyranne-2-sulfonamide ;
   s) 7,7-dioxyde de 5,6-dihydro-6-[2-(2-méthoxy)-éthoxy]éthyl-4-éthylamino-4H-thiéno[2,3-b]thiopyranne-2-sulfonamide ;
   t) 7,7-dioxyde de 5,6-dihydro-6-[3-(3-méthoxy)-propoxy]propyl-4-propylamino-4H-thiéno[2,3-b]thiopyranne-2-sulfonamide ;
   u) 7,7-dioxyde de 5,6-dihydro-4-propylamino-6-[3-(2-hydroxyéthylthio)propyl]-4H-thiéno[2,3-b]thiopyranne-2-sulfonamide ;

   ou l'un de ses sels acceptables d'un point de vue pharmaceutique.

3. Procédé selon la revendication 1, pour préparer le composé de formule (I), qui est le diastéréoisomère trans du 7,7-dioxyde de 5,6-dihydro-6-(2-éthoxyéthyl)-4-éthylamino-4H-thiéno[2,3-b]thiopyranne-2-sulfonamide ; du 7,7-dioxyde de 5,6-dihydro-4H-4-éthylamino-6-(3-méthoxypropyl)-thiéno[2,3-b]thiopyranne-2-sulfonamide ; du 7,7-dioxyde de 5,6-dihydro-6-(3-méthoxypropyl)-4-propylamino-4H-thiéno[2,3-b]thiopyranne-2-sulfonamide ; du 7,7-dioxyde de 5,6-dihydro-4-éthylamino-4H-6-(2-hydroxyéthyl)aminométhylthiéno[2,3-b]thiopyranne-2- sulfonamide ; du 7,7-dioxyde de 5,6-dihydro-6-allyloxyméthyl-4-éthylamino-4H-thiéno[2,3-b]thiopyranne-2-sulfonamide ; du 7,7-dioxyde de 5,6-dihydro-6-(3-allyloxy)propyl-4-propylamino-4H-thiéno[2,3-b]thiopyranne-2-sulfonamide ; du 7,7-dioxyde de 5,6-dihydro-6-[3(2-méthoxy)éthoxy]propyl-4-propylamino-4H-thiéno[2,3-D3thiopyranne-2-sulfonamide ; du 7,7-dioxyde de 5,6-dihydro-4-éthylamino-6-[3-(2-méthoxy)éthoxy]propyl-4H-thiéno[2,3-b]thiopyranne-2-sulfonamide ; du 7,7-dioxyde de 5,6-dihydro-6-[3-(2-éthoxy)éthoxy]propyl-4-propylamino-4H-thiéno[2,3-b]thiopyranne-2-sulfonamide ; du 7,7-dioxyde de 5,6-dihydro-4-éthylamino-6-[2-(2-hydroxyéthylthio)éthyl]-4H-thiéno[2,3-b]thiopyranne-2-sulfonamide ; ou du 7,7-dioxyde de 5,6-dihydro-4-propylamino-6-(2-méthoxyéthoxy)méthyl-4H-thiéno[2,3-b]thiopyranne-2-sulfonamide.

4. Procédé selon la revendication 1, pour préparer le composé de formule (I), qui est l'énantiomère trans-(S,S) du 7,7-dioxyde de 5,6-dihydro-6-(2-éthoxyéthyl)-4-éthylamino-4H-thiéno[2,3-b]thiopyranne-2-sulfonamide ; du 7,7-dioxyde de 5,6-dihydro-4H-4-éthylamino-6-(3-méthoxypropyl)thiéno[2,3-b]thiopyranne-2-sulfonamide ; du 7,7-dioxyde de 5,6-dihydro-6-(3-méthoxypropyl)-4-propylamino-4H-thiéno[2,3-b]thiopyranne-2-sulfonamide ; du 7,7-dioxyde de 5,6-dihydro-4-éthylamino-4H-6-(2-hydroxyéthylamino)méthyl-thiéno[2,3-b]thiopyranne-2-sulfonamide ;

du 7,7-dioxyde de 5,6-dihydro-6-(3-allyloxy)propyl-4-propylamino-4H-thiéno[2,3-b]thiopyranne-2-sulfonamide ; du 7,7-dioxyde de 5,6-dihydro-6-[3(2-méthoxy)éthoxy]propyl-4-propylamino-4H-thiéno[2,3-b]thiopyranne-2-sulfonamide ; du 7,7-dioxyde de 5,6-dihydro-6-[3-(2-éthoxy)éthoxy]-propyl-4-propylamino-4H-thiéno[2,3-b]thiopyranne-2-sulfonamide ; du 7,7-dioxyde de 5,6-dihydro-6-(3-hydroxypropyl)-4-propylamino-4H-thiéno[2,3-b]thiopyranne-2-sulfonamide ; du 7,7-dioxyde de 5,6-dihydro-6-[2-(2-méthoxy)éthoxy]éthyl-4-éthylamino-4H-thiéno[2,3-b]thiopyranne-2-sulfonamide ; du 7,7-dioxyde de 5,6-dihydro-6-[3-(2-méthoxy)éthoxy]-propyl-4-éthylamino-4H-thiéno[2,3-b]thiopyranne-2-sulfonamide ; du 7,7-dioxyde de 5,6-dihydro-6-[3-(3-méthoxy)propoxy]-propyl-4-propylamino-4H-thiéno[2,3-b]thiopyranne-2-sulfonamide ; ou du 7,7-dioxyde de 5,6-dihydro-4-propylamino-6-[3-(2-hydroxyéthylthio)propyl]-4H-thiéno[2,3-b]thiopyranne-2-sulfonamide.

**5.** Procédé selon la revendication 1, pour préparer le composé de formule (I) qui est le diastéréoisomère cis du 7,7-dioxyde de 5,6-dihydro-6-(2-éthoxyéthyl)-4-éthylamino-4H-thiéno[2,3-b]thiopyranne-2-sulfonamide ; 7,7-dioxyde de 5,6-dihydro-4H-4-éthylamino-6-(3-méthoxypropyl)thiéno[2,3-b]thiopyranne-2-sulfonamide ; 7,7-dioxyde de 5,6-dihydro-6-(3-méthoxypropyl)-4-propylamino-4H-thiéno[2,3-b]thiopyranne-2-sulfonamide ; 7,7-dioxyde de 5,6-dihydro-4-éthylamino-4H-6-(2-hydroxyéthylamino)-méthylthiéno[2,3-b]thiopyranne-2-sulfonamide ; 7,7-dioxydede 5,6-dihydro-6-allyloxyméthyl-4-éthylamino-4H-thiéno[2,3-b]thiopyranne-2-sulfonamide ; ou du 7,7-dioxyde de 5,6-dihydro-4-propylamino-6-(2-méthoxyéthoxy)méthyl-4H-thiéno[2,3-b]thiopyranne-2-sulfonamide.

**6.** Procédé selon la revendication 1, pour préparer le composé de formule (I), qui est le 7,7-dioxyde de 5,6-dihydro-6-(3-méthoxypropyl)-4-propylamino-4H-thiéno-[2,3-b]thiopyranne-2-sulfonamide.